# EUROPEAN PATENT SPECIFICATION

(11) **EP 3 487 493 B1**
(45) Date of publication and mention of the grant of the patent: **01.09.2021**
(21) Application number: 17746025.0
(22) Date of filing: 20.07.2017
(51) Int. Cl.: A61K 31/57, A61K 31/40, A61P 5/10, A61K 38/22, A61P 15/08

(54) **OXYTOCIN ANTAGONIST DOSING REGIMENS FOR PROMOTING EMBRYO IMPLANTATION AND PREVENTING MISCARRIAGE**
OXYTOCINANTAGONISTDOSIERPLÄNE ZUR FÖRDERUNG DER EMBRYOIMPLANTATION UND ZUR VORBEUGUNG DER FEHLGEBURT
RÉGIMES POSOLOGIQUES D'ANTAGONISTES DE L'OCYTOCINE POUR FAVORISER L'IMPLANTATION D'EMBRYONS ET PRÉVENIR LES FAUSSES COUCHES

(30) Priority: 21.07.2016 US 201662365147 P; 30.06.2017 US 201762527721 P
(43) Date of publication of application: 29.05.2019
(73) Proprietor: ObsEva S.A., 1228 Plan-les-Ouates/Geneve (CH)
(72) Inventor: LOUMAYE, Ernest, 1223 Cologny (CH); GOTTELAND, Jean-Pierre, 1205 Geneva (CH)
(74) Representative: KATZAROV S.A.
(86) International application number: PCT/EP2017/068378
(87) International publication number: WO 2018/015497

(56) References cited:
- EP-A1- 2 845 850
- WO-A1-2014/080032
- US-A1- 2008 318 847
- US-A1- 2011 020 847
- US-A1- 2015 164 859
- US-A1- 2016 002 160
- US-A1- 2016 175 283
- PEI-YI CHOU ET AL: "Use of an oxytocin antagonist infertilizationembryo transfer for women with repeated implantation failure: A retrospective study", TAIWANESE JOURNAL OF OBSTETRICS AND GYNECOLOGY, ELSEVIER (SINGAPORE) PTE LTD, HONG KONG BRANCH, HONG KONG, HK, vol. 50, no. 2, 21 February 2011 (2011-02-21), pages 136-140, XP028246160, ISSN: 1028-4559, DOI: 10.1016/J.TJOG.2011.04.003 [retrieved on 2011-04-27]
- PIERZYNSKI ET AL: "Oxytocin antagonists may improve infertility treatment", FERTILITY AND STERILITY, ELSEVIER SCIENCE INC, NEW YORK, NY, USA, vol. 88, no. 1, 1 July 2007 (2007-07-01), pages 213.e19-213.e22, XP022137899, ISSN: 0015-0282, DOI: 10.1016/J.FERTNSTERT.2006.09.017
- MORALOGLU O ET AL: "Treatment with oxytocin antagonists before embryo transfer may increase implantation rates after IVF", REPRODUCTIVE BIOMEDICINE ONLINE, ELSEVIER, AMSTERDAM, NL, vol. 21, no. 3, 1 September 2010 (2010-09-01), pages 338-343, XP027238024, ISSN: 1472-6483 [retrieved on 2010-08-25]
- MONA M ABOULGHAR ET AL: "The use of vaginal natural progesterone for prevention of preterm birth in IVF/ICSI pregnancies", REPRODUCTIVE BIOMEDICINE ONLINE, ELSEVIER, AMSTERDAM, NL, vol. 25, no. 2, 27 March 2012 (2012-03-27) , pages 133-138, XP028408735, ISSN: 1472-6483, DOI: 10.1016/J.RBMO.2012.03.013 [retrieved on 2012-04-03]
- L. ZHU ET AL: "Uterine peristalsis before embryo transfer affects the chance of clinical pregnancy in fresh and frozen-thawed embryo transfer cycles", HUMAN REPRODUCTION, vol. 29, no. 6, 23 March 2014 (2014-03-23) , pages 1238-1243, XP055411083, GB ISSN: 0268-1161, DOI: 10.1093/humrep/deu058
- Anonymous: "ObsEva Announces Results of the IMPLANT Phase 2 Trial of OBE001 (nolasiban) for the Improvement of Pregnancy and Live Birth Rates Following IVF/ICSI | ObsEva", , 24 October 2016 (2016-10-24), XP055411137, Retrieved from the Internet: URL:http://www.obseva.com/news/obseva-anno unces-results-of-the-implant-phase-2-trial -of-obe001-nolasiban-for-the-improvement-o f-pregnancy-and-live-birth-rates-following -ivf-icsi [retrieved on 2017-09-28]

## Description

### Field of the Invention

The invention relates to composition and methods for dosing subjects with oxytocin antagonists to enhance endometrial receptivity and reduce the likelihood of embryo implantation failure in subjects undergoing embryo transfer therapy.

### Background of the Invention

Despite recent progress in assisted reproductive technology, the overall effectiveness of even advanced treatments, such as in vitro fertilization (IVF) followed by embryo transfer (IVF/ET) remains relatively low, resulting in an average of about 30% live births per treatment cycle (Andersen et al., Human Reproduction 24:1267-1287 (2009)). Moreover, the embryo implantation success rate tends to decrease with age. Many current treatment strategies to promote successful embryo implantation in a subject undergoing embryo transfer therapy have focused on the inhibition of uterine contractions prior to embryo transfer. Such treatment modalities include the administration of β-adrenergic receptor agonists and non-steroidal anti-inflammatory drugs (NSAIDS), which have not been shown to provide sufficient clinical benefit (Bernabeu et al., Human Reproduction 21:364-368 (2006); Moon et al., Fertility and Sterility 82:816-820 (2004); and Tsirigotis et al., Human Reproduction 15:10 (2000)).

US 2008/0318847 describes the use of certain oxytocin antagonists, particularly barusiban and atosiban, in assisted reproduction technology. US 2016/0175283 describes assisted reproduction technology procedures that are carried out using embryos of particular ages. WO 2014/080032 describes methods of analyzing uterine contractility in patients undergoing an embryo transfer process. Chou et al., Taiwanese Journal of Obstetrics and Gynecology 50:136-140 (2011) describes assisted reproduction technology applied to subjects that have previously experienced embryo implantation failure. Pierzynski et al., Fertility and Sterility 88:213.e19-213.e22 (2007) describes the use of atosiban in methods aimed at improving uterine receptivity. Moraloglu et al., Reproductive Biomedicine Online 21:338-343 (2010) describes the administration of atosiban to subjects undergoing embryo transfer procedures. EP 2845850 describes nolasiban and pharmaceutical compositions containing the same that have particular levels of purity, as well as medical uses of nolasiban. US 2016/0002160 describes crystalline forms of nolasiban. US 2015/0164859 describes orally-bioavailable formulations of nolasiban. Aboulghar et al., Reproductive Biomedicine Online 25:133-138 (2012) describes the use of vaginal progesterone in conjunction with assisted reproduction technology procedures. US 2011/0020847 describes in vitro assays for measuring progesterone concentrations. Zhu et al., Human Reproduction 29:1238-1243 (2014) describes the effects of uterine peristalsis on pregnancy outcomes in assisted reproduction technology procedures.

There remains a need for treatment procedures and dosing regimens that can be used to promote successful embryo implantation, for instance, by enhancing endometrial receptivity upon embryo transfer in patients undergoing assisted reproductive technology procedures.

### Summary of the Invention

The present invention is defined by the independent claims. The dependent claims depict other embodiments of the invention.

The disclosure provides methods of dosing a subject undergoing embryo transfer therapy with an oxytocin antagonist, for example, to enhance endometrial receptivity upon embryo implantation and to reduce the likelihood of embryo implantation failure and miscarriage. In some embodiments of the disclosure, the oxytocin antagonist is an inhibitor of the oxytocin receptor. Oxytocin antagonists that can be used in conjunction with the compositions and methods described herein include substituted pyrrolidin-3-one oxime derivatives, such as (3Z,5S)-5-(hydroxymethyl)-1-[(2'-methyl-1,1'-biphenyl-4-yl)carbonyl]pyrrolidin-3-one O-methyloxime. Additional examples of oxytocin antagonists that may be used in conjunction with the compositions and methods described herein include epelsiban, retosiban, barusiban, and atosiban, as well as derivatives and variants thereof. Using the compositions and methods described herein, oxytocin antagonists such as the foregoing can be administered to a subject prior to, concurrently with, or after embryo transfer so as to improve endometrial receptivity and reduce the likelihood of embryo implantation failure. The oxytocin antagonist can be administered to the subject in a single dose or in multiple doses, such as doses of varying strength or repeat doses of the same strength. For instance, the oxytocin antagonist may be administered to the subject undergoing embryo transfer in a single high dose or in multiple, lower-strength doses so as to achieve a maximal plasma concentration of the oxytocin antagonist (for instance, of from about 1 µM to about 20 µM, such as from about 1 µM to about 20 µM of a compound represented by formula (I) or (II) as described herein). According to the methods of the disclosure, oxytocin receptor antagonists such as those described herein can be administered to a subject prior to, concurrently with, or after intrauterine transfer of one or more embryos produced ex vivo, for instance, by in vitro fertilization (IVF) or intracytoplasmic sperm injection (ICSI) procedures. The one or more embryos may, for example, be produced by fertilization of an ovum derived from the subject that is undergoing the embryo transfer procedure, or may be derived from a donor that is not undergoing the embryo transfer procedure.

In a first aspect, the disclosure provides the use of an oxytocin antagonist in a method of treating a subject undergoing embryo transfer therapy, wherein the concentration of P4 in a sample isolated from the subject has been determined, wherein the method includes:
a. comparing the concentration of P4 to a P4 reference level; and
b. administering to the subject a therapeutically effective amount of an oxytocin antagonist if the concentration of P4 in the sample isolated from the subject is below the P4 reference level;
wherein one or more embryos are transferred to the uterus of the subject.

In another aspect, the disclosure features the use of an oxytocin antagonist in a method of treating a subject undergoing embryo transfer therapy, wherein the method includes:
a. determining the concentration of P4 in a sample isolated from the subject;
b. comparing the concentration of P4 to a P4 reference level; and
c. administering to the subject a therapeutically effective amount of an oxytocin antagonist if the concentration of P4 in the sample isolated from the subject is below the P4 reference level;
wherein one or more embryos are transferred to the uterus of the subject.

In some embodiments, the subject is identified as having a concentration of P4 in the sample isolated from the subject that is less than the P4 reference level. Thus, in some embodiments, the method includes comparing the concentration of P4 in the sample isolated from the subject to a P4 reference level, determining that the concentration of P4 in the sample isolated from the subject is less than the P4 reference level, and administering a therapeutically effective amount of the oxytocin antagonist to the subject.

In some embodiments, the method includes the step of informing the subject that the subject has been identified as having a concentration of P4 in the sample isolated from the subject that is less than the P4 reference level.

In another aspect, the disclosure features the use of an oxytocin antagonist in a method of treating a subject undergoing embryo transfer therapy, wherein the concentration of P4 in a sample isolated from the subject has been determined, wherein the method includes:
a. comparing the concentration of P4 to a P4 reference level;
b. administering to the subject a therapeutically effective amount of an oxytocin antagonist if the concentration of P4 in the sample isolated from the subject is below the P4 reference level; and
c. transferring one or more embryos to the uterus of the subject.

In another aspect, the invention features the use of an oxytocin antagonist in a method of treating a subject undergoing embryo transfer therapy, wherein the method includes:
a. determining the concentration of P4 in a sample isolated from the subject;
b. comparing the concentration of P4 to a P4 reference level;
c. administering to the subject a therapeutically effective amount of an oxytocin antagonist if the concentration of P4 in the sample isolated from the subject is below the P4 reference level; and
d. transferring one or more embryos to the uterus of the subject.

In some embodiments, the subject is identified as having a concentration of P4 in the sample isolated from the subject that is less than the P4 reference level. Thus, in some embodiments, the method includes comparing the concentration of P4 in the sample isolated from the subject to a P4 reference level, determining that the concentration of P4 in the sample isolated from the subject is less than the P4 reference level, administering a therapeutically effective amount of the oxytocin antagonist to the subject, and transferring one or more embryos to the uterus of the subject.

In some embodiments, the method includes the step of informing the subject that the subject has been identified as having a concentration of P4 in the sample isolated from the subject that is less than the P4 reference level.

In another aspect, the disclosure features a method of determining whether a subject undergoing embryo transfer therapy is likely to benefit from oxytocin antagonist treatment, wherein the concentration of P4 in a sample isolated from the subject has been determined, the method including comparing the concentration of P4 to a P4 reference level, wherein a reduced concentration of P4 in the sample isolated from the subject relative to the P4 reference level identifies the subject as likely to benefit from oxytocin antagonist treatment prior to, concurrently with, and/or following transfer of one or more embryos to the subject.

In another aspect, the disclosure features a method of determining whether a subject undergoing embryo transfer therapy is likely to benefit from oxytocin antagonist treatment, the method including determining the concentration of P4 in a sample isolated from the subject and comparing the concentration of P4 to a P4 reference level, wherein a reduced concentration of P4 in the sample isolated from the subject relative to the P4 reference level identifies the subject as likely to benefit from oxytocin antagonist treatment prior to, concurrently with, and/or following transfer of one or more embryos to the subject.

In another aspect, the disclosure features a method of collecting data for determining whether a subject undergoing embryo transfer therapy is likely to benefit from oxytocin antagonist treatment, wherein the concentration of P4 in a sample isolated from the subject has been determined, the method including comparing the concentration of P4 to a P4 reference level, wherein a reduced concentration of P4 in the sample isolated from the subject relative to the P4 reference level identifies the subject as likely to benefit from oxytocin antagonist treatment prior to, concurrently with, and/or following transfer of one or more embryos to the subject.

In another aspect, the disclosure features a method of collecting data for determining whether a subject undergoing embryo transfer therapy is likely to benefit from oxytocin antagonist treatment, the method including determining the concentration of P4 in a sample isolated from the subject and comparing the concentration of P4 to a P4 reference level, wherein a reduced concentration of P4 in the sample isolated from the subject relative to the P4 reference level identifies the subject as likely to benefit from oxytocin antagonist treatment prior to, concurrently with, and/or following transfer of one or more embryos to the subject.

In another aspect, the disclosure features a probe for specifically detecting P4 in the manufacture of a kit for use in a method of determining whether a subject undergoing embryo transfer therapy is likely to benefit from oxytocin antagonist treatment, wherein the concentration of P4 in a sample isolated from the subject has been determined, the method including comparing the concentration of P4 to a P4 reference level, wherein a reduced concentration of P4 in the sample isolated from the subject relative to the P4 reference level identifies the subject as likely to benefit from oxytocin antagonist treatment prior to, concurrently with, and/or following transfer of one or more embryos to the subject.

In another aspect, the disclosure features a probe for specifically detecting P4 in the manufacture of a kit for use in a method of determining whether a subject undergoing embryo transfer therapy is likely to benefit from oxytocin antagonist treatment, the method including determining the concentration of P4 in a sample isolated from the subject and comparing the concentration of P4 to a P4 reference level, wherein a reduced concentration of P4 in the sample isolated from the subject relative to the P4 reference level identifies the subject as likely to benefit from oxytocin antagonist treatment prior to, concurrently with, and/or following transfer of one or more embryos to the subject.

In another aspect, the disclosure features a method of determining whether a subject undergoing embryo transfer therapy is likely to exhibit enhanced endometrial receptivity in response to oxytocin antagonist treatment, wherein the concentration of P4 in a sample isolated from the subject has been determined, the method including comparing the concentration of P4 to a P4 reference level, wherein a reduced concentration of P4 in the sample isolated from the subject relative to the P4 reference level identifies the subject as likely to exhibit enhanced endometrial receptivity in response to oxytocin antagonist treatment prior to, concurrently with, and/or following transfer of one or more embryos to the subject.

In another aspect, the disclosure features a method of determining whether a subject undergoing embryo transfer therapy is likely to exhibit enhanced endometrial receptivity in response to oxytocin antagonist treatment, the method including determining the concentration of P4 in a sample isolated from the subject and comparing the concentration of P4 to a P4 reference level, wherein a reduced concentration of P4 in the sample isolated from the subject relative to the P4 reference level identifies the subject as likely to exhibit enhanced endometrial receptivity in response to oxytocin antagonist treatment prior to, concurrently with, and/or following transfer of one or more embryos to the subject.

In another aspect, the disclosure features a method of collecting data for determining whether a subject undergoing embryo transfer therapy is likely to exhibit enhanced endometrial receptivity in response to oxytocin antagonist treatment, wherein the concentration of P4 in a sample isolated from the subject has been determined, the method including comparing the concentration of P4 to a P4 reference level, wherein a reduced concentration of P4 in the sample isolated from the subject relative to the P4 reference level identifies the subject as likely to exhibit enhanced endometrial receptivity in response to oxytocin antagonist treatment prior to, concurrently with, and/or following transfer of one or more embryos to the subject.

In another aspect, the disclosure features a method of collecting data for determining whether a subject undergoing embryo transfer therapy is likely to exhibit enhanced endometrial receptivity in response to oxytocin antagonist treatment, the method including determining the concentration of P4 in a sample isolated from the subject and comparing the concentration of P4 to a P4 reference level, wherein a reduced concentration of P4 in the sample isolated from the subject relative to the P4 reference level identifies the subject as likely to exhibit enhanced endometrial receptivity in response to oxytocin antagonist treatment prior to, concurrently with, and/or following transfer of one or more embryos to the subject.

In another aspect, the disclosure features a probe for specifically detecting progesterone in the manufacture of a kit for use in a method of determining whether a subject undergoing embryo transfer therapy is likely to exhibit enhanced endometrial receptivity in response to oxytocin antagonist treatment, wherein the concentration of P4 in a sample isolated from the subject has been determined, the method including comparing the concentration of P4 to a P4 reference level, wherein a reduced concentration of P4 in the sample isolated from the subject relative to the P4 reference level identifies the subject as likely to exhibit enhanced endometrial receptivity in response to oxytocin antagonist treatment prior to, concurrently with, and/or following transfer of one or more embryos to the subject.

In another aspect, the disclosure features a probe for specifically detecting progesterone in the manufacture of a kit for use in a method of determining whether a subject undergoing embryo transfer therapy is likely to exhibit enhanced endometrial receptivity in response to oxytocin antagonist treatment, the method including determining the concentration of P4 in a sample isolated from the subject and comparing the concentration of P4 to a P4 reference level, wherein a reduced concentration of P4 in the sample isolated from the subject relative to the P4 reference level identifies the subject as likely to exhibit enhanced endometrial receptivity in response to oxytocin antagonist treatment prior to, concurrently with, and/or following transfer of one or more embryos to the subject.

In some embodiments of the foregoing twelve aspects of the disclosure, the subject is identified as having a concentration of P4 in the sample isolated from the subject that is less than the P4 reference level.

In some embodiments, the method includes the step of informing the subject that the subject has been identified as having a concentration of P4 in the sample isolated from the subject that is less than the P4 reference level. Thus, in some embodiments, the method includes the step of informing the subject that the subject has been identified as likely to benefit from oxytocin antagonist treatment. In some embodiments, the method includes the step of informing the subject that the subject has been identified as likely to exhibit enhanced endometrial receptivity in response to oxytocin antagonist treatment.

In some embodiments, the method includes administering a therapeutically effective amount of an oxytocin antagonist to the subject if a reduced concentration of P4 in the sample isolated from the subject relative to the P4 reference level is detected. Thus, in some embodiments, the method includes comparing the concentration of P4 to a P4 reference level, determining that the concentration of P4 in the sample isolated from the subject is less than the P4 reference level, identifying the subject as likely to benefit from oxytocin antagonist treatment and/or identifying the subject as likely to exhibit enhanced endometrial receptivity in response to oxytocin antagonist treatment, and administering a therapeutically effective amount of an oxytocin antagonist to the subject.

In some embodiments of any of the above aspects of the disclosure, administering of the oxytocin antagonist reduces the likelihood of embryo implantation failure in the subject.

In some embodiments of any of the above aspects of the disclosure, the oxytocin antagonist is administered to the subject prior to the transfer of the one or more embryos to the uterus of the subject.

In some embodiments of any of the above aspects of the disclosure, the oxytocin antagonist is administered to the subject from about 1 hour to about 24 hours prior to the transfer of the one or more embryos to the subject. In some embodiments, the oxytocin antagonist is administered to the subject from about 1 hour to about 12 hours prior the transfer of the one or more embryos to the subject. In some embodiments, the oxytocin antagonist is administered to the subject from about 12 hours to about 24 hours prior the transfer of the one or more embryos to the subject.

In some embodiments of any of the above aspects of the disclosure, the oxytocin antagonist is administered to the subject from about 1 hour to about 10 hours prior the transfer of the one or more embryos to the subject. In some embodiments, the oxytocin antagonist is administered to the subject from about 1 hour to about 9 hours prior the transfer of the one or more embryos to the subject. In some embodiments, the oxytocin antagonist is administered to the subject from about 1 hour to about 8 hours prior the transfer of the one or more embryos to the subject. In some embodiments, the oxytocin antagonist is administered to the subject from about 1 hour to about 7 hours prior the transfer of the one or more embryos to the subject. In some embodiments, the oxytocin antagonist is administered to the subject from about 1 hour to about 6 hours prior the transfer of the one or more embryos to the subject. In some embodiments, the oxytocin antagonist is administered to the subject from about 1 hour to about 5 hours prior the transfer of the one or more embryos to the subject. In some embodiments, the oxytocin antagonist is administered to the subject from about 1 hour to about 4 hours prior the transfer of the one or more embryos to the subject.

In some embodiments of any of the above aspects of the disclosure, the oxytocin antagonist is administered to the subject from about 2 hours to about 6 hours prior the transfer of the one or more embryos to the subject. In some embodiments, the oxytocin antagonist is administered to the subject from about 3 hours to about 5 hours prior the transfer of the one or more embryos to the subject. In some embodiments, the oxytocin antagonist is administered to the subject about 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, or 24 hours, or more prior to the transfer of the one or more embryos to the subject.

In some embodiments of any of the above aspects of the disclosure, the oxytocin antagonist is administered to the subject about 4 hours prior to the transfer of the one or more embryos to the subject.

In some embodiments of any of the above aspects of the disclosure, the oxytocin antagonist is administered to the subject prior to embryo transfer in a single dose.

In some embodiments of any of the above aspects of the disclosure, the oxytocin antagonist is administered to the subject prior to embryo transfer (i.e., prior to the transfer of the one or more embryos to the uterus of the subject) in multiple doses (for instance, in multiple periodic doses), such as from 1 to 20 doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, prior to embryo transfer. In some embodiments, the oxytocin antagonist is administered to the subject prior to embryo transfer in from 1 to 20 doses per 24 hours, such as 1 dose per 24 hours, 2 doses per 24 hours, 3 doses per 24 hours, 4 doses per 24 hours, 5 doses per 24 hours, 6 doses per 24 hours, 7 doses per 24 hours, 8 doses per 24 hours, 9 doses per 24 hours, 10 doses per 24 hours, 11 doses per 24 hours, 12 doses per 24 hours, 13 doses per 24 hours, 14 doses per 24 hours, 15 doses per 24 hours, 16 doses per 24 hours, 17 doses per 24 hours, 18 doses per 24 hours, 19 doses per 24 hours, 20 doses per 24 hours. In some embodiments, the oxytocin antagonist is administered to the subject prior to embryo transfer in more than 20 doses per 24 hours.

For instance, in some embodiments, the oxytocin antagonist is administered to the subject in from 1 to 10 doses, for example, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, prior to embryo transfer. In some embodiments, the oxytocin antagonist is administered to the subject prior to embryo transfer in from 1 to 10 doses per 24 hours, such as 1 dose per 24 hours, 2 doses per 24 hours, 3 doses per 24 hours, 4 doses per 24 hours, 5 doses per 24 hours, 6 doses per 24 hours, 7 doses per 24 hours, 8 doses per 24 hours, 9 doses per 24 hours, 10 doses per 24 hours.

In some embodiments, the oxytocin antagonist is administered to the subject in from 1 to 5 doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, prior to embryo transfer. In some embodiments, the oxytocin antagonist is administered to the subject in from 10 to 20 doses, for instance, per 12 hours, per 24 hours, per 36 hours, per 48 hours, per 60 hours, per 72 hours, per 84 hours, per 96 hours, per 108 hours, per 120 hours, per 132 hours, per 144 hours, per 156 hours, per 168 hours, or longer, prior to embryo transfer. In some embodiments, the oxytocin antagonist is administered to the subject in from 10 to 15 doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, prior to embryo transfer.

In some embodiments, the oxytocin antagonist is administered to the subject in 1, 2, 3, 4, 5, 6, 7, 8, 9, 10, 11, 12, 13, 14, 15, 16, 17, 18, 19, 20, or more, doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, prior to embryo transfer.

In some embodiments, the oxytocin antagonist is administered to the subject prior to embryo transfer in up to 7 doses (e.g., 1, 2, 3, 4, 5, 6, or 7 doses) per 24 hours, such as in up to 7 x 100 mg doses per 24 hours of compound (II). In some embodiments, the oxytocin antagonist is administered to the subject prior to embryo transfer in 1 dose per 24 hours, such as 1 dose per 24 hours of compound (II). In some embodiments, the oxytocin antagonist is administered to the subject prior to embryo transfer in 2 doses per 24 hours, such as 2 doses per 24 hours of compound (II). In some embodiments, the oxytocin antagonist is administered to the subject prior to embryo transfer in 3 doses per 24 hours, such as 3 doses per 24 hours of compound (II). In some embodiments, the oxytocin antagonist is administered to the subject prior to embryo transfer in 4 doses per 24 hours, such as 4 doses per 24 hours of compound (II). In some embodiments, the oxytocin antagonist is administered to the subject prior to embryo transfer in 5 doses per 24 hours, such as 5 doses per 24 hours of compound (II). In some embodiments, the oxytocin antagonist is administered to the subject prior to embryo transfer in 6 doses per 24 hours, such as 6 doses per 24 hours of compound (II). In some embodiments, the oxytocin antagonist is administered to the subject prior to embryo transfer in 7 doses per 24 hours, such as 7 doses per 24 hours of compound (II).

The multiple doses may be administered, for example, starting at from about 1 hour to about 14 days, or more, prior to embryo transfer. In some embodiments, the multiple doses are administered starting at from about 1 hour to about 7 days, or more, prior to embryo transfer. In some embodiments, the multiple doses may be administered starting at from about 1 day to about 14 days prior to embryo transfer. In some embodiments, the multiple doses may be administered starting at from about 3 days to about 11 days prior to embryo transfer. In some embodiments, the multiple doses may be administered starting at from about 1 day to about 7 days prior to embryo transfer. In some embodiments, the multiple doses may be administered starting at from about 2 days to about 5 days prior to embryo transfer. In some embodiments, the multiple doses may be administered starting at from about 3 days to about 4 days prior to embryo transfer. For instance, the multiple doses may be administered starting at 1 hour, 2 hours, 3 hours, 4 hours, 5 hours, 6 hours, 7 hours, 8 hours, 9 hours, 10 hours, 11 hours, 12 hours, 24 hours, 36 hours, 48 hours, 60 hours, 72 hours, 84 hours, 96 hours, 108 hours, 120 hours, 132 hours, 144 hours, 156 hours, 168 hours, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, or more, prior to embryo transfer to the subject.

In some embodiments, the multiple doses are administered starting at about 2 days prior to embryo transfer.

In some embodiments, the multiple doses are administered starting at about 3 days prior to embryo transfer.

In some embodiments, the multiple doses are administered starting at about 4 days prior to embryo transfer.

In some embodiments, the multiple doses are administered starting at about 5 days prior to embryo transfer.

In some embodiments, the multiple doses are administered starting at about 6 days prior to embryo transfer.

In some embodiments, the multiple doses are administered starting at about 7 days prior to embryo transfer.

In some embodiments, the multiple doses terminate on the day of embryo transfer to the subject. In some embodiments, the multiple doses terminate with a final dose of the oxytocin antagonist that is administered concurrently with (e.g., within 60 minutes of) transfer of the one or more embryos to the subject.

In some embodiments of any of the above aspects of the disclosure, the multiple doses continue following embryo transfer. For instance, the oxytocin antagonist may be administered to the subject in one or more additional doses concurrently with embryo transfer. In some embodiments, the oxytocin antagonist is administered to the subject in one or more additional doses following embryo transfer (for instance, in multiple periodic doses), such as in one or more additional doses administered within about 1 hour to about 1 week, or longer (e.g., within about 1 hour, 2 hours, 3 hours, 4 hours, 5 hours, 6 hours, 7 hours, 8 hours, 9 hours, 10 hours, 11 hours, 12 hours, 24 hours, 36 hours, 48 hours, 60 hours, 72 hours, 84 hours, 96 hours, 108 hours, 120 hours, 132 hours, 144 hours, 156 hours, 168 hours, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, or more) following the transfer of the one or more embryos to the subject.

For example, in some embodiments, the oxytocin antagonist is administered to the subject in one or more additional doses within about 1 hour to about 24 hours following the transfer of the one or more embryos to the subject. In some embodiments, the oxytocin antagonist is administered to the subject in one or more additional doses within about 1 hour to about 12 hours following the transfer of the one or more embryos to the subject. In some embodiments, the oxytocin antagonist is administered to the subject in one or more additional doses within from about 12 hours to about 24 hours following the transfer of the one or more embryos to the subject. In some embodiments, the oxytocin antagonist is administered to the subject in one or more additional doses within from about 1 hour to about 10 hours following the transfer of the one or more embryos to the subject. In some embodiments, the oxytocin antagonist is administered to the subject in one or more additional doses within from about 1 hour to about 9 hours following the transfer of the one or more embryos to the subject. In some embodiments, the oxytocin antagonist is administered to the subject in one or more additional doses within from about 1 hour to about 8 hours following the transfer of the one or more embryos to the subject. In some embodiments, the oxytocin antagonist is administered to the subject in one or more additional doses within from about 1 hour to about 7 hours following the transfer of the one or more embryos to the subject. In some embodiments, the oxytocin antagonist is administered to the subject in one or more additional doses within from about 1 hour to about 6 hours following the transfer of the one or more embryos to the subject. In some embodiments, the oxytocin antagonist is administered to the subject in one or more additional doses within from about 1 hour to about 5 hours following the transfer of the one or more embryos to the subject. In some embodiments, the oxytocin antagonist is administered to the subject in one or more additional doses within from about 1 hour to about 4 hours following the transfer of the one or more embryos to the subject. In some embodiments, the oxytocin antagonist is administered to the subject in one or more additional doses within from about 2 hours to about 6 hours following the transfer of the one or more embryos to the subject. In some embodiments, the oxytocin antagonist is administered to the subject in one or more additional doses within from about 3 hours to about 5 hours following the transfer of the one or more embryos to the subject.

In some embodiments, the oxytocin antagonist is administered to the subject in one or more additional doses starting at about 1 hour, 2 hours, 3 hours, 4 hours, 5 hours, 6 hours, 7 hours, 8 hours, 9 hours, 10 hours, 11 hours, 12 hours, 24 hours, 36 hours, 48 hours, 60 hours, 72 hours, 84 hours, 96 hours, 108 hours, 120 hours, 132 hours, 144 hours, 156 hours, 168 hours, or more, following the transfer of the one or more embryos to the subject.

In some embodiments, the oxytocin antagonist is administered to the subject in multiple additional doses following embryo transfer, such as in from 1 to 20 additional doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer. In some embodiments, the oxytocin antagonist is additionally administered to the subject following embryo transfer in from 1 to 20 doses per 24 hours, such as 1 dose per 24 hours, 2 doses per 24 hours, 3 doses per 24 hours, 4 doses per 24 hours, 5 doses per 24 hours, 6 doses per 24 hours, 7 doses per 24 hours, 8 doses per 24 hours, 9 doses per 24 hours, 10 doses per 24 hours, 11 doses per 24 hours, 12 doses per 24 hours, 13 doses per 24 hours, 14 doses per 24 hours, 15 doses per 24 hours, 16 doses per 24 hours, 17 doses per 24 hours, 18 doses per 24 hours, 19 doses per 24 hours, 20 doses per 24 hours. In some embodiments, the oxytocin antagonist is additionally administered to the subject following embryo transfer in more than 20 doses per 24 hours.

For instance, in some embodiments, the oxytocin antagonist is administered to the subject in from 1 to 10 additional doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer. In some embodiments, the oxytocin antagonist is additionally administered to the subject following embryo transfer in from 1 to 10 doses per 24 hours, such as 1 dose per 24 hours, 2 doses per 24 hours, 3 doses per 24 hours, 4 doses per 24 hours, 5 doses per 24 hours, 6 doses per 24 hours, 7 doses per 24 hours, 8 doses per 24 hours, 9 doses per 24 hours, 10 doses per 24 hours.

In some embodiments, the oxytocin antagonist is administered to the subject in from 1 to 5 additional doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer. In some embodiments, the oxytocin antagonist is administered to the subject in from 10 to 20 additional doses, for instance, per 12 hours, per 24 hours, per 36 hours, per 48 hours, per 60 hours, per 72 hours, per 84 hours, per 96 hours, per 108 hours, per 120 hours, per 132 hours, per 144 hours, per 156 hours, per 168 hours, or longer, following embryo transfer. In some embodiments, the oxytocin antagonist is administered to the subject in from 10 to 15 additional doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer.

In some embodiments, the oxytocin antagonist is administered to the subject in 1, 2, 3, 4, 5, 6, 7, 8, 9, 10, 11, 12, 13, 14, 15, 16, 17, 18, 19, 20, or more, additional doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer.

In some embodiments, the oxytocin antagonist is administered to the subject following embryo transfer in up to 7 additional doses (e.g., 1, 2, 3, 4, 5, 6, or 7 doses) per 24 hours, such as in up to 7 x 100 mg doses per 24 hours of compound (II). In some embodiments, the oxytocin antagonist is additionally administered to the subject following embryo transfer in 1 dose per 24 hours, such as 1 additional dose per 24 hours of compound (II). In some embodiments, the oxytocin antagonist is additionally administered to the subject following embryo transfer in 2 doses per 24 hours, such as 2 additional doses per 24 hours of compound (II). In some embodiments, the oxytocin antagonist is additionally administered to the subject following embryo transfer in 3 doses per 24 hours, such as 3 additional doses per 24 hours of compound (II). In some embodiments, the oxytocin antagonist is additionally administered to the subject following embryo transfer in 4 doses per 24 hours, such as 4 additional doses per 24 hours of compound (II). In some embodiments, the oxytocin antagonist is additionally administered to the subject following embryo transfer in 5 doses per 24 hours, such as 5 additional doses per 24 hours of compound (II). In some embodiments, the oxytocin antagonist is additionally administered to the subject following embryo transfer in 6 doses per 24 hours, such as 6 additional doses per 24 hours of compound (II). In some embodiments, the oxytocin antagonist is additionally administered to the subject following embryo transfer in 7 doses per 24 hours, such as 7 additional doses per 24 hours of compound (II).

When one or more additional doses of the oxytocin antagonist are administered to the subject following embryo transfer, administration of the oxytocin antagonist may terminate, for instance, within from about 1 hour to about 14 days, or more, following embryo transfer. For instance, administration of the oxytocin antagonist may terminate within about 1 hour, 2 hours, 3 hours, 4 hours, 5 hours, 6 hours, 7 hours, 8 hours, 9 hours, 10 hours, 11 hours, 12 hours, 24 hours, 36 hours, 48 hours, 60 hours, 72 hours, 84 hours, 96 hours, 108 hours, 120 hours, 132 hours, 144 hours, 156 hours, 168 hours, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, or more, following embryo transfer.

Thus, in some embodiments, the oxytocin antagonist is administered to the subject in additional daily doses following embryo transfer for about 1 day to about 14 days following embryo transfer. In some embodiments, the additional daily doses are administered to the subject for about 3 days to about 11 days following embryo transfer. In some embodiments, the additional daily doses are administered to the subject for 7 days following embryo transfer.

In some embodiments of any of the above aspects of the disclosure, the oxytocin antagonist is administered to the subject concurrently with the transfer of the one or more embryos to the uterus of the subject

In some embodiments, the oxytocin antagonist is administered to the subject concurrently with embryo transfer in a single dose.

In some embodiments, the oxytocin antagonist is administered to the subject in multiple doses beginning during embryo transfer (for instance, in multiple periodic doses) and continuing after embryo transfer, such as from 1 to 20 doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, beginning during embryo transfer and continuing following embryo transfer. For instance, in some embodiments, the oxytocin antagonist is administered to the subject in from 1 to 10 doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, beginning during embryo transfer and continuing following embryo transfer. In some embodiments, the oxytocin antagonist is administered to the subject in from 1 to 5 doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, beginning during embryo transfer and continuing following embryo transfer. In some embodiments, the oxytocin antagonist is administered to the subject in from 10 to 20 doses, for instance, per 12 hours, per 24 hours, per 36 hours, per 48 hours, per 60 hours, per 72 hours, per 84 hours, per 96 hours, per 108 hours, per 120 hours, per 132 hours, per 144 hours, per 156 hours, per 168 hours, or longer, beginning during embryo transfer and continuing following embryo transfer. In some embodiments, the oxytocin antagonist is administered to the subject in from 10 to 15 doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, beginning during embryo transfer and continuing following embryo transfer. In some embodiments, the oxytocin antagonist is administered to the subject in 1, 2, 3, 4, 5, 6, 7, 8, 9, 10, 11, 12, 13, 14, 15, 16, 17, 18, 19, 20, or more, doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer. In some embodiments, the oxytocin antagonist is administered to the subject beginning during embryo transfer and continuing following embryo transfer in up to 7 doses (e.g., 1, 2, 3, 4, 5, 6, or 7 doses) per 24 hours, such as in up to 7 x 100 mg doses per 24 hours of compound (II).

For example, in some embodiments, the oxytocin antagonist is first administered to the subject concurrently with the transfer of the one or more embryos to the uterus of the subject, and the oxytocin antagonist is subsequently administered to the subject in one or more additional doses within about 1 hour to about 24 hours following the transfer of the one or more embryos to the subject. For instance, in some embodiments, the oxytocin antagonist is administered to the subject in one or more additional doses within about 1 hour to about 12 hours following the transfer of the one or more embryos to the subject. In some embodiments, the oxytocin antagonist is administered to the subject in one or more additional doses within from about 12 hours to about 24 hours following the transfer of the one or more embryos to the subject. In some embodiments, the oxytocin antagonist is administered to the subject in one or more additional doses within from about 1 hour to about 10 hours following the transfer of the one or more embryos to the subject. In some embodiments, the oxytocin antagonist is administered to the subject in one or more additional doses within from about 1 hour to about 9 hours following the transfer of the one or more embryos to the subject. In some embodiments, the oxytocin antagonist is administered to the subject in one or more additional doses within from about 1 hour to about 8 hours following the transfer of the one or more embryos to the subject. In some embodiments, the oxytocin antagonist is administered to the subject in one or more additional doses within from about 1 hour to about 7 hours following the transfer of the one or more embryos to the subject. In some embodiments, the oxytocin antagonist is administered to the subject in one or more additional doses within from about 1 hour to about 6 hours following the transfer of the one or more embryos to the subject. In some embodiments, the oxytocin antagonist is administered to the subject in one or more additional doses within from about 1 hour to about 5 hours following the transfer of the one or more embryos to the subject. In some embodiments, the oxytocin antagonist is administered to the subject in one or more additional doses within from about 1 hour to about 4 hours following the transfer of the one or more embryos to the subject. In some embodiments, the oxytocin antagonist is administered to the subject in one or more additional doses within from about 2 hours to about 6 hours following the transfer of the one or more embryos to the subject. In some embodiments, the oxytocin antagonist is administered to the subject in one or more additional doses within from about 3 hours to about 5 hours following the transfer of the one or more embryos to the subject.

In some embodiments, the oxytocin antagonist is first administered to the subject concurrently with the transfer of the one or more embryos to the uterus of the subject, and the oxytocin antagonist is subsequently administered to the subject in one or more additional doses starting at about 1 hour, 2 hours, 3 hours, 4 hours, 5 hours, 6 hours, 7 hours, 8 hours, 9 hours, 10 hours, 11 hours, 12 hours, 24 hours, 36 hours, 48 hours, 60 hours, 72 hours, 84 hours, 96 hours, 108 hours, 120 hours, 132 hours, 144 hours, 156 hours, 168 hours, or more, following the transfer of the one or more embryos to the subject.

In some embodiments, the oxytocin antagonist is first administered to the subject concurrently with the transfer of the one or more embryos to the uterus of the subject, and the oxytocin antagonist is subsequently administered to the subject in multiple additional doses following embryo transfer, such as in from 1 to 20 additional doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer. In some embodiments, the oxytocin antagonist is additionally administered to the subject following embryo transfer in from 1 to 20 doses per 24 hours, such as 1 dose per 24 hours, 2 doses per 24 hours, 3 doses per 24 hours, 4 doses per 24 hours, 5 doses per 24 hours, 6 doses per 24 hours, 7 doses per 24 hours, 8 doses per 24 hours, 9 doses per 24 hours, 10 doses per 24 hours, 11 doses per 24 hours, 12 doses per 24 hours, 13 doses per 24 hours, 14 doses per 24 hours, 15 doses per 24 hours, 16 doses per 24 hours, 17 doses per 24 hours, 18 doses per 24 hours, 19 doses per 24 hours, 20 doses per 24 hours. In some embodiments, the oxytocin antagonist is additionally administered to the subject following embryo transfer in more than 20 doses per 24 hours.

For instance, in some embodiments, the oxytocin antagonist is first administered to the subject concurrently with the transfer of the one or more embryos to the uterus of the subject, and the oxytocin antagonist is subsequently administered to the subject in from 1 to 10 additional doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer. In some embodiments, the oxytocin antagonist is additionally administered to the subject following embryo transfer in from 1 to 10 doses per 24 hours, such as 1 dose per 24 hours, 2 doses per 24 hours, 3 doses per 24 hours, 4 doses per 24 hours, 5 doses per 24 hours, 6 doses per 24 hours, 7 doses per 24 hours, 8 doses per 24 hours, 9 doses per 24 hours, 10 doses per 24 hours.

In some embodiments, the oxytocin antagonist is first administered to the subject concurrently with the transfer of the one or more embryos to the uterus of the subject, and the oxytocin antagonist is subsequently administered to the subject in from 1 to 5 additional doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer. In some embodiments, the oxytocin antagonist is administered to the subject in from 10 to 20 additional doses, for instance, per 12 hours, per 24 hours, per 36 hours, per 48 hours, per 60 hours, per 72 hours, per 84 hours, per 96 hours, per 108 hours, per 120 hours, per 132 hours, per 144 hours, per 156 hours, per 168 hours, or longer, following embryo transfer. In some embodiments, the oxytocin antagonist is administered to the subject in from 10 to 15 additional doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer.

In some embodiments, the oxytocin antagonist is first administered to the subject concurrently with the transfer of the one or more embryos to the uterus of the subject, and the oxytocin antagonist is subsequently administered to the subject in 1, 2, 3, 4, 5, 6, 7, 8, 9, 10, 11, 12, 13, 14, 15, 16, 17, 18, 19, 20, or more, additional doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer.

In some embodiments, the oxytocin antagonist is first administered to the subject concurrently with the transfer of the one or more embryos to the uterus of the subject, and the oxytocin antagonist is subsequently administered to the subject following embryo transfer in up to 7 additional doses (e.g., 1, 2, 3, 4, 5, 6, or 7 doses) per 24 hours, such as in up to 7 x 100 mg doses per 24 hours of compound (II). In some embodiments, the oxytocin antagonist is additionally administered to the subject following embryo transfer in 1 dose per 24 hours, such as 1 additional dose per 24 hours of compound (II). In some embodiments, the oxytocin antagonist is additionally administered to the subject following embryo transfer in 2 doses per 24 hours, such as 2 additional doses per 24 hours of compound (II). In some embodiments, the oxytocin antagonist is additionally administered to the subject following embryo transfer in 3 doses per 24 hours, such as 3 additional doses per 24 hours of compound (II). In some embodiments, the oxytocin antagonist is additionally administered to the subject following embryo transfer in 4 doses per 24 hours, such as 4 additional doses per 24 hours of compound (II). In some embodiments, the oxytocin antagonist is additionally administered to the subject following embryo transfer in 5 doses per 24 hours, such as 5 additional doses per 24 hours of compound (II). In some embodiments, the oxytocin antagonist is additionally administered to the subject following embryo transfer in 6 doses per 24 hours, such as 6 additional doses per 24 hours of compound (II). In some embodiments, the oxytocin antagonist is additionally administered to the subject following embryo transfer in 7 doses per 24 hours, such as 7 additional doses per 24 hours of compound (II).

When one or more additional doses of the oxytocin antagonist are administered to the subject following embryo transfer, administration of the oxytocin antagonist may terminate, for instance, within from about 1 hour to about 14 days, or more, following embryo transfer. For instance, administration of the oxytocin antagonist may terminate within about 1 hour, 2 hours, 3 hours, 4 hours, 5 hours, 6 hours, 7 hours, 8 hours, 9 hours, 10 hours, 11 hours, 12 hours, 24 hours, 36 hours, 48 hours, 60 hours, 72 hours, 84 hours, 96 hours, 108 hours, 120 hours, 132 hours, 144 hours, 156 hours, 168 hours, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, or more, following embryo transfer.

Thus, in some embodiments, the oxytocin antagonist is first administered to the subject concurrently with the transfer of the one or more embryos to the uterus of the subject, and the oxytocin antagonist is subsequently administered to the subject in additional daily doses following embryo transfer for about 1 day to about 14 days following embryo transfer. In some embodiments, the additional daily doses are administered to the subject for about 3 days to about 11 days following embryo transfer. In some embodiments, the additional daily doses are administered to the subject for 7 days following embryo transfer.

In some embodiments of any of the above aspects of the invention, the oxytocin antagonist is administered to the subject following the transfer of the one or more embryos to the uterus of the subject

In some embodiments, the oxytocin antagonist is administered to the subject within about 1 hour to about 24 hours following the transfer of the one or more embryos to the subject. For instance, in some embodiments, the oxytocin antagonist is administered to the subject within about 1 hour to about 12 hours following the transfer of the one or more embryos to the subject. In some embodiments, the oxytocin antagonist is administered to the subject within from about 12 hours to about 24 hours following the transfer of the one or more embryos to the subject. In some embodiments, the oxytocin antagonist is administered to the subject within from about 1 hour to about 10 hours following the transfer of the one or more embryos to the subject. In some embodiments, the oxytocin antagonist is administered to the subject within from about 1 hour to about 9 hours following the transfer of the one or more embryos to the subject. In some embodiments, the oxytocin antagonist is administered to the subject within from about 1 hour to about 8 hours following the transfer of the one or more embryos to the subject. In some embodiments, the oxytocin antagonist is administered to the subject within from about 1 hour to about 7 hours following the transfer of the one or more embryos to the subject. In some embodiments, the oxytocin antagonist is administered to the subject within from about 1 hour to about 6 hours following the transfer of the one or more embryos to the subject. In some embodiments, the oxytocin antagonist is administered to the subject within from about 1 hour to about 5 hours following the transfer of the one or more embryos to the subject. In some embodiments, the oxytocin antagonist is administered to the subject within from about 1 hour to about 4 hours following the transfer of the one or more embryos to the subject. In some embodiments, the oxytocin antagonist is administered to the subject within from about 2 hours to about 6 hours following the transfer of the one or more embryos to the subject. In some embodiments, the oxytocin antagonist is administered to the subject within from about 3 hours to about 5 hours following the transfer of the one or more embryos to the subject.

For instance, in some embodiments, the oxytocin antagonist is administered to the subject about 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 36 hours, 48 hours, 60 hours, 72 hours, 84 hours, 96 hours, 108 hours, 120 hours, 132 hours, 144 hours, 156 hours, 168 hours, or more, following the transfer of the one or more embryos to the subject.

In some embodiments, the oxytocin antagonist is administered to the subject after embryo transfer in a single dose.

In some embodiments, the oxytocin antagonist is administered to the subject in multiple doses following embryo transfer, such as in multiple periodic doses. In some embodiments, the oxytocin antagonist is administered to the subject in from 1 to 20 doses following embryo transfer, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer. In some embodiments, the oxytocin antagonist is administered to the subject following embryo transfer in from 1 to 20 doses per 24 hours, such as 1 dose per 24 hours, 2 doses per 24 hours, 3 doses per 24 hours, 4 doses per 24 hours, 5 doses per 24 hours, 6 doses per 24 hours, 7 doses per 24 hours, 8 doses per 24 hours, 9 doses per 24 hours, 10 doses per 24 hours, 11 doses per 24 hours, 12 doses per 24 hours, 13 doses per 24 hours, 14 doses per 24 hours, 15 doses per 24 hours, 16 doses per 24 hours, 17 doses per 24 hours, 18 doses per 24 hours, 19 doses per 24 hours, 20 doses per 24 hours. In some embodiments, the oxytocin antagonist is administered to the subject following embryo transfer in more than 20 doses per 24 hours.

For instance, in some embodiments, the oxytocin antagonist is administered to the subject in from 1 to 10 doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer. In some embodiments, the oxytocin antagonist is administered to the subject following embryo transfer in from 1 to 10 doses per 24 hours, such as 1 dose per 24 hours, 2 doses per 24 hours, 3 doses per 24 hours, 4 doses per 24 hours, 5 doses per 24 hours, 6 doses per 24 hours, 7 doses per 24 hours, 8 doses per 24 hours, 9 doses per 24 hours, 10 doses per 24 hours.

In some embodiments, the oxytocin antagonist is administered to the subject in from 1 to 5 doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer. In some embodiments, the oxytocin antagonist is administered to the subject in from 10 to 20 doses, for instance, per 12 hours, per 24 hours, per 36 hours, per 48 hours, per 60 hours, per 72 hours, per 84 hours, per 96 hours, per 108 hours, per 120 hours, per 132 hours, per 144 hours, per 156 hours, per 168 hours, or longer, following embryo transfer. In some embodiments, the oxytocin antagonist is administered to the subject in from 10 to 15 doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer.

In some embodiments, the oxytocin antagonist is administered to the subject in 1, 2, 3, 4, 5, 6, 7, 8, 9, 10, 11, 12, 13, 14, 15, 16, 17, 18, 19, 20 doses, or more, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, following embryo transfer.

In some embodiments, the oxytocin antagonist is administered to the subject following embryo transfer in up to 7 doses (e.g., 1, 2, 3, 4, 5, 6, or 7 doses) per 24 hours, such as in up to 7 x 100 mg doses per 24 hours of compound (II). In some embodiments, the oxytocin antagonist is administered to the subject following embryo transfer in 1 dose per 24 hours, such as 1 dose per 24 hours of compound (II). In some embodiments, the oxytocin antagonist is administered to the subject following embryo transfer in 2 doses per 24 hours, such as 2 doses per 24 hours of compound (II). In some embodiments, the oxytocin antagonist is administered to the subject following embryo transfer in 3 doses per 24 hours, such as 3 doses per 24 hours of compound (II). In some embodiments, the oxytocin antagonist is administered to the subject following embryo transfer in 4 doses per 24 hours, such as 4 doses per 24 hours of compound (II). In some embodiments, the oxytocin antagonist is administered to the subject following embryo transfer in 5 doses per 24 hours, such as 5 doses per 24 hours of compound (II). In some embodiments, the oxytocin antagonist is administered to the subject following embryo transfer in 6 doses per 24 hours, such as 6 doses per 24 hours of compound (II). In some embodiments, the oxytocin antagonist is administered to the subject following embryo transfer in 7 doses per 24 hours, such as 7 doses per 24 hours of compound (II).

When the oxytocin antagonist is administered in multiple doses following embryo transfer, administration of the oxytocin antagonist may terminate, for instance, within from about 1 hour to about 14 days, or more, following embryo transfer. For instance, administration of the oxytocin antagonist may terminate within about 1 hour, 2 hours, 3 hours, 4 hours, 5 hours, 6 hours, 7 hours, 8 hours, 9 hours, 10 hours, 11 hours, 12 hours, 24 hours, 36 hours, 48 hours, 60 hours, 72 hours, 84 hours, 96 hours, 108 hours, 120 hours, 132 hours, 144 hours, 156 hours, 168 hours, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, or more, following embryo transfer.

Thus, in some embodiments, the oxytocin antagonist is administered to the subject in daily doses following embryo transfer for about 1 day to about 14 days following embryo transfer. In some embodiments, the daily doses are administered to the subject for about 3 days to about 11 days following embryo transfer. In some embodiments, the daily doses are administered to the subject for 7 days following embryo transfer.

In some embodiments of any of the above aspects of the disclosure, administration of the oxytocin antagonist to the subject reduces the likelihood of the subject having a miscarriage following the transfer of the one or more embryos to the subject.

In some embodiments, the sample is a blood sample.

In some embodiments, the embryo transfer therapy includes the transfer of from 1 to 2 embryos to the subject. In some embodiments, the embryo transfer therapy includes the transfer of 1 embryo to the subject. In some embodiments, the embryo transfer therapy includes the transfer of 2 embryos to the subject.

In some embodiments, the subject is a mammal and the one or more embryos are mammalian embryos. In some embodiments, the mammal is a human and the one or more embryos are human embryos.

In some embodiments, the one or more embryos are produced ex vivo by IVF, such as by IVF of one or more ova derived from the subject.

In some embodiments, the one or more embryos are produced ex vivo by ICSI, such as by ICSI into one or more ova derived from the subject.

In some embodiments, the one or more ova are derived from one or more oocytes isolated from the subject. In some embodiments, the one or more oocytes are isolated from the subject from about 1 day to about 7 days prior to the transfer of the one or more embryos to the subject. In some embodiments, the one or more oocytes are isolated from the subject about 2 days prior to the transfer of the one or more embryos to the subject. In some embodiments, the one or more oocytes are isolated from the subject about 3 days prior to the transfer of the one or more embryos to the subject. In some embodiments, the one or more oocytes are isolated from the subject about 4 days prior to the transfer of the one or more embryos to the subject. In some embodiments, the one or more oocytes are isolated from the subject about 5 days prior to the transfer of the one or more embryos to the subject.

In some embodiments, the one or more oocytes include from 1 to 4 mature oocytes (i.e., 1 to 4 ova).

In some embodiments, a GnRH antagonist is administered to the subject prior to isolation of the one or more oocytes (e.g., containing one or more mature oocytes) from the subject.

In some embodiments, hCG is administered to the subject prior to isolation of the one or more oocytes (e.g., containing one or more mature oocytes) from the subject, such as by a single intravenous injection, for instance, to induce final follicular maturation.

In some embodiments, progesterone is administered to the subject following isolation of the one or more oocytes from the subject. The progesterone may be administered intravaginally. In some embodiments, about 300 mg to about 600 mg of progesterone per dose is administered to the subject. In some embodiments, the progesterone is administered to the subject daily, such as beginning within about 24 hours of isolation of the one or more oocytes from the subject and continuing for about 6 or more weeks following the transfer of the one or more embryos to the subject.

In some embodiments, the one or more ova are isolated directly from the subject. In some embodiments, the one or more ova are isolated from the subject from about 1 day to about 7 days prior to the transfer of the one or more embryos to the subject. In some embodiments, the one or more ova are isolated from the subject about 2 days prior to the transfer of the one or more embryos to the subject. In some embodiments, the one or more ova are isolated from the subject about 3 days prior to the transfer of the one or more embryos to the subject. In some embodiments, the one or more ova are isolated from the subject about 4 days prior to the transfer of the one or more embryos to the subject. In some embodiments, the one or more ova are isolated from the subject about 5 days prior to the transfer of the one or more embryos to the subject.

In some embodiments, a GnRH antagonist is administered to the subject prior to isolation of the one or more ova from the subject, such as in a single intravenous injection.

In some embodiments, hCG is administered to the subject prior to isolation of the one or more ova from the subject, such as by a single intravenous injection, for instance, to induce final follicular maturation.

In some embodiments, progesterone is administered to the subject following isolation of the one or more ova from the subject. The progesterone may be administered intravaginally. In some embodiments, about 300 mg to about 600 mg of progesterone per dose is administered to the subject. In some embodiments, the progesterone is administered to the subject daily, such as beginning within about 24 hours of isolation of the one or more ova from the subject and continuing for about 6 or more weeks following the transfer of the one or more embryos to the subject.

In some embodiments, the one or more embryos are transferred to the subject during the same menstrual cycle as isolation of the one or more oocytes from the subject.

In some embodiments, the one or more embryos are transferred to the subject during the same menstrual cycle as isolation of the one or more ova from the subject.

In some embodiments, the one or more embryos are frozen and thawed prior to the transfer of the one or more embryos to the subject.

In some embodiments, the one or more embryos each contain from 6 to 8 blastomeres immediately prior to the transfer of the one or more embryos to the subject. The blastomeres may be of approximately equal sizes as assessed by visual microscopy prior to the transfer of the one or more embryos to the subject. In some embodiments, the one or more embryos comprise an embryo having the form of a morula. In some embodiments, the one or more embryos comprise an embryo having the form of a blastula (e.g., a mammalian blastocyst).

In some embodiments, the compound is (3Z,5S)-5-(hydroxymethyl)-1-[(2'-methyl-1,1'-biphenyl-4-yl)carbonyl]pyrrolidin-3-one O-methyloxime, represented by formula (II)

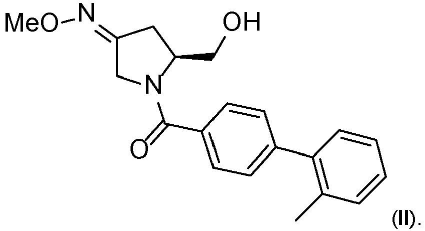

In some embodiments, the compound represented by formula (II) (i.e., (3Z,5S)-5-(hydroxymethyl)-1-[(2'-methyl-1,1'-biphenyl-4-yl)carbonyl]pyrrolidin-3-one O-methyloxime) is substantially pure. For instance, in some embodiments, the compound represented by formula (II) has a purity of at least 85%, such as a purity of from 85% to 99.9% or more (e.g., a purity of 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, or more). The purity of the compound represented by formula (II) may be assessed, for instance, using NMR techniques and/or chromatographic methods, such as HPLC procedures, that are known in the art and described herein, such as those techniques that are described in US Patent No. 9,670,155.

In some embodiments, the compound represented by formula (II) is substantially pure with respect to diastereomers of this compound and other by-products that may be formed during the synthesis of this compound. For instance, in some embodiments, the compound represented by formula (II) has a purity of at least 85%, such as a purity of from 85% to 99.9% or more (e.g., a purity of 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, or more) with respect to diastereomers of this compound and other by-products that may be formed during the synthesis of this compound, such as a by-product that is formed during the synthesis of this compound as described in US Patent No. 9,670,155. The purity of the compound represented by formula (II) may be assessed, for instance, using NMR techniques and/or chromatographic methods, such as HPLC procedures, that are known in the art and described herein, such as those techniques that are described in US Patent No. 9,670,155.

In some embodiments, the compound represented by formula (II) is substantially pure with respect to its (3E) diastereomer, (3*E*,5*S*)-5-(hydroxymethyl)-1-[(2'-methyl-1,1'-biphenyl-4-yl)carbonyl]pyrrolidin-3-one O-methyloxime. For instance, in some embodiments, the compound represented by formula (II) has a purity of at least 85%, such as a purity of from 85% to 99.9% or more (e.g., a purity of 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, or more) with respect to (3E,5S)-5-(hydroxymethyl)-1-[(2'-methyl-1,1'-biphenyl-4-yl)carbonyl]pyrrolidin-3-one O-methyloxime. For instance, compound (II) may be administered in the form of a composition (e.g., a tablet, such as a dispersible tablet, capsule, gel cap, powder, liquid solution, or liquid suspension) that contains less than 15% of the (3E) diastereomer. For example, compound (II) may be administered in the form of a composition (e.g., a tablet, such as a dispersible tablet, capsule, gel cap, powder, liquid solution, or liquid suspension) that contains less than 14%, less than 13%, less than 12%, less than 11%, less than 10%, less than 9%, less than 8%, less than 7%, less than 6%, less than 5%, less than 4%, less than 3%, less than 2%, less than 1%, less than 0.1%, less than 0.01%, less than 0.001%, or less of the (3E) diastereomer. The purity of the compound represented by formula (II) may be assessed, for instance, using NMR techniques and/or chromatographic methods, such as HPLC procedures, that are known in the art and described herein, such as those techniques that are described in US Patent No. 9,670,155.

In some embodiments, the compound is in a crystalline state. In some embodiments, the compound exhibits characteristic X-ray powder diffraction peaks at about 7.05° 2θ, about 13.13° 2θ, and about 23.34° 2θ. For instance, the compound may exhibits characteristic X-ray powder diffraction peaks at about 7.05° 2θ, about 12.25° 2θ, about 13.13° 2θ, about 16.54° 2θ, about 18.00° 2θ, about 21.84° 2θ, and about 23.34° 2θ. In some embodiments, the compound exhibits characteristic X-ray powder diffraction peaks as set forth in Table 1, above.

In some embodiments, the compound is administered orally to the subject. In some embodiments, the compound is administered intravenously to the subject. For instance, the compound may be administered to the subject in the form of a tablet, capsule, gel cap, powder, liquid solution, or liquid suspension. In some embodiments, the compound is administered to the subject in the form of a tablet, such as a dispersible tablet. The dispersible tablet may have, for example, one or more, or all, of the following components:
a. about 1-20% by weight of calcium silicate;
b. about 0.1-20% by weight of PVP30K;
c. about 0.01-5% by weight of poloxamer 188;
d. about 0.5-20% by weight of sodium croscarmellose;
e. about 1-90% by weight of microcrystalline cellulose 112;
f. about 1-90% by weight of lactose monohydrate;
g. about 0.01-0.5% by weight of sodium saccharine; and
h. about 0.1-10% by weight of glycerol dibehenate.

For instance, the dispersible tablet may have the following composition:
a. about 5% by weight of calcium silicate;
b. about 1% by weight of PVP30K;
c. about 2% by weight of poloxamer 188;
d. about 5% by weight of sodium croscarmellose;
e. about 1.5% by weight of microcrystalline cellulose 112;
f. about 47.8% by weight of lactose monohydrate;
g. about 0.2% by weight of sodium saccharine; and
h. about 4% by weight of glycerol dibehenate.

In some embodiments, the compound is administered to the subject in a unit dosage form containing from about 25 mg to about 250 mg of the compound, such as a unit dosage form containing about 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg, or more, of the compound. In some embodiments, the compound is administered to the subject in a unit dosage form containing from about 25 mg to about 75 mg of the compound, such as a unit dosage form containing about 50 mg of the compound. In some embodiments, the compound is administered to the subject in a unit dosage form containing from about 175 mg to about 225 mg of the compound, such as a unit dosage form containing about 200 mg of the compound.

In some embodiments, the subject is administered from about 50 mg to about 950 mg of the compound per dose. For instance, the subject may be administered about 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg, 305 mg, 310 mg, 315 mg, 320 mg, 325 mg, 330 mg, 335 mg, 340 mg, 345 mg, 350 mg, 355 mg, 360 mg, 365 mg, 370 mg, 375 mg, 380 mg, 385 mg, 390 mg, 400 mg, 405 mg, 410 mg, 415 mg, 420 mg, 425 mg, 430 mg, 435 mg, 440 mg, 445 mg, 450 mg, 455 mg, 460 mg, 465 mg, 470 mg, 475 mg, 480 mg, 485 mg, 490 mg, 500 mg, 505 mg, 510 mg, 515 mg, 520 mg, 525 mg, 530 mg, 535 mg, 540 mg, 545 mg, 555 mg, 560 mg, 565 mg, 570 mg, 575 mg, 580 mg,585 mg, 590 mg, 595 mg, 600 mg, 605 mg, 610 mg, 615 mg, 620 mg, 625 mg, 630 mg, 635 mg, 640 mg, 645 mg, 655 mg, 660 mg, 665 mg, 670 mg, 675 mg, 680 mg, 685 mg, 690 mg, 695 mg, 700 mg, 705 mg, 710 mg, 715 mg, 720 mg, 725 mg, 730 mg, 735 mg, 740 mg, 745 mg, 750 mg, 755 mg, 760 mg, 765 mg, 770 mg, 775 mg, 780 mg, 785 mg, 790 mg, 795 mg, 800 mg, 805 mg, 810 mg, 815 mg, 820 mg, 825 mg, 830 mg, 835 mg, 840 mg, 845 mg, 850 mg, 855 mg, 860 mg, 865 mg, 870 mg, 875 mg, 880 mg, 885 mg, 890 mg, 895 mg, 900 mg, 905 mg, 910 mg, 915 mg, 920 mg, 925 mg, 930 mg, 935 mg, 940 mg, 945 mg, 950 mg, or more, of the compound per dose.

In some embodiments, the subject is administered from about 50 mg to about 150 mg of the compound per dose, such as about 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, or 150 mg of the compound per dose. In some embodiments, the subject is administered about 100 mg of the compound per dose.

In some embodiments, the subject is administered from about 250 mg to about 350 mg of the compound per dose, such as about 250 mg, 255 mg, 260 mg, 265 mg, 270 mg, 275 mg, 280 mg, 285 mg, 290 mg, 295 mg, 300 mg, 305 mg, 310 mg, 315 mg, 320 mg, 325 mg, 330 mg, 335 mg, 340 mg, 345 mg, or 350 mg of the compound per dose. In some embodiments, the subject is administered about 300 mg of the compound per dose.

In some embodiments, the subject is administered from about 850 mg to about 950 mg of the compound per dose, such as about 850 mg, 855 mg, 860 mg, 865 mg, 870 mg, 875 mg, 880 mg, 885 mg, 890 mg, 895 mg, 900 mg, 905 mg, 910 mg, 915 mg, 920 mg, 925 mg, 930 mg, 935 mg, 940 mg, 945 mg, or 950 mg of the compound per dose. In some embodiments, the subject is administered about 900 mg of the compound per dose.

In some embodiments, the oxytocin antagonist is epelsiban, or a salt, derivative, variant, crystal form, or formulation thereof, such as a salt, derivative, variant, crystal form, or formulation described in US Patent No. 7,514,437; 8,367,673; 8,541,579; 7,550,462; 7,919,492; 8,202,864; 8,742,099; 9,408,851; 8,716,286; or 8,815,856.

In some embodiments, the oxytocin antagonist is retosiban, or a salt, derivative, variant, crystal form, or formulation thereof, such as a salt, derivative, variant, crystal form, or formulation described in US Patent No. 7,514,437; 8,367,673; 8,541,579; 8,071,594; 8,357,685; 8,937,179; or 9,452,169.

In some embodiments, the oxytocin antagonist is barusiban, or a salt, derivative, variant, crystal form, or formulation thereof, such as a salt, derivative, variant, crystal form, or formulation described in US Patent No. 6,143,722; 7,091,314; 7,816,489; or 9,579,305, or WO 2017/060339.

In some embodiments, the oxytocin antagonist is atosiban, or a salt, derivative, variant, crystal form, or formulation thereof, such as a salt, derivative, variant, crystal form, or formulation described in US Patent No. 4,504,469 or 4,402,942.

In some embodiments, the oxytocin antagonist is administered orally.

In some embodiments, the oxytocin antagonist is administered parenterally.

In some embodiments, the oxytocin antagonist is administered intravenously.

In some embodiments, the P4 reference level is from about 1.0 ng/ml to about 2.0 ng/ml. For instance, the P4 reference level may be 1.0 ng/ml, 1.1 ng/ml, 1.2 ng/ml, 1.3 ng/ml, 1.4 ng/ml, 1.5 ng/ml, 1.6 ng/ml, 1.7 ng/ml, 1.8 mg/ml, 1.9 ng/ml, or 2.0 ng/ml, among others. In some embodiments, the P4 reference level is 1.5 ng/ml.

In some embodiments, the sample is isolated from the subject from about 1 day to about 7 days prior to the transfer of the one or more embryos to the subject. For instance, in some embodiments, the sample is isolated from the subject about 2 days prior to the transfer of the one or more embryos to the subject. In some embodiments, the sample is isolated from the subject about 3 days prior to the transfer of the one or more embryos to the subject. In some embodiments, the sample is isolated from the subject about 4 days prior to the transfer of the one or more embryos to the subject. In some embodiments, the sample is isolated from the subject about 5 days prior to the transfer of the one or more embryos to the subject.

In some embodiments, the sample is isolated from the subject up to 24 hours prior (e.g., 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, or 24 hours prior) to isolation of one or more oocytes (e.g., containing one or more mature oocytes) from the subject. In some embodiments, the sample is isolated from the subject immediately prior to isolation of one or more oocytes from the subject.

In some embodiments, the sample is isolated from the subject up to 24 hours prior (e.g., 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, or 24 hours prior) to isolation of one or more ova from the subject. In some embodiments, the sample is isolated from the subject immediately prior to isolation of one or more ova from the subject.

In some embodiments, the sample is isolated from the subject within about 1 hour of administering hCG to the subject.

In some embodiments, the P4 reference level is from about 200 nM to about 400 nM. In some embodiments, the P4 reference level is 320 nM. In some embodiments, the sample is isolated from the subject up to 24 hours prior to transfer of the one or more embryos to the subject, such as from 1 hour to 24 hours prior to embryo transfer, from 1 hour to 12 hours prior to embryo transfer, from 1 hour to 8 hours prior to embryo transfer, from 1 hour to 4 hours prior to embryo transfer, or from immediately prior to embryo transfer to 1 hour prior to embryo transfer. In some embodiments, the sample is isolated from the subject immediately prior to transfer of the one or more embryos to the subject (i.e., up to 60 minutes prior to the scheduled transfer of one or more embryos to the subject).

In some embodiments, the subject exhibits an increase in endometrial and/or myometrial PGE2 expression following administration of the oxytocin antagonist to the subject, for instance, as assessed by mass spectrometric and/or spectroscopic techniques described herein or known in the art. In some embodiments, the subject exhibits an increase in endometrial and/or myometrial PGF2α expression following administration of the oxytocin antagonist to the subject, for instance, as assessed by mass spectrometric and/or spectroscopic techniques described herein or known in the art. In some embodiments, the subject exhibits a reduction in endometrial and/or myometrial PGF2α signaling following administration of the oxytocin antagonist, for instance, as assessed by detecting an increase in the concentration of PIP₂ and/or a decrease in the concentration of one or more secondary messengers involved in PGF2α signal transduction, such as DAG, IP₃, and/or intracellular Ca²⁺ released from Ca²⁺ stores, such as sarcoplasmic reticula. For instance, the subject may exhibit a transient increase in endometrial and/or myometrial PGF2α expression, followed by a reduction in PGF2α signalling in these tissues, as evidenced, for instance, by a reduction in endometrial and/or myometrial [DAG], [IP₃], and/or [Ca²⁺].

In some embodiments, the subject sustains pregnancy for at least about 14 days following the transfer of the one or more embryos to the subject, such as for about 14 days, 15 days, 16 days, 17 days, 18 days, 19 days, 20 days, 21 days, 22 days, 23 days, 24 days, 25 days, 26 days, 27 days, 28 days, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, or more, following the transfer of the one or more embryos to the subject. In some embodiments, the subject sustains pregnancy for at least about 6 weeks following the transfer of the one or more embryos to the subject, such as for about 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, or more. In some embodiments, the subject sustains pregnancy for at least about 10 weeks following the transfer of the one or more embryos to the subject and/or following the retrieval of one or more oocytes or ova from the subject, such as for about 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, or more, following the transfer of the one or more embryos to the subject and/or following the retrieval of one or more oocytes or ova from the subject.

In some embodiments, pregnancy is assessed by a blood pregnancy test, such as by detecting the presence and/or quantity of hCG in a blood sample isolated from the subject. In some embodiments, pregnancy is assessed by detecting intrauterine embryo heartbeat, for instance, at about 6 weeks or more (e.g., about 6 weeks following the transfer of the one or more embryos to the subject and/or following the retrieval of one or more oocytes or ova from the subject, such as for about 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, or more) following the transfer of the one or more embryos to the subject and/or following the retrieval of one or more oocytes or ova from the subject.

In some embodiments, the subject sustains pregnancy and exhibits a live birth following administration of the oxytocin antagonist to the subject. For instance, in some embodiments, the subject sustains pregnancy following administration of the oxytocin antagonist to the subject and exhibits a live birth at a gestational age of at least about 24 weeks, such as at a gestational age of about 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, or more.

### Definitions

As used herein, the term "about" refers to a value that is within 10% above or below the value being described. For instance, the phrase "about 50 mg" refers to a value between and including 45 mg and 55 mg.

As used herein, the term "affinity" refers to the strength of a binding interaction between two molecules, such as a ligand and a receptor. The term "Kᵢ", as used herein, is intended to refer to the inhibition constant of an antagonist for a particular molecule of interest, and can be expressed as a molar concentration (M). Kᵢ values for antagonist-target interactions can be determined, e.g., using methods established in the art. Methods that can be used to determine the Kᵢ of an antagonist for a molecular target include competitive binding experiments, such as competitive radioligand binding assays, for instance, as described in US Patent No. 9,670,155. The term "K_{d}", as used herein, is intended to refer to the dissociation constant, which can be obtained, for example, from the ratio of the rate constant for the dissociation of the two molecules (k_{d}) to the rate constant for the association of the two molecules (kₐ) and is expressed as a molar concentration (M). K_{d} values for receptor-ligand interactions can be determined, e.g., using methods established in the art. Methods that can be used to determine the K_{d} of a receptor-ligand interaction include surface plasmon resonance, e.g., through the use of a biosensor system such as a BIACORE® system.

As used herein, the term "assisted reproductive technology" or "ART" refers to a fertility treatment in which one or more female gametes (ova) and male gametes (sperm cells) are manipulated ex vivo so as to promote ovum fertilization and formation of a zygote or embryo. The zygote or embryo is then transferred to the uterus of a female subject, for instance, using the compositions and methods described herein. Exemplary assisted reproductive technology procedures include in vitro fertilization (IVF) and intracytoplasmic sperm injection (ICSI) techniques described herein and known in the art.

As used herein, the term "benefit' in the context of a subject undergoing embryo transfer therapy refers to any clinical improvement in the subject's condition or ability to undergo successful embryo implantation and development. Exemplary benefits in this context, such as in the context of a subject treated with an oxytocin antagonist prior to, concurrently with, and/or after the transfer of one or more embryos to the subject, include an increase in the subject's endometrial receptivity, as well as the prevention of a miscarriage in a subject following transfer of one or more embryos to the subject. A subject can be determined to benefit, for instance, from oxytocin antagonist treatment as described herein by observing an elevated endometrial receptivity in the subject (for instance, as assessed by detecting a reduction in prostaglandin F2α (PGF2α) signal transduction as described herein and/or by assessing the subject's ability to sustain a pregnancy for at least 14 days, 6 weeks, 10 weeks, or more, following the transfer of one or more embryos to the subject and/or following the retrieval of one or more oocytes or ova from the subject, and/or by detecting the ability of the subject to give birth to a live offspring at least 24 weeks following the transfer of one or more embryos to the subject. Additionally or alternatively, a subject can be determined to benefit from oxytocin antagonist treatment as described herein by monitoring the subject for a miscarriage following the transfer of one or more embryos to the subject and observing that the subject has not undergone a miscarriage.

As used herein, the term "concurrently with" in the context of administration of a therapeutic agent, such as an oxytocin antagonist described herein, during embryo transfer therapy describes a process in which the therapeutic agent is administered to a subject at substantially the same time as one or more embryos are transferred to the uterus of the subject. For instance, a therapeutic agent is considered to be administered to the subject concurrently with the transfer of one or more embryos if the therapeutic agent is administered to the subject within 1 hour or less (e.g., 60 minutes, 55 minutes, 50 minutes, 45 minutes, 40 minutes, 35 minutes, 30 minutes, 25 minutes, 20 minutes, 15 minutes, 10 minutes, 5 minutes, or less) of the transfer of the one or more embryos to the uterus of the subject.

As used herein, the term "controlled ovarian hyperstimulation" refers to a procedure in which ovulation is induced in a subject, such as a human subject, prior to oocyte or ovum retrieval for use in embryo formation, for instance, by in vitro fertilization (IVF) or intracytoplasmic sperm injection (ICSI). Controlled ovarian hyperstimulation procedures may involve administration of human chorionic gonadotropin (hCG) and/or a gonadotropin-releasing hormone (GnRH) antagonist to the subject so as to promote follicular maturation. Controlled ovarian hyperstimulation methods are known in the art and are described, for instance, in US Patent Nos. 7,405,197 and 7,815,912.

As used herein, the term "crystalline" or "crystalline form" means having a physical state that is a regular three-dimensional array of atoms, ions, molecules or molecular assemblies. Crystalline forms have lattice arrays of building blocks called asymmetric units that are arranged according to well-defined symmetries into unit cells that are repeated in three-dimensions. In contrast, the term "amorphous" or "amorphous form" refers to an unorganized (no orderly) structure. The physical state of a therapeutic compound may be determined by exemplary techniques such as x-ray diffraction, polarized light microscopy, thermal gravimetric analysis, and/or differential scanning calorimetry.

As used herein, the term "derived from" in the context of a cell derived from a subject refers to a cell, such as a mammalian ovum, that is either isolated from the subject or obtained from expansion, division, maturation, or manipulation (e.g., ex vivo expansion, division, maturation, or manipulation) of one or more cells isolated from the subject. For instance, an ovum is "derived from" a subject or an oocyte as described herein if the ovum is directly isolated from the subject or obtained from the maturation of an oocyte isolated from the subject, such as an oocyte isolated from the subject from about 1 day to about 7 days prior to the subject undergoing an embryo transfer procedure (e.g., an oocyte isolated from the subject from about 3 days to about 5 days prior to the subject undergoing an embryo transfer procedure).

As used herein, the term "dispersible tablet" refers to a tablet capable of rapidly disintegrating in water and that is swallowed by a subject, or that is intended to be disintegrated rapidly in water and subsequently swallowed by a subject, such as a subject undergoing embryo transfer therapy as described herein.

As used herein, the term "dose" refers to the quantity of a therapeutic agent, such as an oxytocin antagonist described herein, that is administered to a subject for the treatment of a disorder or condition, such as to enhance endometrial receptivity and promote successful embryo implantation in the context of assisted reproductive technology. A therapeutic agent as described herein may be administered in a single dose or in multiple doses. In each case, the therapeutic agent may be administered using one or more unit dosage forms of the therapeutic agent. For instance, a single dose of 100 mg of a therapeutic agent may be administered using, e.g., two 50 mg unit dosage forms of the therapeutic agent. Similarly, a single dose of 300 mg of a therapeutic agent may be administered using, e.g., six 50 mg unit dosage forms of the therapeutic agent or two 50 mg unit dosage forms of the therapeutic agent and one 200 mg unit dosage form of the therapeutic agent, among other combinations. Similarly, a single dose of 900 mg of a therapeutic agent may be administered using, e.g., six 50 mg unit dosage forms of the therapeutic agent and three 200 mg unit dosage forms of the therapeutic agent or ten 50 mg unit dosage form of the therapeutic agent and two 200 mg unit dosage forms of the therapeutic agent, among other combinations.

As used herein, the term "embryo" refers to a multicellular, post-zygotic derivative of a fertilized ovum. An embryo may contain two or more blastomeres. For instance, embryos for use with the compositions and methods of the invention include those that contain from 6 to 8 blastomeres. Embryos may be produced ex vivo, for instance, by in vitro fertilization (IVF) of an ovum, such as an ovum isolated from a subject undergoing embryo transfer therapy or from a donor, or an ovum produced by maturation of an oocyte isolated from a subject undergoing embryo transfer therapy or from a donor. Embryos may be produced ex vivo, for instance, by intracytoplasmic sperm injection (ICSI) of an ovum, such as an ovum isolated from a subject undergoing embryo transfer therapy or from a donor, or an ovum produced by maturation of an oocyte isolated from a subject undergoing embryo transfer therapy or from a donor. An embryo may have a variety of multicellular forms resulting from ovum fertilization and mitosis of the ensuing zygote. For instance, an embryo may have the form of a morula, which is typically formed from about 3 days to about 4 days following ovum fertilization, and contains two or more cells (such as from 2 to 16 cells, for instance, from 6 to 8 cells) packed contiguously in a spherical arrangement. An embryo may have the form of a blastula (e.g., a mammalian blastocyst), which is typically formed from about 5 days to about 7 days following ovum fertilization, characterized by a spherical morphology containing an outer lining of cells (e.g., a mammalian trophoblast or trophectoderm) surrounding an inner cell mass and a fluid-filled cavity (e.g., a mammalian blastocoele). A blastocyst may contain, for instance, from about 20 to about 300 cells (e.g., about 20 cells, 25 cells, 30 cells, 35 cells, 40 cells, 45 cells, 50 cells, 55 cells, 60 cells, 65 cells, 70 cells, 75 cells, 80 cells, 85 cells, 90 cells, 95 cells, 100 cells, 105 cells, 110 cells, 115 cells, 120 cells, 125 cells, 130 cells, 135 cells, 140 cells, 145 cells, 150 cells, 155 cells, 160 cells, 165 cells, 170 cells, 175 cells, 180 cells, 185 cells, 190 cells, 195 cells, 200 cells, 205 cells, 210 cells, 215 cells, 220 cells, 225 cells, 230 cells, 235 cells, 240 cells, 255 cells, 265 cells, 270 cells, 275 cells, 280 cells, 285 cells, 290 cells, 295 cells, or 300 cells) or more.

As used herein, the term "embryo transfer therapy" refers to a procedure in which one or more embryos are administered to the uterus of a subject, such as a mammalian subject (e.g., a human subject) so as to promote implantation of the one or more embryos into the endometrium of the subject. The embryo may be produced ex vivo, for instance, by in vitro fertilization (IVF) or by intracytoplasmic sperm injection (ICSI), optionally using one or more ova derived from the subject (e.g., one or more ova obtained from maturation of one or more oocytes isolated from the subject) or using one or more ova derived from a donor (e.g., one or more ova obtained from maturation of one or more oocytes isolated from a donor). The embryo may be freshly transferred to the subject, for example, by performing intrauterine embryo transfer using one or more embryos produced by fertilization within about 1 day to about 7 days, such as within about 3 days to about 5 days, of oocyte retrieval from the subject or donor. Embryo transfer is considered "fresh" when ovarian hyperstimulation and ovum/oocyte retrieval from the subject are performed during the same menstrual cycle as embryo transfer to the subject. Alternatively, the embryo may be cryopreserved for long-term storage and subsequently thawed prior to embryo transfer. This process is referred to herein as frozen embryo transfer (FET).

As used herein, the term "endogenous" describes a molecule (e.g., a polypeptide, nucleic acid, or cofactor) that is found naturally in a particular organism (e.g., a human) or in a particular location within an organism (e.g., an organ, a tissue, or a cell, such as a human cell).

As used herein, the term "endometrial receptivity" refers to the ability of the uterus to provide optimal conditions to promote proper implantation and development of an embryo, such as an embryo produced ex vivo by in vitro fertilization of, or intracytoplasmic sperm injection into, an ovum (e.g., an ovum obtained directly from a subject undergoing an embryo transfer procedure therapy or by maturation of one or more oocytes obtained from a subject undergoing an embryo transfer procedure, or an ovum obtained directly from a donor not undergoing an embryo transfer procedure or by maturation of one or more oocytes obtained from a donor not undergoing an embryo transfer procedure). Endometrial receptivity may be enhanced (i.e., increased) using the compositions and methods described herein, for instance, by administration of an oxytocin antagonist to a subject undergoing embryo transfer therapy prior to, concurrently with, and/or following the transfer of one or more embryos to the subject. Enhanced endometrial receptivity may manifest clinically in one or more ways. For instance, a subject exhibiting enhanced endometrial receptivity (e.g., in response to treatment with an oxytocin antagonist prior to, concurrently with, and/or following the transfer of one or more embryos to the subject) may exhibit decreased prostaglandin F2α (PGF2α) signaling in the subject's endometrial and/or myometrial tissue. For instance, a subject can be determined to exhibit enhanced endometrial receptivity in response to oxytocin antagonist administration if the subject demonstrates a reduced concentration of one or more secondary messengers involved in PGF2α signal transduction, such as diacylglycerol (DAG), inositol-1,4,5-trisphosphate (IP₃), and/or intracellular calcium (Ca²⁺) released from Ca²⁺ stores, such as sarcoplasmic reticula. For instance, a subject can be determined to exhibit enhanced endometrial receptivity in response to oxytocin antagonist treatment as described herein by detecting a decrease in the concentration of one or more of the foregoing secondary messengers in a tissue sample, cell sample, or blood sample isolated from the subject's endometrium and/or myometrium of 5%, 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, or more, relative to a measure of the secondary messenger prior to administration of the oxytocin antagonist. Enhanced endometrial receptivity in a subject undergoing embryo transfer therapy can also be observed by assessing the ability of the subject to sustain pregnancy for a period of time following embryo transfer to the uterus of the subject. For instance, a subject exhibiting enhanced endometrial receptivity in response to oxytocin antagonist therapy may sustain pregnancy for at least 14 days following transfer of one or more embryos to the subject, as assessed, for instance, by a blood pregnancy test, such as by detecting the presence and/or quantity of human chorionic gonadotropin (hCG) in a blood sample isolated from the subject using hCG tests known in the art and/or described herein. A subject exhibiting enhanced endometrial receptivity in response to oxytocin antagonist therapy may sustain pregnancy for at least 6 weeks, such as for 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, or 40 weeks, following transfer of one or more embryos to the subject and/or following the retrieval of one or more oocytes or ova from the subject, as assessed, for instance, by detecting intrauterine embryo heartbeat. A subject exhibiting enhanced endometrial receptivity in response to oxytocin antagonist therapy may give birth to a live offspring at a gestational age of at least 24 weeks, for instance, at a gestational age of 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, or 40 weeks.

As used herein, the term "exogenous" describes a molecule (e.g., a polypeptide, nucleic acid, or cofactor) that is not found naturally in a particular organism (e.g., a human) or in a particular location within an organism (e.g., an organ, a tissue, or a cell, such as a human cell). Exogenous materials include those that are provided from an external source to an organism or to cultured matter extracted there from.

As used herein, the term "gestational age" describes how far along a particular pregnancy is, and is measured from the first day of a pregnant female subject's last menstrual cycle to the current date. As used herein, the term "labor" (which may also be termed birth) relates to the expulsion of the fetus and placenta from the uterus of a pregnant female subject. For a normal pregnancy, labor may occur at a gestational age of about 40 weeks. "Preterm labor" as used herein refers to a condition in which labor commences more than three weeks before the full gestation period, which is typically about 40 weeks. That is, preterm labor occurs at any stage prior to, e.g., 38 weeks of gestation. Preterm labor typically leads to the occurrence of labor, or physiological changes associated with labor in a pregnant female subject, if not treated. Preterm labor may or may not be associated with vaginal bleeding or rupture of uterine membranes. Preterm labor may also be referred to as premature labor. The avoidance of preterm labor in a subject will prolong the term of pregnancy and may therefore avoid preterm delivery, thus reducing the risk of neonatal mortality and morbidity.

As used herein, the term "gonadotropin-releasing hormone antagonist" or "GnRH antagonist" refers to a compound capable of inhibiting the gonadotropin-releasing hormone receptor, e.g., such that release of one or more gonadotropins (such as follicle stimulating hormone and luteinizing hormone) is inhibited. GnRH antagonists include 2-phenylethylpyrimidine-2,4(1H,3H)-dione derivatives, such as those described in US Patent Nos. 7,056,927; 7,176,211; and 7,419,983. Exemplary GnRH antagonists include elagolix, relugolix, ASP-1707, and SKI2670, among others.

As used herein, the term "IC₅₀" refers to the concentration of a substance (antagonist) that reduces the efficacy of a reference agonist or the constitutive activity of a biological target by 50%, for instance, as measured in a competitive ligand binding assay or in a cell-based functional assay, such as a Ca²⁺ mobilization assay. Exemplary Ca²⁺ mobilization assays that can be used to determine the IC₅₀ of oxytocin antagonist include fluorimetric imaging assays, such as those described in US Patent No. 9,670,155.

As used herein, the term "in vitro fertilization" (IVF) refers to a process in which an ovum, such as a human ovum, is contacted ex vivo with one or more sperm cells so as to promote fertilization of the ovum and zygote formation. The ovum can be derived from a subject, such as a human subject, undergoing embryo transfer therapy. For instance, the ovum may be obtained from maturation of one or more oocytes isolated from the subject, e.g., from about 1 day to about 7 days prior to embryo transfer to the subject (such as from about 3 days to about 5 days prior to embryo transfer to the subject). The ovum may also be retrieved directly from the subject, for instance, by transvaginal ovum retrieval procedures known in the art. Alternatively, the ovum may be derived or isolated from a donor.

As used herein, the term "intracytoplasmic sperm injection" (ICSI) refers to a process in which a sperm cell is injected directly into an ovum, such as a human ovum, so as to promote fertilization of the ovum and zygote formation. The sperm cell may be injected into the ovum, for instance, by piercing the oolemma with a microinjector so as to deliver the sperm cell directly to the cytoplasm of the ovum. ICSI procedures useful in conjunction with the compositions and methods described herein are known in the art and are described, for instance, in WO 2013/158658, WO 2008/051620, and WO 2000/009674, among others.

As used herein, the term "miscarriage" refers to a naturally-occurring, spontaneous termination of a pregnancy at a stage in which the embryo or fetus is incapable of surviving independently of the mother. For instance, in human subjects, an embryo or fetus may be incapable of surviving independently of the mother at a gestational age of less than about 20 weeks (e.g., a gestational age of less than about 1 week, 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, or 20 weeks).

As used herein, the term "oral bioavailability" refers to the fraction of a compound administered to a subject, such as a mammal (e.g., a human) that reaches systemic circulation in the subject, and that is not sequestered in a non-target organ or excreted without absorption via the gastrointestinal tract. The term refers to a blood plasma concentration that is integrated over time and is typically expressed as a percentage of the orally administered dose.

As used herein, the terms "ovum" and "mature oocyte" refer to a mature haploid female reproductive cell or gamete. In the context of assisted reproductive technology as described herein, ova may be produced ex vivo by maturation of one or more oocytes isolated from a subject undergoing embryo transfer therapy. Ova may also be isolated directly from the subject, for example, by transvaginal ovum retrieval methods described herein or known in the art.

As used herein, the term "oxytocin antagonist" or "OT antagonist" refers to a compound capable of inhibiting the oxytocin receptor, for example, such that activity of one or more downstream signaling molecules in the oxytocin signal transduction cascade is inhibited. Oxytocin antagonists for use with the compositions and methods described herein include pyrrolidin-3-one oxime derivatives, such as those described in US Patent No. 7,115,754. For instance, oxytocin antagonists include (3Z,5S)-5-(hydroxymethyl)-1-[(2'-methyl-1,1'-biphenyl-4-yl)carbonyl]pyrrolidin-3-one O-methyloxime, as described, for instance, in US Patent No. 9,670,155. Additional examples of oxytocin antagonists include atosiban, retosiban, barusiban, and epelsiban, as well as derivatives thereof, among others. For instance, oxytocin antagonists that may be used in conjunction with the compositions and methods described herein include epelsiban, as well as salts, derivatives, variants, crystal forms, and formulations thereof, such as a salt, derivative, variant, crystal form, or formulation described in US Patent No. 7,514,437; 8,367,673; 8,541,579; 7,550,462; 7,919,492; 8,202,864; 8,742,099; 9,408,851; 8,716,286; or 8,815,856. Additional oxytocin antagonists that may be used in conjunction with the compositions and methods described herein include retosiban, as well as salts, derivatives, variants, crystal forms, and formulations thereof, such as a salt, derivative, variant, crystal form, or formulation described in US Patent No. 7,514,437; 8,367,673; 8,541,579; 8,071,594; 8,357,685; 8,937,179; or 9,452,169. Oxytocin antagonists useful in conjunction with the compositions and methods described herein further include barusiban, as well as salts, derivatives, variants, crystal forms, and formulations thereof, such as a salt, derivative, variant, crystal form, or formulation described in US Patent No. 6,143,722; 7,091,314; 7,816,489; or 9,579,305, or WO 2017/060339. Oxytocin antagonists useful in conjunction with the compositions and methods described herein additionally include atosiban, as well as salts, derivatives, variants, crystal forms, and formulations thereof, such as a salt, derivative, variant, crystal form, or formulation described in US Patent No. 4,504,469 or 4,402,942.

As used herein, the term "pharmaceutical composition" refers to a mixture containing a therapeutic compound, such as an oxytocin antagonist described herein, to be administered to a subject, such as a mammal, e.g., a human, in order to prevent, treat or control a particular disease or condition affecting or that may affect the mammal, such as to reduce the likelihood of embryo implantation failure in a subject undergoing embryo transfer therapy.

As used herein, the term "pharmaceutically acceptable" refers to those compounds, materials, compositions and/or dosage forms, which are suitable for contact with the tissues of a subject, such as a mammal (e.g., a human) without excessive toxicity, irritation, allergic response and other problem complications commensurate with a reasonable benefit/risk ratio.

As used herein, the term "probe" refers to an agent, such as an antibody, capable of specifically binding to, and detecting the presence of, an analyte of interest. Exemplary probes for use in the detection of progesterone include monoclonal antibodies described herein and known in the art, such as those produced and released by ATCC Accession Number HB 8886 as described in US Patent No. 4,720,455.

As used herein, the term "prostaglandin F2α signaling" or "PGF2α signaling" refers to the endogenous signal transduction cascade by which PGF2α potentiates the intracellular activity of the PGF2α receptor so as to effect one or more biological responses. PGF2α signaling encompasses the PGF2α-mediated stimulation of the PGF2α receptor (FP), a G protein-coupled receptor, which leads to the activation of the G_{q} protein and, in turn phospholipase C (PLC), phosphatidylinositol-3-kinase (PI3K), and extracellular signal-regulated kinases (ERK) 1 and 2. PGF2α signaling can be detected by observing an increase in the concentration of phosphatidylinsolitol-4,5-bisphosphate (PIP₂) and/or a decrease in the concentration of one or more secondary messengers involved in PGF2α signal transduction, such as diacylglycerol (DAG), inositol-1,4,5-trisphosphate (IP₃), and/or intracellular calcium (Ca²⁺) released from Ca²⁺ stores, such as sarcoplasmic reticula. The PGF2α signal transduction cascade is described in detail, for instance, in Xu et al., Reproduction 149:139-146 (2015).

As used herein, the terms "progesterone reference level" and "P4 reference level" refer to a concentrations of progesterone present within a mammalian subject (e.g., a human subject undergoing an embryo transfer procedure) or within a sample isolated therefrom (such as a serum sample) that, below which, indicates that the subject is likely to benefit from oxytocin antagonist treatment prior to, concurrently with, and/or following the transfer of one or more embryos to the uterus of the subject. P4 reference levels, as described herein, may have different values depending on the point in time during which the serum progesterone level of the patient is assessed. For instance, a P4 reference level of about 320 nM may be used in conjunction with the compositions and methods described herein when being compared to the concentration of P4 present in the serum of a human subject on the day of the embryo transfer procedure. In another example, a P4 reference level of about 1.5 ng/ml may be used in conjunction with the compositions and methods described herein when being compared to the concentration of P4 present in the serum of a human subject the day of oocyte or ovum retrieval from the subject.

As used herein, the term "sample" refers to a specimen (e.g., blood, blood component (e.g., serum or plasma), urine, saliva, amniotic fluid, cerebrospinal fluid, tissue (e.g., placental or dermal), pancreatic fluid, chorionic villus sample, and/or cells) isolated from a subject.

As used herein, the phrases "specifically binds" and "binds" refer to a binding reaction which is determinative of the presence of a particular protein in a heterogeneous population of proteins and other biological molecules that is recognized, e.g., by a ligand with particularity. A ligand (e.g., a protein, peptide, or small molecule) that specifically binds to a protein will bind to the protein, e.g., with a K_{D} of less than 100 nM. For example, a ligand that specifically binds to a protein may bind to the protein with a K_{D} of up to 100 nM (e.g., between 1 pM and 100 nM). A ligand that does not exhibit specific binding to a protein or a domain thereof may exhibit a K_{D} of greater than 100 nM (e.g., greater than 200 nM, 300 nM, 400 nM, 500 nM, 600 nm, 700 nM, 800 nM, 900 nM, 1 µM, 100 µM, 500 µM, or 1 mM) for that particular protein or domain thereof. A variety of assay formats may be used to determine the affinity of a ligand for a specific protein. For example, solid-phase ELISA assays are routinely used to identify ligands that specifically bind a target protein. See, e.g., Harlow & Lane, Antibodies, A Laboratory Manual, Cold Spring Harbor Press, New York (1988) and Harlow & Lane, Using Antibodies, A Laboratory Manual, Cold Spring Harbor Press, New York (1999), for a description of assay formats and conditions that can be used to determine specific protein binding.

As used herein, the terms "subject" and "patient" are interchangeable and refer to an organism that receives treatment for a particular disease or condition as described herein. Examples of subjects and patients include mammals, such as humans, receiving treatment for diseases or conditions, such as to reduce the likelihood of embryo implantation failure before, during, or after embryo transfer therapy sex hormone-dependent diseases.

As used herein, the term "substantially pure" refers to a compound that has a purity of at least 85%, as assessed, for instance, using nuclear magnetic resonance (NMR) and/or high-performance liquid chromatography (HPLC) techniques described herein or known in the art.

As used herein, the term "tₘₐₓ" refers to the time following administration of a compound to a subject at which the compound exhibits a maximum concentration in the blood (e.g., serum or plasma) of the subject.

A compound, salt form, crystal polymorph, therapeutic agent, or other composition described herein may be referred to as being characterized by graphical data "substantially as depicted in" a figure. Such data may include powder X-ray diffractograms, NMR spectra, differential scanning calorimetry curves, and thermogravimetric analysis curves, among others. As is known in the art, such graphical data may provide additional technical information to further define the compound, salt form, crystal polymorph, therapeutic agent, or other composition. As is understood by one of skill in the art, such graphical representations of data may be subject to small variations, e.g., in peak relative intensities and peak positions due to factors such as variations in instrument response and variations in sample concentration and purity. Nonetheless, one of skill in the art will readily be capable of comparing the graphical data in the figures herein with graphical data generated for a compound, salt form, crystal polymorph, therapeutic agent, or other composition and confirm whether the two sets of graphical data are characterizing the same material or two different materials. For instance, a crystal form of (3Z,5S)-5-(hydroxymethyl)-1-[(2'-methyl-1,1'-biphenyl-4-yl)carbonyl]pyrrolidin-3-one O-methyloxime referred to herein as being characterized by graphical data "substantially as depicted in" a figure will thus be understood to include any crystal form of (3Z,5*S*)-5-(hydroxymethyl)-1-[(2'-methyl-1,1'-biphenyl-4-yl)carbonyl]pyrrolidin-3-one O-methyloxime characterized by the graphical data, optionally having one or more of small variations, e.g., one or more variations described above or known to one of skill in the art.

As used herein, the terms "treat" or "treatment" in the context of a subject undergoing embryo transfer therapy refer to treatment, for instance, by administration of an oxytocin antagonist, with the intention of enhancing endometrial receptivity thereby reducing the likelihood of embryo implantation failure and promoting pregnancy in the subject. Those in need of treatment include, for example, female mammalian subjects, such as female human subjects, that are undergoing embryo transfer therapy, such as subjects undergoing oocyte or ovum retrieval followed by in vitro fertilization or intracytoplasmic sperm injection and subsequent embryo transfer. Those in need of treatment also include, for example, female mammalian subjects, such as female human subjects, that are undergoing embryo transfer therapy, for example, using embryos produced ex vivo by in vitro fertilization or intracytoplasmic sperm injections of one or more ova derived from a donor (e.g., isolated directly from a donor by transvaginal ovum retrieval or by maturation of one or more oocytes obtained directly from the donor). The subject may be undergoing fresh embryo transfer or frozen embryo transfer, and may be transferred, for instance, one, two, three, or more embryos according to the methods described herein. The subject may be one that has previously undergone embryo transfer therapy, either successfully or unsuccessfully, including subjects that have previously undergone one or more cycles (for instance, one, two, three, four, five, six, seven, eight, nine, ten, or more cycles) of failed embryo transfer therapy. A subject can be considered to have been treated, for instance, by administration of an oxytocin antagonist according to the methods described herein, if the subject exhibits endometrial receptivity following administration of the therapeutic agent. Endometrial receptivity can be observed in a variety of clinical manifestations, including a reduction in prostaglandin F2α (PGF2α) signal transduction following oxytocin antagonist administration, successful implantation of the embryo into the endometrium of the subject, as well as the subject's capacity to achieve and sustain pregnancy following embryo transfer, such as for about 14 days, 15 days, 16 days, 17 days, 18 days, 19 days, 20 days, 21 days, 22 days, 23 days, 24 days, 25 days, 26 days, 27 days, 28 days, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, or more, following the transfer of one or more embryos to the subject and/or following the retrieval of one or more oocytes or ova from the subject. Pregnancy can be assessed using methods described herein or known in the art, such as by detecting and/or quantifying human chorionic gonadotropin (hCG) in a blood sample isolated from the subject and/or by detecting intrauterine embryo heartbeat.

As used herein, the term "unit dosage form" refers to a single composition containing a therapeutic agent, such as an oxytocin antagonist described herein, formulated in a manner appropriate for administration to a subject, such as a subject undergoing embryo transfer therapy as described herein. Unit dosage forms include solid and liquid formulations, such as tablets (e.g., dispersible tablets), capsules, gel caps, powders, liquid solutions, and liquid suspensions. A subject may be administered a single dose of a therapeutic agent by administration of one or more unit dosage forms. For instance, a single dose of 100 mg of a therapeutic agent can be administered using two 50 mg unit dosage forms of the therapeutic agent.

As used herein, the term "acyl" refers to the chemical moiety -C(O)R in which R is C₁-C₆ alkyl, aryl, heteroaryl, C₁-C₆ alkyl aryl, or C₁-C₆ alkyl heteroaryl.

As used herein, the term "acylamino" refers to the chemical moiety -NRC(O)R' in which each of R and R' is independently hydrogen, C₁-C₆-alkyl, aryl, heteroaryl, C₁-C₆ alkyl aryl, or C₁-C₆ alkyl heteroaryl.

As used herein, the term "acyloxy" refers to the chemical moiety -OC(O)R in which R is C₁-C₆ alkyl, aryl, heteroaryl, C₁-C₆ alkyl aryl, or C₁-C₆ alkyl heteroaryl.

As used herein, the term "alkoxy" refers to the chemical moiety -O-R in which R is C₁-C₆ alkyl, aryl, heteroaryl, C₁-C₆ alkyl aryl, or C₁-C₆ alkyl heteroaryl. Exemplary alkoxy groups include methoxy, ethoxy, phenoxy, and the like.

As used herein, the term "alkoxycarbonyl" refers to the chemical moiety -C(O)OR in which R is hydrogen, C₁-C₆ alkyl, aryl, heteroaryl, C₁-C₆ alkyl aryl, or C₁-C₆ alkyl heteroaryl.

As used herein, the term "amino" refers to the chemical moiety -NRR' in which each of R and R' is independently hydrogen, C₁-C₆ alkyl, aryl, heteroaryl, C₁-C₆ alkyl aryl, C₁-C₆ alkyl heteroaryl, cycloalkyl, or heterocycloalkyl, or R and R', together with the nitrogen atom to which they are bound, can optionally form a 3-8-membered heterocycloalkyl ring.

As used herein, the term "aminocarbonyl" refers to the chemical moiety -C(O)NRR' in which each of R and R' is independently hydrogen, C₁-C₆ alkyl, aryl, heteroaryl, C₁-C₆ alkyl aryl, or C₁-C₆ alkyl heteroaryl.

As used herein, the term "aryl" refers to an unsaturated aromatic carbocyclic group of from 6 to 14 carbon atoms having a single ring (e.g., optionally substituted phenyl) or multiple condensed rings (e.g., optionally substituted naphthyl). Exemplary aryl groups include phenyl, naphthyl, phenanthrenyl, and the like. As used herein, the term "aryl" includes substituted aryl substituents, such as an aryl moiety containing a C₁-C₆ alkyl, C₂-C₆ alkenyl, C₂-C₆ alkynyl, cycloalkyl, heterocycloalkyl, C₁-C₆ alkyl aryl, C₁-C₆ alkyl heteroaryl, C₁-C₆ alkyl cycloalkyl, C₁-C₆-alkyl heterocycloalkyl, amino, ammonium, acyl, acyloxy, acylamino, aminocarbonyl, alkoxycarbonyl, ureido, carbamate, aryl, heteroaryl, sulfinyl, sulfonyl, alkoxy, sulfanyl, halogen, carboxy, trihalomethyl, cyano, hydroxy, mercapto, or nitro substituent, or the like. Exemplary substituted aryl groups include biphenyl and substituted biphenyl substituents.

As used herein, the term "C₁-C₆ alkyl" refers to an optionally branched alkyl moiety having from 1 to 6 carbon atoms, such as methyl, ethyl, propyl, isopropyl, butyl, isobutyl, sec-butyl, tert-butyl, pentyl, isopentyl, neopentyl, tert-pentyl, hexyl, and the like.

As used herein, the term "C₂-C₆ alkenyl" refers to an optionally branched alkenyl moiety having from 2 to 6 carbon atoms, such as vinyl, allyl, 1-propenyl, isopropenyl, 1-butenyl, 2-butenyl, 2-methylallyl, and the like.

As used herein, the term "C₂-C₆ alkynyl" refers to an optionally branched alkynyl moiety having from 2 to 6 carbon atoms, such as ethynyl, 2-propynyl, and the like.

As used herein, the term "carboxy" refers to the chemical moiety -C(O)OH, as well as the ionized form thereof, -C(O)O-, and salts thereof.

As used herein, the term "cycloalkyl" refers to a monocyclic cycloalkyl group having, for instance, from 3 to 8 carbon atoms, such as cyclopropyl, cyclobutyl, cyclopentyl, cyclohexyl, cycloheptyl, cyclooctyl, and the like.

As used herein, the term "halogen" refers to a fluorine atom, a chlorine atom, a bromine atom, or an iodine atom.

As used herein, the term "heteroaryl" refers to a monocyclic heteroaromatic, or a bicyclic or a tricyclic fused-ring heteroaromatic group. Exemplary heteroaryl groups include optionally substituted pyridyl, pyrrolyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadia-zolyl, 1,2,5-oxadiazolyl, 1,3,4-oxadiazolyl, 1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo[1,2-a]pyridyl, benzothiazolyl, benzoxazolyl, quinolizinyl, quinazolinyl, pthalazinyl, quinoxalinyl, cinnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl, benzoquinolyl, and the like.

As used herein, the term "heterocycloalkyl" refers to a 3 to 8-membered heterocycloalkyl group having one or more heteroatoms, such as a nitrogen atom, an oxygen atom, a sulfur atom, and the like, and optionally having one or more oxo groups. Exemplary heterocycloalkyl substituents include pyrrolidinyl, piperidinyl, oxopiperidinyl, morpholinyl, piperazinyl, 1-methylpiperazinyl, oxopiperazinyl, thiomorpholinyl, azepanyl, diazepanyl, oxazepanyl, thiazepanyl, dioxothiazepanyl, azokanyl, tetrahydrofuranyl, tetrahydropyranyl, and the like.

As used herein, the term "sulfanyl" refers to the chemical moiety -S-R in which R is C₁-C₆ alkyl, aryl, heteroaryl, C₁-C₆ alkyl aryl, or C₁-C₆ alkyl heteroaryl. Exemplary sulfanyl groups include methylsulfanyl, ethylsulfanyl, and the like.

As used herein, the term "sulfinyl" refers to the chemical moiety -S(O)-R in which R is hydrogen, C₁-C₆ alkyl, C₁-C₆ alkyl substituted with one or more halogens, such as a -SO-CF₃ substituent, aryl, heteroaryl, C₁-C₆ alkyl aryl, or C₁-C₆ alkyl heteroaryl.

As used herein, the term "sulfonyl" refers to chemical moiety -SO₂-R in which R is hydrogen, aryl, heteroaryl, C₁-C₆ alkyl, C₁-C₆ alkyl substituted with one or more halogens, such as a -SO₂₋CF₃ substituent, C₁-C₆ alkyl aryl, or C₁-C₆ alkyl heteroaryl.

As used herein, the term "sulfonylamino" refers to the chemical moiety -NRSO₂-R' in which each of R and R' is independently hydrogen, C₁-C₆ alkyl, aryl, heteroaryl, C₁-C₆ alkyl aryl, or C₁-C₆ alkyl heteroaryl.

As used herein, the term "sulfonyloxy" refers to the chemical moiety -OSO₂₋R in which R is hydrogen, C₁-C₆ alkyl, C₁-C₆ alkyl substituted with one or more halogens, such as a -OSO₂-CF₃ substituent, aryl, heteroaryl, C₁-C₆ alkyl aryl, or C₁-C₆ alkyl heteroaryl.

As used herein, the term "ureido" refers to the chemical moiety -NRC(O)NR'R" where each of R, R', and R" is independently hydrogen, C₁-C₆ alkyl, C₂-C₆ alkenyl, C₂-C₆ alkynyl, C₃-C₈ cycloalkyl, heterocycloalkyl, aryl, heteroaryl, C₁-C₆ alkyl aryl, C₁-C₆ alkyl heteroaryl, C₂-C₆ alkenyl aryl, C₂-C₆ alkenyl heteroaryl, C₂-C₆ alkynyl aryl, C₂-C₆ alkynyl heteroaryl, C₁-C₆ alkyl cycloalkyl, or C₁-C₆ alkyl heterocycloalkyl, or R' and R", together with the nitrogen atom to which they are bound, can optionally form a 3-8-membered heterocycloalkyl ring.

Unless otherwise constrained by the definition of the individual substituent, the foregoing chemical moieties, such as "alkyl", "alkenyl", "alkynyl", "aryl," and "heteroaryl" groups can optionally be substituted with, for example, from 1 to 5 substituents selected from the group consisting of C₁-C₆ alkyl, C₂-C₆ alkenyl, C₂-C₆ alkynyl, cycloalkyl, heterocycloalkyl, C₁-C₆ alkyl aryl, C₁-C₆ alkyl heteroaryl, C₁-C₆ alkyl cycloalkyl, C₁-C₆-alkyl heterocycloalkyl, amino, ammonium, acyl, acyloxy, acylamino, aminocarbonyl, alkoxycarbonyl, ureido, carbamate, aryl, heteroaryl, sulfinyl, sulfonyl, alkoxy, sulfanyl, halogen, carboxy, trihalomethyl, cyano, hydroxy, mercapto, nitro, and the like. The substitution may include situations in which neighboring substituents have undergone ring closure, such as ring closure of vicinal functional substituents, to form, for instance, lactams, lactones, cyclic anhydrides, acetals, hemiacetals, thioacetals, aminals, and hemiaminals, formed by ring closure, for example, to furnish a protecting group.

### Brief Description of the Figures

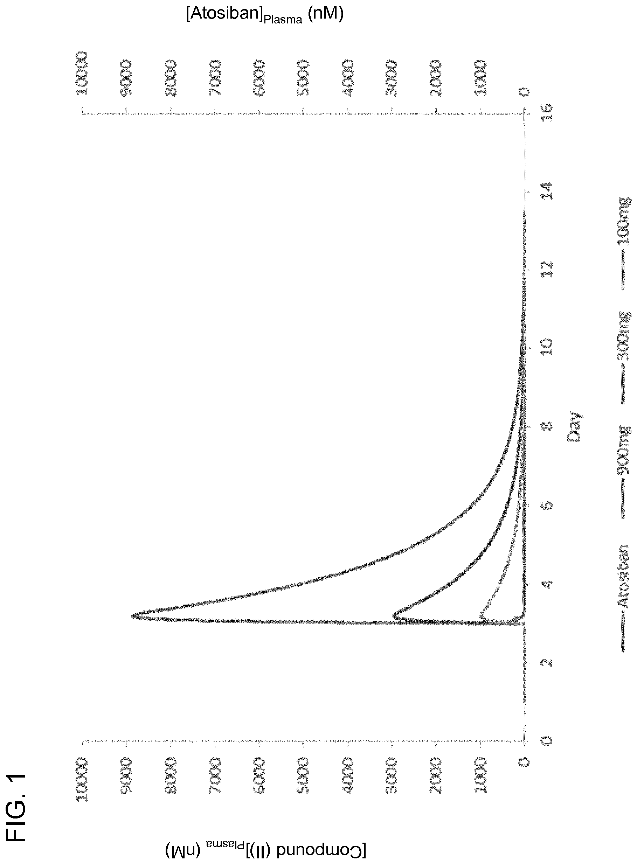

**Figure 1** is a graph showing calculated plasma concentrations of compound (II) following administration of 100 mg (third curve from the top), 300 mg (second curve from the top), and 900 mg (first curve from the top) of this compound to a human subject three days following oocyte retrieval from the subject in preparation for embryo transfer therapy. These pharmacokinetic profiles are contrasted with the calculated plasma concentration of atosiban (first curve from the bottom) in a human subject following administration of atosiban three days after oocyte retrieval in preparation for embryo transfer therapy. The indicated doses of compound (II) were administered orally to the human subject. Atosiban was administered to the human subject intravenously as a 6.75 mg bolus infusion, followed by an 18 mg/hr infusion for 0-1 hours and a subsequent 6 mg/hr infusion for 1-3 hours.

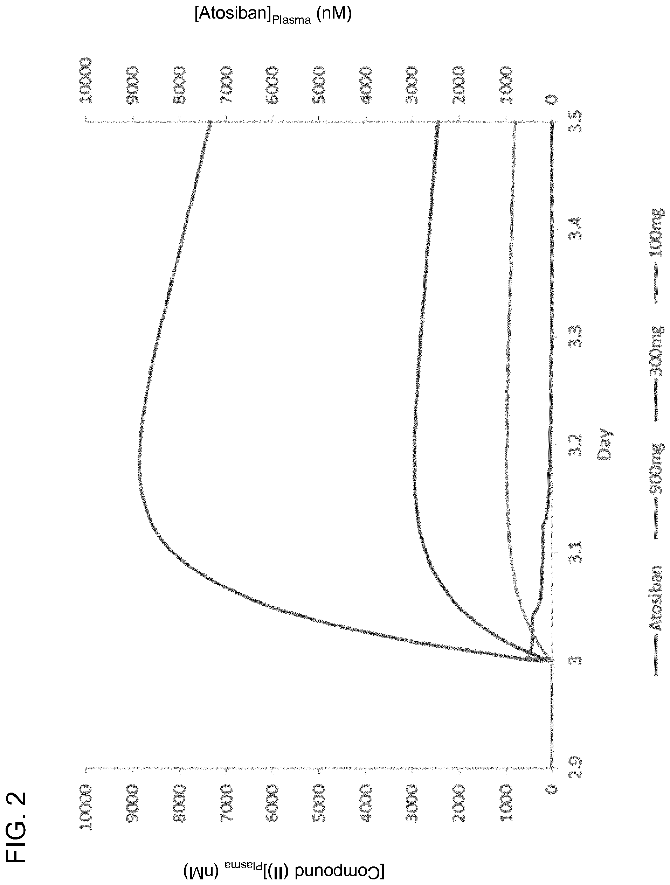

**Figure 2** is a magnified representation of the calculated pharmacokinetic profiles shown in Figure 1. For clarity, the x-axis is restricted to values from 2.9 days to 3.5 days following oocyte retrieval.

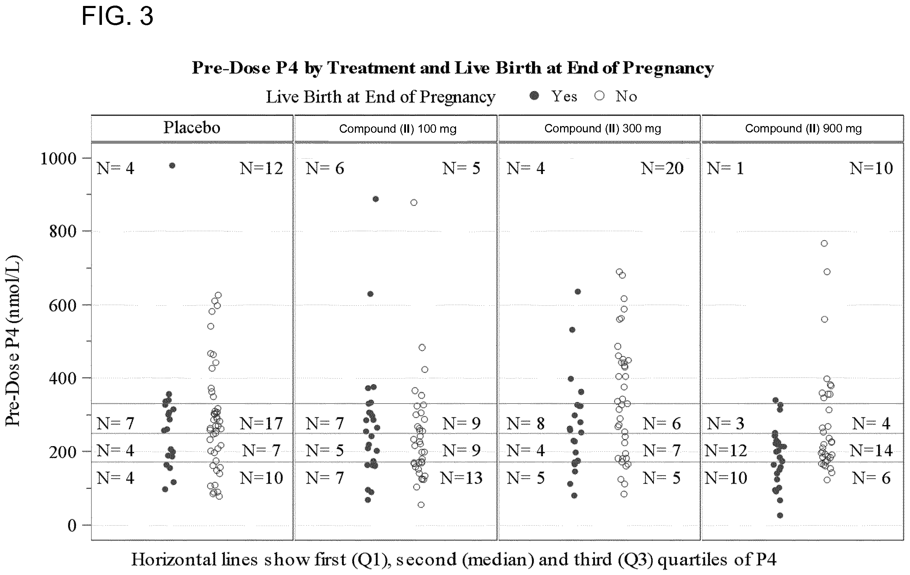

**Figure 3** is a chart showing the quantity of human subjects that did (filled-in circles) and did not (empty circles) exhibit a live birth at the end of pregnancy following treatment with placebo (left column) or 100 mg, 300 mg, or 900 mg of compound (II) (first, second, and third columns on the right, respectively) about 4 hours prior to embryo transfer as described in Example 1, below. The quantity of subjects that did and did not exhibit a live birth are plotted in relation to each subject's pre-treatment serum progesterone concentration on the day of embryo transfer, shown on the y-axis in units of nM. Horizontal lines through each column designate the first (25^{th} percentile), second (median), and third (75^{th} percentiles) quartiles of pre-dose serum progesterone concentrations on the day of embryo transfer among all subjects.

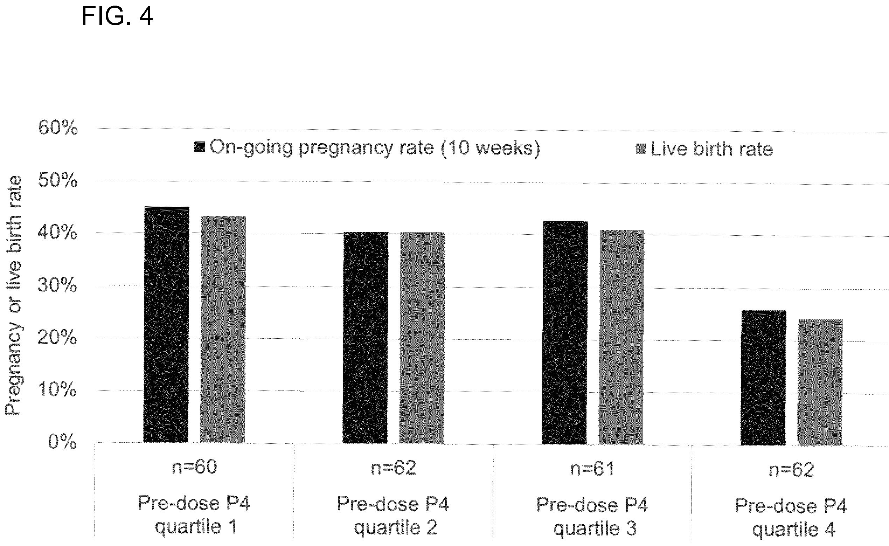

**Figure 4** is a graph showing the percentage of human subjects that tested positive for ongoing pregnancy at 10 weeks following oocyte retrieval (black bars) and subjects that exhibited a live birth at a gestational age of at least 24 weeks (grey bars) in the study descried in Example 1. The proportions of subjects that demonstrated these characteristics are plotted as a function of pre-treatment serum progesterone concentration quartile as measured on the day of embryo transfer, which is shown on the x-axis.

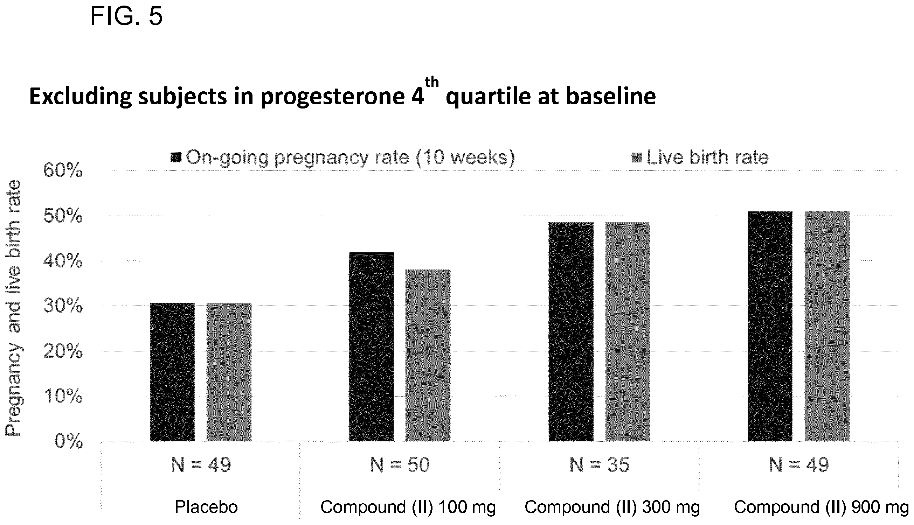

**Figure 5** is a graph showing the percentage of human subjects that tested positive for ongoing pregnancy at 10 weeks following oocyte retrieval (black bars) and subjects that exhibited a live birth at a gestational age of at least 24 weeks (grey bars) in the study descried in Example 1. The proportions plotted in Figure 5 exclude data from subjects that exhibited a pre-treatment serum progesterone concentration on the day of embryo transfer in the upper quartile of this metric.

### Detailed Description

The disclosure features compositions and methods for use in conjunction with assisted reproductive technology. For instance, the compositions and methods described herein can be used to treat subjects undergoing embryo transfer therapy by administering to the subject an oxytocin antagonist so as to enhance the endometrial receptivity of the subject and to reduce the likelihood of embryo implantation failure. The compositions and methods described herein can similarly reduce the likelihood of miscarriage in a subject that has undergone embryo transfer therapy. Using the methods described herein, an oxytocin antagonist can be administered to the subject before, during, and/or after the transfer of one or more embryos to the uterus of the subject so as to promote successful embryo implantation and a sustained pregnancy. The oxytocin antagonist can be administered in a single dose or in multiple doses, such as doses of varying strength or repeat doses of the same strength. For instance, the oxytocin antagonist may be administered in a single high dose or in multiple, lower-strength doses so as to achieve a maximal plasma concentration of the oxytocin antagonist. Oxytocin antagonists useful in conjunction with the compositions and methods described herein include pyrrolidin-3-one oxime compounds represented by formula (I) 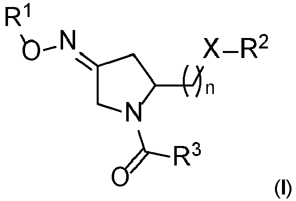 or a geometric isomer, enantiomer, diastereomer, racemate, or salt thereof, wherein
n is an integer from 1 to 3;
R¹ is selected from the group consisting of hydrogen and C₁-C₆ alkyl;
R² is selected from the group consisting of hydrogen, C₁-C₆ alkyl, C₁-C₆ alkyl aryl, heteroaryl, C₁-C₆ alkyl heteroaryl, C₂-C₆ alkenyl, C₂-C₆ alkenyl aryl, C₂-C₆ alkenyl heteroaryl, C₂-C₆ alkynyl, C₂-C₆ alkynyl aryl, C₂-C₆ alkynyl heteroaryl, C₃-C₆ cycloalkyl, heterocycloalkyl, C₁-C₆ alkyl cycloalkyl, C₁-C₆ alkyl heterocycloalkyl, C₁-C₆ alkyl carboxy, acyl, C₁-C₆ alkyl acyl, C₁-C₆ alkyl acyloxy, C₁-C₆ alkyl alkoxy, alkoxycarbonyl, C₁-C₆ alkyl alkoxycarbonyl, aminocarbonyl, C₁-C₆ alkyl aminocarbonyl, C₁-C₆ alkyl acylamino, C₁-C₆ alkyl ureido, amino, C₁-C₆ alkyl amino, sulfonyloxy, C₁-C₆ alkyl sulfonyloxy, sulfonyl, C₁-C₆ alkyl sulfonyl, sulfinyl, C₁-C₆ alkyl sulfinyl, C₁-C₆ alkyl sulfanyl, and C₁-C₆ alkyl sulfonylamino;
R³ is selected from the group consisting of aryl and heteroaryl;
X is selected from the group consisting of oxygen and NR⁴; and
R⁴ is selected from the group consisting of hydrogen, C₁-C₆ alkyl, C₁-C₆ alkyl aryl, C₁-C₆ alkyl heteroaryl, aryl, and heteroaryl, wherein R² and R⁴, together with the nitrogen to which they are bound, can form a 5-8 membered saturated or unsaturated heterocycloalkyl ring. Compounds of this genus are described, for example, in US Patent No. 7,115,754. For instance, oxytocin antagonists that can be used in conjunction with the compositions and methods described herein include (3Z,5S)-5-(hydroxymethyl)-1-[(2'-methyl-1,1'-biphenyl-4-yl)carbonyl]pyrrolidin-3-one O-methyloxime, represented by formula (II), below.

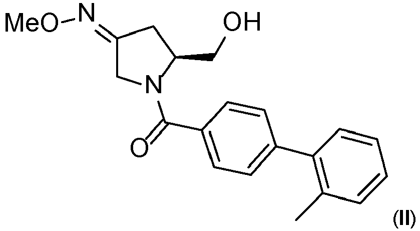

Using the methods described herein, one can administer an oxytocin antagonist, such as compound (I) or compound (II), to a subject, such as a mammalian subject (e.g., a female human subject) in order to promote enhanced endometrial receptivity, reduce the likelihood of embryo implantation failure, and/or prevent miscarriage in a subject following the transfer of one or more embryos to the uterus of the subject. According to the methods described herein, a compound of formula (I), such as compound (II), may be administered to a subject prior to, concurrently with, and/or following the transfer of one or more embryos to the uterus of the subject so as to achieve a serum concentration of the compound of, for example, from about 1 µM to about 20 µM.

The subject may be one that has previously undergone one or more successful or unsuccessful embryo implantation procedures. Alternatively, the subject may be one that has not undergone a previous embryo transfer cycle. According to the methods described herein, the one or more embryos that are ultimately transferred to the subject can be obtained, for instance, by in vitro fertilization (IFV) or intracytoplasmic sperm injection (ICSI) of an ovum isolated or derived from the subject or from a donor. For instances in which the ovum is isolated or derived from the subject, the ovum may be isolated from the subject directly or may be produced ex vivo by inducing maturation of one or more oocytes isolated from the subject. The ova or oocytes may be isolated from the subject, for instance, from about 1 day to about 7 days prior to embryo transfer. In some embodiments, the ova or oocytes are isolated from the subject from about 2 days to about 5 days prior to embryo transfer (e.g., 2 days, 3 days, 4 days, or 5 days prior to embryo transfer). Following fertilization of the ovum by contact with one or more sperm cells, the subsequently formed zygote can be matured ex vivo so as to produce an embryo, such as a morula or blastula (e.g., a mammalian blastocyst), which can then be transferred to the uterus of the subject for implantation into the endometrium. Embryo transfers that can be performed using the methods described herein include fresh embryo transfers, in which the ovum or oocyte used for embryo generation is retrieved from the subject and the ensuing embryo is transferred to the subject during the same menstrual cycle. The embryo can alternatively be produced and cryopreserved for long-term storage prior to transfer to the subject.

The present disclosure additionally features dosing regimens that can be applied to a subject undergoing embryo transfer therapy with an oxytocin antagonist, such as a compound of formula (I) or formula (II) or another oxytocin antagonist described herein, such as epelsiban, retosiban, barusiban, and atosiban, or a salt, derivative, variant, crystal form, or formulation thereof. Using the methods described herein, an oxytocin antagonist such as one of the foregoing agents can be administered to a subject before, during, or after embryo transfer in order to enhance endometrial receptivity, promote successful embryo implantation, and/or prevent the occurrence of a miscarriage in the subject.

For instance, a compound of formula (I) or formula (II) can be administered to a subject hours prior to embryo transfer, such as from about 1 hour to about 24 hours prior to the transfer of one or more embryos to the uterus of the subject, for instance, in a single dose of about 100 mg, 300 mg, or 900 mg to the subject or in multiple doses of lower strength, for example, in repeat doses of about 100 mg each. In some embodiments, the compound is administered to the subject from about 1 hour to about 12 hours prior to embryo transfer, such as about 4 hours prior to embryo transfer. Using the methods described herein, the oxytocin antagonist, such as a compound of formula (I) or formula (II), can be administered to the subject concurrently with the transfer of one or more embryos to the uterus of the subject, such as within 60 minutes of embryo transfer, for instance, in a single dose of about 100 mg, 300 mg, or 900 mg to the subject or in multiple doses of lower strength, for example, in repeat doses of about 100 mg each. Additionally or alternatively, the oxytocin antagonist can be administered to the subject following embryo transfer, such as from about 1 hour to about 24 hours following embryo transfer. For instance, the oxytocin antagonist can be administered following embryo transfer in a single dose of about 100 mg, 300 mg, or 900 mg to the subject or in multiple doses of lower strength, for example, in repeat doses of about 100 mg each. In dosing regimens in which the oxytocin antagonist is administered in multiple doses, the compound (e.g., compound (I) or compound (II)) may be administered in multiple doses per day, such as in from 1 dose to 7 doses per day. The dosing may terminate, for instance, on the day of embryo transfer to the subject, or may continue following embryo transfer.

The sections that follow provide a description of various oxytocin antagonists useful in conjunction with the compositions and methods provided by the disclosure, as well as a description of dosing regimens that may guide the administration of oxytocin antagonists to a subject so as to enhance endometrial receptivity upon embryo transfer, reduce the likelihood of embryo implantation failure, and/or prevent the occurrence of a miscarriage in a subject undergoing an assisted reproduction procedure.

### (3Z,5S)-5-(hydroxymethyl)-1-[(2'-methyl-1,1'-biphenyl-4-yl)carbonyl]pyrrolidin-3-one O-methyloxime (Compound II)

Compounds of formula (I), such as (3Z,5S)-5-(hydroxymethyl)-1-[(2'-methyl-1,1'-biphenyl-4-yl)carbonyl]pyrrolidin-3-one O-methyloxime, represented by formula (II), above, are non-peptide oxytocin antagonists that can be used to enhance endometrial receptivity, promote successful embryo implantation, and reduce the likelihood of miscarriage in subjects undergoing or that have undergone embryo transfer therapy. Compound (II), in particular, is an orally-active oxytocin antagonist capable of inhibiting human oxytocin receptor with a Kᵢ of 52 nM and suppressing Ca²⁺ mobilization in cultured HEK293EBNA cells with an IC₅₀ of 81 nM. Additionally, compound (II) selectively inhibits the oxytocin receptor over the vasopressin Via receptor, as compound (II) inhibits the vasopressin Via receptor with a Kᵢ of 120 nM. Compound (II) additionally demonstrates a variety of favorable pharmacokinetic properties, as this compound exhibits an oral bioavailability of from 42-100%, with a serum half-life of from 11-12 hours and a tmax of from about 1-4 hours. Additionally, compound (II) is safely tolerated in human subjects, for instance, at doses of up to 1500 mg. The foregoing biochemical properties of compound (II), as well as methods for the synthesis and purification of this compound, are described in detail, for instance, in US Patent No. 9,670,155.

### Synthesis of Compound (II)

An exemplary procedure for the synthesis of compound (II) is shown in Scheme 1, below.

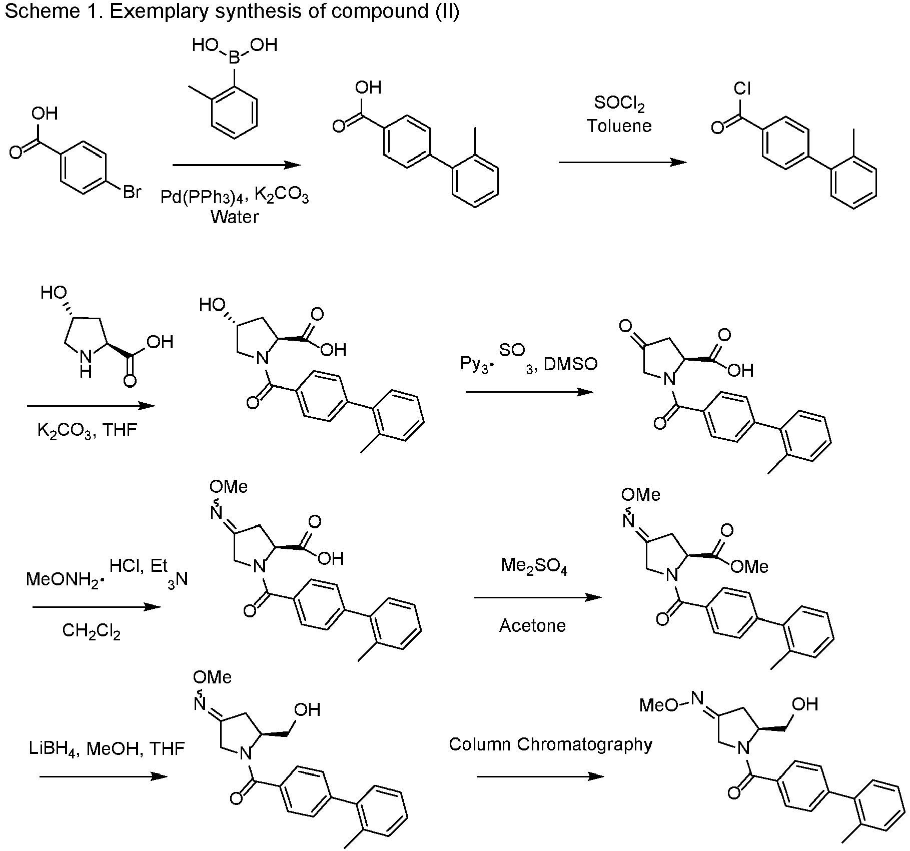

### Purity of Compound (II)

In some embodiments, the compound represented by formula (II) (i.e., (3Z,5S)-5-(hydroxymethyl)-1-[(2'-methyl-1,1'-biphenyl-4-yl)carbonyl]pyrrolidin-3-one O-methyloxime) is substantially pure. For instance, in some embodiments, the compound represented by formula (II) has a purity of at least 85%, such as a purity of from 85% to 99.9% or more (e.g., a purity of 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, or more). The purity of the compound represented by formula (II) may be assessed, for instance, using NMR techniques and/or chromatographic methods, such as HPLC procedures, that are known in the art and described herein, such as those techniques that are described in US Patent No. 9,670,155.

In some embodiments, the compound represented by formula (II) is substantially pure with respect to diastereomers of this compound and other by-products that may be formed during the synthesis of this compound. For instance, in some embodiments, the compound represented by formula (II) has a purity of at least 85%, such as a purity of from 85% to 99.9% or more (e.g., a purity of 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, or more) with respect to diastereomers of this compound and other by-products that may be formed during the synthesis of this compound, such as a by-product that is formed during the synthesis of this compound as described in US Patent No. 9,670,155. The purity of the compound represented by formula (II) may be assessed, for instance, using NMR techniques and/or chromatographic methods, such as HPLC procedures, that are known in the art and described herein, such as those techniques that are described in US Patent No. 9,670,155.

In some embodiments, the compound represented by formula (II) is substantially pure with respect to its (3E) diastereomer, (3*E*,5*S*)-5-(hydroxymethyl)-1-[(2'-methyl-1,1'-biphenyl-4-yl)carbonyl]pyrrolidin-3-one O-methyloxime. For instance, in some embodiments, the compound represented by formula (II) has a purity of at least 85%, such as a purity of from 85% to 99.9% or more (e.g., a purity of 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, or more) with respect to (3E,5S)-5-(hydroxymethyl)-1-[(2'-methyl-1,1'-biphenyl-4-yl)carbonyl]pyrrolidin-3-one O-methyloxime. For instance, compound (II) may be administered in the form of a composition (e.g., a tablet, such as a dispersible tablet, capsule, gel cap, powder, liquid solution, or liquid suspension) that contains less than 15% of the (3E) diastereomer. For example, compound (II) may be administered in the form of a composition (e.g., a tablet, such as a dispersible tablet, capsule, gel cap, powder, liquid solution, or liquid suspension) that contains less than 14%, less than 13%, less than 12%, less than 11%, less than 10%, less than 9%, less than 8%, less than 7%, less than 6%, less than 5%, less than 4%, less than 3%, less than 2%, less than 1%, less than 0.1%, less than 0.01%, less than 0.001 %, or less of the (3E) diastereomer. The purity of the compound represented by formula (II) may be assessed, for instance, using NMR techniques and/or chromatographic methods, such as HPLC procedures, that are known in the art and described herein, such as those techniques that are described in US Patent No. 9,670,155.

### Therapeutic Activity

The present invention is based in part on the discovery that compounds of formula (I), such as compound (II), is capable of promoting successful endometrial implantation of a transferred embryo in female human subjects and prolonging the duration of pregnancy relative to subjects not treated with this compound. Specifically, compound (II) has been found to reduce the risk of embryo implantation failure in clinical studies conducted with human subjects that previously underwent ovarian hyperstimulation and oocyte retrieval. It has been discovered that compounds of formula (I), such as compound (II), increase the rate of successful embryo implantation as assessed by a variety of metrics. These manifestations have been found to include an increase in the rate of positive pregnancy tests at 14 days, 6 weeks, and 10 weeks following embryo transfer and/or oocyte retrieval, as well as an increase in the rate of live births at a gestational age of at least 24 weeks.

Surprisingly, oxytocin antagonists, such as compounds of formula (I) and (II) and other oxytocin antagonists described herein, are particularly effective in subjects that do not exhibit elevated serum concentrations of progesterone (P4). For instance, as is described in detail in Example 1, below, compound (II) was found to improve successful embryo implantation rate (for example, as assessed by the above metrics) in a dose-dependent manner. This dose-dependent response was found to be particularly strong in subjects that exhibited a pre-treatment serum P4 concentration of less than 320 nM, such as from about 200 nM to about 300 nM or less. The foregoing P4 concentrations were measured on the day of transfer of one or more embryos to the subject. These heightened P4 levels are indicative of an elevated P4 concentration on the day of oocyte or ovum retrieval from the subject, such as a P4 concentration of from 1.0 ng/ml to 2.0 ng/ml (e.g., a P4 concentration of 1.0 ng/ml, 1.1 ng/ml, 1.2 ng/ml, 1.3 ng/ml, 1.4 ng/ml, 1.5 ng/ml, 1.6 ng/ml, 1.7 ng/ml, 1.8 ng/ml, 1.9 ng/ml, or 2.0 ng/ml, and particularly, 1.5 ng/ml). Thus, it has been discovered that a subject's propensity to benefit from treatment with an oxytocin antagonist, such as a compound of formula (I) or formula (II), or another oxytocin antagonist described herein, such as epelsiban, retosiban, barusiban, or atosiban, or a salt, derivative, variant, crystal form, or formulation thereof, can be determined based on the subject's pre-treatment serum level of P4.

Using the compositions and methods described herein, one of skill in the art can assess a patient's likelihood of benefitting from (e.g., experiencing enhanced (i.e., increased) endometrial receptivity in response to) oxytocin antagonist treatment by determining the subject's serum P4 concentration prior to treatment with an oxytocin antagonist. If the subject exhibits a serum P4 concentration below a reference level, such as a serum P4 concentration of below 320 nM on the day of embryo transfer (e.g., up to 24 hours prior to a scheduled embryo transfer, such as immediately prior to a scheduled embryo transfer) or a serum P4 concentration of less than 1.5 ng/ml on the day of oocyte or ovum retrieval (e.g., from 1 to 7 days prior to embryo transfer for a patient undergoing an IVF-ET procedure, such as from 3 to 5 days prior to embryo transfer for a patient undergoing an IVF-ET procedure), the subject may be administered an oxytocin antagonist, for instance, prior to, concurrently with, and/or following the transfer of one or more embryos to the subject. If the subject exhibits a serum P4 concentration above a reference level, such as a serum P4 concentration of above 320 nM on the day of embryo transfer (e.g., up to 24 hours prior to a scheduled embryo transfer, such as immediately prior to a scheduled embryo transfer) or a serum P4 concentration of greater than 1.5 ng/ml on the day of oocyte or ovum retrieval (e.g., from 1 to 7 days prior to embryo transfer for a patient undergoing an IVF-ET procedure, such as from 3 to 5 days prior to embryo transfer for a patient undergoing an IVF-ET procedure), a physician of skill in the art may determine that the subject will not be administered an oxytocin antagonist, and/or that the subject will be re-scheduled for oocyte or ovum retrieval or embryo transfer until such a time as the subject's serum P4 concentration declines to beneath the P4 reference level.

Additionally, without being limited by mechanism, it has been discovered that oxytocin antagonists such as compounds of formula (I) and (II), and other oxytocin antagonists described herein, may promote the transient overexpression of prostaglandin F2α (PGF2α) and prostaglandin E2 (PGE2) and subsequently inhibit the propagation of PGF2α signal transduction. The attenuation of PGF2α signalling may occur, for instance, by desensitization of the PGF2α receptor in response to the initial flare in PGF2α secretion. This pattern of (i) transiently heightened expression of PGF2α followed by (ii) the reduction in PGF2α signaling induced by oxytocin antagonists such as compounds of formula (I) and (II), as well as other oxytocin antagonists described herein, can in turn enhance the receptivity of the endometrium to one or more exogenous embryos, thereby promoting endometrial implantation and reducing the likelihood of embryo implantation failure. Notably, P4 is a negative regulator of PGF2α expression, which may explain why oxytocin antagonists such as compounds of formula (I) and (II), among other oxytocin antagonists described herein, can have a particularly robust therapeutic effect on subjects that do not exhibit elevated pre-treatment serum P4 concentrations. Such subjects include those that do not exhibit pre-treatment serum P4 concentrations of 320 nM or greater on the day of embryo transfer and/or pre-treatment serum P4 concentrations of 1.5 ng/ml or greater on the day of oocyte or ovum retrieval, as described in Examples 1 and 2, below.

The foregoing discoveries form important bases for the oxytocin antagonist dosing regimens described herein. To optimally enhance endometrial receptivity to one or more transferred embryos, compounds of formulas (I) and (II), as well as additional oxytocin antagonists described herein and known in the art, such as epelsiban, retosiban, barusiban, and atosiban, or a salt, derivative, variant, crystal form, or formulation thereof, can be administered to a subject so as to saturate the oxytocin receptor and achieve complete (i.e., 100%) inhibition of the receptor at the time of embryo implantation. This can be achieved, for instance, by administering compounds of formula (I) or (II) or another oxytocin antagonist described herein or known in the art, such as epelsiban, retosiban, barusiban, and atosiban, or a salt, derivative, variant, crystal form, or formulation thereof, to a subject undergoing embryo transfer therapy such that a maximum plasma concentration of the compound is reached at the time of embryo transfer.

For instance, compounds of formula (I) or (II) can be administered to a subject from about 1 hour to about 24 hours prior to embryo transfer, such as from about 1 hour to about 8 hours prior to embryo transfer so as to achieve a maximum plasma concentration of the compound at the time of embryo transfer. In some embodiments, the compound is administered about 4 hours prior to embryo transfer, as it has been discovered that oral administration of various doses of compound (II) results in a peak plasma concentration of the compound at from about 1 hour to about 4 hours following administration of the compound. Compounds of formula (I) or (II) may be administered prior to, during, and/or after embryo transfer in order to enhance endometrial receptivity and promote successful embryo implantation, for instance, as described below.

The sections that follow describe in further detail additional oxytocin antagonists that may be used in conjunction with the compositions and methods of the disclosure, as well as dosing schedules for the administration of oxytocin antagonists to subjects undergoing embryo transfer therapy and methods of assessing whether a subject is likely to benefit from oxytocin antagonist treatment on the basis of the subject's pre-treatment progesterone level(s).

### Oxytocin Antagonist Dosing Regimens

To promote endometrial receptivity and successful embryo implantation and to reduce the likelihood of miscarriage in a subject undergoing or that has undergone embryo transfer therapy, compounds of formula (I) or (II), or another oxytocin antagonist described herein, may be administered to a subject (e.g., a human subject) before, during, or after embryo transfer. In each case, compounds of formula (I) or (II), or another oxytocin antagonist described herein, may be administered to the subject so as to saturate the oxytocin receptor and achieve inhibition (e.g., 50%, 55%, 60%, 65%, 70%, 75%, 80%, 85%, 90%, 95%, 96%, 97%, 98%, 99%, or 100% inhibition) of the receptor prior to embryo transfer, at the time of embryo transfer, and/or following embryo transfer.

### Administration beginning prior to embryo transfer therapy

Compounds of formula (I) or (II) or another oxytocin antagonist described herein, such as epelsiban, retosiban, barusiban, and atosiban, or a salt, derivative, variant, crystal form, or formulation thereof, may be administered to the subject prior to embryo transfer, such as from about 1 hour to about 24 hours prior to the transfer of the one or more embryos to the subject. In some embodiments, the compound is administered to the subject so as to achieve a maximum plasma concentration of the compound at the time of embryo transfer. For instance, in some embodiments, the compound is administered to the subject from about 1 hour to about 8 hours prior to embryo transfer, such as about four hours prior to embryo transfer.

Compounds of formula (I) or (II) may be administered to a subject undergoing embryo transfer therapy in a single dose, such as a single dose of about 100 mg, 300 mg, or 900 mg of the compound, or in multiple doses. When multiple doses of the compound are administered, the compound may be administered in multiple repeat doses of the same strength (e.g., serial doses of about 100 mg, 300 mg, or 900 mg of the compound) or in multiple doses of varying strength (e.g., one or more doses of about 100 mg of the compound followed by one or more doses of about 300 mg of the compound and/or one or more doses of about 900 mg of the compound; one or more doses of about 300 mg of the compound followed by one or more doses of about 100 mg of the compound and/or one or more doses of about 900 mg of the compound; or one or more doses of about 900 mg of the compound followed by one or more doses of about 100 mg of the compound and/or one or more doses of about 300 mg of the compound). When multiple doses are administered, the compound may be administered in lower quantities, such as in multiple doses of about 100 mg or 300 mg. In some embodiments, multiple doses of higher quantities, such as about 900 mg of the compound, may be administered to the subject prior to embryo transfer therapy.

Compounds of formula (I) or (II) may be administered to the subject in a single dose prior to embryo transfer or in multiple doses. For instance, in preparation for embryo transfer, the subject may be administered from 1 to 20 doses, for instance, per 12 hours, per 24 hours, per 36 hours, per 48 hours, per 60 hours, per 72 hours, per 84 hours, per 96 hours, per 108 hours, 120 hours, per 132 hours, per 144 hours, per 156 hours, per 168 hours, or longer, prior to embryo transfer. In some embodiments, the compound is administered to the subject prior to embryo transfer in up to 7 doses (e.g., 1, 2, 3, 4, 5, 6, or 7 doses) per 24 hours, such as in up to 7 doses of 100 mg of compound (II) per 24 hours. The multiple doses may be administered starting at various time points prior to embryo transfer, such as from about 1 hour to about 7 days, or more, prior to embryo transfer. For example, in some instances, the multiple doses are administered starting at from about 1 day to about 7 days prior to embryo transfer. Additionally or alternatively, compounds of formula (I) or formula (II) can be administered to a subject up to 2 weeks, 3 weeks, 4 weeks, or more, in preparation for embryo transfer therapy. Administration of the compound to the subject can begin, for instance, on the day of oocyte or ovum retrieval from the subject, which may occur, e.g., from about 1 day to about 7 days prior to embryo transfer, such as from about 2 days to about 5 days prior to embryo transfer. The subject may be administered low quantities of high strength doses (e.g., one or more doses of a compound of formula (I) or (II) at about 300 mg or 900 mg per dose). Additionally or alternatively, the subject may be administered higher quantities of lower strength doses (e.g., two or more doses of a compound of formula (I) or (II) at about 100 mg).

When multiple doses of the oxytocin antagonist are administered to the subject in preparation for embryo transfer, the multiple doses may terminate, for instance, on the day of embryo transfer to the subject. In some embodiments, the multiple doses terminate with a final dose of the compound that is administered concurrently with (e.g., within 60 minutes of) transfer of the one or more embryos to the subject. Alternatively, the multiple doses of compound (I) or (II) may continue following embryo transfer. For instance, the compound may be administered to the subject in one or more additional doses following embryo transfer, for instance, in multiple repeat doses or doses of varying strength. The compound may be administered to the subject in one or more additional doses administered within, for instance, from about 1 hour to about 1 week, or longer (e.g., within about 1 hour, 2 hours, 3 hours, 4 hours, 5 hours, 6 hours, 7 hours, 8 hours, 9 hours, 10 hours, 11 hours, 12 hours, 24 hours, 36 hours, 48 hours, 60 hours, 72 hours, 84 hours, 96 hours, 108 hours, 120 hours, 132 hours, 144 hours, 156 hours, 168 hours, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, or more) following the transfer of the one or more embryos to the subject. When multiple doses of compound (I) or compound (II) are administered to a subject following embryo transfer, the subject may be administered the additional doses, for instance, in regular intervals. Compounds of formula (I) or formula (II) may be administered to the subject following embryo transfer therapy, for example, in from 1 to 20 additional doses per day, per week, per month, or longer. For instance, the compound may be administered to the subject following embryo transfer in up to 7 doses (e.g., 1, 2, 3, 4, 5, 6, or 7 doses) per 24 hours, such as in up to 7 doses of 100 mg of compound (II) per 24 hours.

### Administration beginning during embryo transfer therapy

Compounds of formula (I) or (II) or another oxytocin antagonist described herein, such as epelsiban, retosiban, barusiban, and atosiban, or a salt, derivative, variant, crystal form, or formulation thereof, may be administered to a subject during embryo transfer, such as within about 60 minutes or less of transfer of the embryo to the uterus of the subject. In such instances, the compound may be administered to the subject in a single dose, such as a single dose of about 100 mg, 300 mg, or 900 mg of the compound of formula (I) or (II), or in multiple doses. A single dose of the compound can be administered, for instance, at the initiation of the embryo transfer procedure. For example, a compound of formula (I) or (II) may be administered to the subject upon entrance of an embryo delivery device, such as a catheter containing the one or more embryos to be transferred to the subject, into the vaginal canal of the subject. Additionally or alternatively, the compound may be administered to the subject upon entrance of the embryo delivery device beyond the cervix and into the uterus of the subject. The compound may be administered to the subject upon expulsion of the one or more embryos to be transferred from the embryo delivery device, and/or upon removal of the embryo delivery device from the uterus or vaginal canal of the subject. In some embodiments, multiple doses of the compound are administered throughout the duration of the embryo transfer process. Compounds of formula (I) or (II) may be administered continuously throughout the embryo transfer process, for instance, by continuous intravenous administration.

When multiple doses of compound (I) or compound (II) are administered to the subject beginning during the embryo transfer process, the multiple doses may include multiple repeat doses (e.g., serial doses of about 100 mg, 300 mg, or 900 mg of the compound) or multiple doses of varying strength (e.g., one or more doses of about 100 mg of the compound followed by one or more doses of about 300 mg of the compound and/or one or more doses of about 900 mg of the compound; one or more doses of about 300 mg of the compound followed by one or more doses of about 100 mg of the compound and/or one or more doses of about 900 mg of the compound; or one or more doses of about 900 mg of the compound followed by one or more doses of about 100 mg of the compound and/or one or more doses of about 300 mg of the compound). When multiple doses are administered, the compound may be administered in lower quantities, such as in multiple doses of about 100 mg or 300 mg. In some embodiments, multiple doses of higher quantities, such as about 900 mg of the compound, may be administered to the subject.

Dosing of the oxytocin antagonist that has begun during embryo transfer (e.g., within 60 minutes or less of the embryo transfer) may continue following embryo transfer. For instance, the compound may be administered to the subject in one or more additional doses following embryo transfer, for instance, in multiple repeat doses or doses of varying strength. The compound may be administered to the subject in one or more additional doses administered within, for instance, from about 1 hour to about 1 week, or longer (e.g., within about 1 hour, 2 hours, 3 hours, 4 hours, 5 hours, 6 hours, 7 hours, 8 hours, 9 hours, 10 hours, 11 hours, 12 hours, 24 hours, 36 hours, 48 hours, 60 hours, 72 hours, 84 hours, 96 hours, 108 hours, 120 hours, 132 hours, 144 hours, 156 hours, 168 hours, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, or more) following the transfer of the one or more embryos to the subject. When multiple doses of compound (I) or compound (II) are administered to a subject following embryo transfer, the subject may be administered the additional doses, for instance, in regular intervals. Compounds of formula (I) or formula (II) may be administered to the subject following embryo transfer therapy, for example, in from 1 to 20 additional doses per day, per week, per month, or longer. For instance, the compound may be administered to the subject following embryo transfer in up to 7 doses (e.g., 1, 2, 3, 4, 5, 6, or 7 doses) per 24 hours, such as in up to 7 doses of 100 mg of compound (II) per 24 hours.

### Administration beginning following embryo transfer therapy

Dosing of the oxytocin antagonist (e.g., a compound of formula (I) or (II) or another oxytocin antagonist described herein, such as epelsiban, retosiban, barusiban, and atosiban, or a salt, derivative, variant, crystal form, or formulation thereof) may begin following the completion of the embryo transfer process. For instance, the compound of formula (I) or (II) may be administered to the subject following embryo transfer in a single dose or in multiple doses, such as in multiple repeat doses (e.g., serial doses of about 100 mg, 300 mg, or 900 mg of the compound) or in multiple doses of varying strength (e.g., one or more doses of about 100 mg of the compound followed by one or more doses of about 300 mg of the compound and/or one or more doses of about 900 mg of the compound; one or more doses of about 300 mg of the compound followed by one or more doses of about 100 mg of the compound and/or one or more doses of about 900 mg of the compound; or one or more doses of about 900 mg of the compound followed by one or more doses of about 100 mg of the compound and/or one or more doses of about 300 mg of the compound). When multiple doses are administered, the compound may be administered in lower quantities, such as in multiple doses of about 100 mg or 300 mg. In some embodiments, multiple doses of higher quantities, such as about 900 mg of the compound, may be administered to the subject following embryo transfer therapy.

The compound may be administered to the subject in one or more doses administered within, for instance, from about 1 hour to about 1 week, or longer (e.g., within about 1 hour, 2 hours, 3 hours, 4 hours, 5 hours, 6 hours, 7 hours, 8 hours, 9 hours, 10 hours, 11 hours, 12 hours, 24 hours, 36 hours, 48 hours, 60 hours, 72 hours, 84 hours, 96 hours, 108 hours, 120 hours, 132 hours, 144 hours, 156 hours, 168 hours, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, or more) following the transfer of the one or more embryos to the subject. When multiple doses of compound (I) or compound (II) are administered to a subject following embryo transfer, the subject may be administered the doses, for instance, in regular intervals. Compounds of formula (I) or formula (II) may be administered to the subject following embryo transfer therapy, for example, in from 1 to 20 doses per day, per week, per month, or longer. For instance, the compound may be administered to the subject following embryo transfer in up to 7 doses (e.g., 1, 2, 3, 4, 5, 6, or 7 doses) per 24 hours, such as in up to 7 doses of 100 mg of compound (II) per 24 hours.

### Methods of Assessing Serum Progesterone Levels

Using the compositions and methods described herein, one of skill in the art can assess the likelihood that a subject (e.g., a human subject) undergoing embryo transfer therapy will benefit from oxytocin antagonist treatment by comparing the serum progesterone concentration of a subject to a progesterone reference level. For instance, a physician of skill in the art can withdraw a sample from a subject undergoing embryo transfer therapy at one of multiple time points during an assisted reproductive technology process. Upon comparing the subject's serum progesterone concentration to that of an appropriate progesterone reference level, a determination that the subject exhibits a reduced serum progesterone concentration relative to the progesterone reference level indicates that the subject is particularly well suited for, and likely to benefit from (e.g., likely to exhibit enhanced endometrial receptivity in response to) treatment with an oxytocin antagonist, such as a compound of formula (I) or (II), or another oxytocin antagonist described herein or known in the art, such as epelsiban, retosiban, barusiban, and atosiban, prior to, concurrently with, and/or following the transfer of one or more embryos to the uterus of the subject.

For example, the sample may be withdrawn from the subject on the day of oocyte or ovum retrieval in the case of a subject using an autologous gamete for the ex vivo production of an embryo. In such instances, the progesterone reference level may be from 1.0 ng/ml to 2.0 ng/ml, such as 1.0 ng/ml, 1.1 ng/ml, 1.2 ng/ml, 1.3 ng/ml, 1.4 ng/ml, 1.5 ng/ml, 1.6 ng/ml, 1.7 ng/ml, 1.8 ng/ml, 1.9 ng/ml, or 2.0 ng/ml. The progesterone reference level may be, for instance, 1.5 ng/ml in such instances. The physician may then compare the progesterone level in the sample (e.g., serum sample) isolated from the subject to that of the progesterone reference level. A determination that the subject exhibits a reduced serum progesterone concentration relative to the progesterone reference level indicates that the subject is particularly well suited for, and likely to benefit from (e.g., likely to exhibit enhanced endometrial receptivity in response to), treatment with an oxytocin antagonist.

Additionally or alternatively, the sample may be withdrawn from the subject on the day of the embryo transfer procedure (e.g., following oocyte or ovum retrieval in the case of a subject using an autologous gamete for the ex vivo production of an embryo). In such instances, the progesterone reference level may be from 200 nM to 300 nM or more, such as 320 nM. The physician may then compare the progesterone level in the sample (e.g., serum sample) isolated from the subject to that of the progesterone reference level. A determination that the subject exhibits a reduced serum progesterone concentration relative to the progesterone reference level indicates that the subject is particularly well suited for, and likely to benefit from (e.g., likely to exhibit enhanced endometrial receptivity in response to), treatment with an oxytocin antagonist.

Methods of quantitating the concentration of progesterone in a sample (e.g., serum sample) isolated from a subject are known in the art and include, for instance, competitive enzyme-linked immunosorbant assays (ELISA), such as those described in US Patent No. 9,201,077. Antibodies capable of specifically binding to progesterone and that may be used in conjunction with progesterone detection assays include those produced and released by ATCC Accession Number HB 8886 as described in US Patent No. 4,720,455.

### Follicular Maturation and Oocyte/Ovum Retrieval

A variety of methods can be used in order to induce follicular maturation and to perform oocyte (e.g., mature oocyte) retrieval in conjunction with the compositions and methods described herein. In some embodiments, ova or oocytes are isolated from the subject from about 1 day to about 7 days prior to the transfer of the one or more embryos to the subject, such as from about 2 days to about 5 days prior to embryo transfer. The ova or oocytes isolated from the subject may include mature oocytes, such as from 1 to 4 mature oocytes that are ready for fertilization upon contact with one or more sperm cells. The ova or oocytes may be isolated from a subject undergoing embryo transfer therapy or from a donor, such as a familial donor.

A subject undergoing embryo transfer therapy or a donor may be prepared for ovum or oocyte retrieval by controlled ovarian hyperstimulation, for instance, according to methods described herein or known in the art. For example, a subject or donor may be administered a GnRH antagonist so as to prevent a premature increase in the serum concentration of luteinizing hormone (LH). Additionally or alternatively, final follicular maturation can be achieved by administration of hCG to the subject or donor prior to isolation of the one or more ova or oocytes. For instance, the hCG can be administered to the subject in a single dose or in multiple doses, for instance, by intravenous injection according to procedures known in the art.

In some embodiments, a luteal support is provided to the subject or donor following ovum or oocyte retrieval. This may be performed, for instance, by administering progesterone to the subject or donor following the retrieval procedure. For example, progesterone may be administered to the subject or donor intravaginally at a dose of from about 300 mg to about 600 mg. The progesterone may be administered to the subject in a single dose or in multiple doses. For instance, progesterone may be administered to the subject in regularly spaced intervals beginning within about 24 hours of isolation of the one or more ova or oocytes, such as within 12 hours of retrieval, and continuing for about 6 or more weeks following the transfer of the one or more embryos to the subject.

### Embryo Quality and Condition

Embryos for use in conjunction with the compositions and methods described herein include those that are at, for example, the morula or the blastula stage of embryonic development. For instance, embryos that may be transferred to a subject as described herein include those that contain from 6 to 8 blastomeres immediately prior to transfer of the one or more embryos to the subject. The blastomeres may be of approximately equal sizes as assessed by visual microscopy prior to the transfer of the one or more embryos to the subject.

Embryos for use in conjunction with the compositions and methods described herein include those that are formed, for instance, by IVF or ICSI methods known in the art. In some embodiments, the embryos are freshly transferred to the uterus of the subject, for instance, from about 1 day to about 7 days (e.g., from about 2 days to about 5 days) following the isolation of one or more oocytes or ova from the subject for IVF or ICSI. In some embodiments, the one or more embryos are frozen and cryopreserved for long-term storage prior to thaw and transfer to the subject. Methods for the cryopreservation of embryos are known in the art and have been described, for instance, in WO 1991/003935 and WO 2010/011766.

### Methods of Assessing Pregnancy

Techniques for assessing pregnancy for use in conjunction with the compositions and methods described herein include qualitative and quantitative assessments of a sample isolated from a subject, such as a sample of blood or urine. Methods for assessing pregnancy include detecting the presence and/or quantity of hCG in a sample isolated from a subject. This can be achieved, for instance, using conventional receptor-ligand binding assays known in the art, such as through the use of competitive radioligand binding assays, which are described for the detection of hCG in US Patent No. 4,094,963. Additionally or alternatively, test strips may be used to determine hCG concentrations, as described, for instance, in US Patent No. 7,989,217. Urine samples isolated from a subject can additionally be analyzed in order to determine pregnancy, as described, for instance, in US Patent No. 4,315,908.

Additionally or alternatively, pregnancy may be assessed by detecting intrauterine heartbeat, such as the heartbeat of the embryo or developing fetus following successful embryo implantation. Compositions and methods for detecting embryonic and fetal heartbeat are known in the art and are described, for instance, in US Patent Nos. 3,780,725 and 4,437,467.

Following embryo transfer, for instance, as described herein, a subject may be subject to one or more pregnancy tests, for example, using one or more of the foregoing procedures. The subject may be tested for pregnancy at one or more points following embryo transfer therapy, such as at about 14 days, about 6 weeks, about 10 weeks, or longer, following embryo transfer and/or oocyte retrieval.

### Pharmaceutical Compositions

Oxytocin antagonists for use with the compositions and methods of the disclosure can be formulated into a pharmaceutical composition for administration to a subject, such as a female human subject, in a biologically compatible form suitable for administration in vivo. A pharmaceutical composition containing an oxytocin antagonist (e.g., a compound of formula (I) or (II), above) may additionally contain a suitable diluent, carrier, or excipient. Oxytocin antagonists can be administered to a subject, for example, orally or by intravenous injection. Under ordinary conditions of storage and use, a pharmaceutical composition may contain a preservative, e.g., to prevent the growth of microorganisms. Conventional procedures and ingredients for the selection and preparation of suitable formulations are described, for example, in Remington: The Science and Practice of Pharmacy (2012, 22nd ed.) and in The United States Pharmacopeia: The National Formulary (2015, USP 38 NF 33).

In some embodiments, compound (II) is administered to a subject according to the methods described herein in crystalline form. For instance, compound (II) may be administered to a subject undergoing embryo transfer therapy in a crystalline form that exhibits characteristic X-ray powder diffraction peaks at about 7.05° 2θ, about 13.13° 2θ, and about 23.34° 2θ. For instance, the compound may exhibits characteristic X-ray powder diffraction peaks at about 7.05° 2θ, about 12.25° 2θ, about 13.13° 2θ, about 16.54° 2θ, about 18.00° 2θ, about 21.84° 2θ, and about 23.34° 2θ. In some embodiments, the compound exhibits characteristic X-ray powder diffraction peaks as set forth in Table 1, below.

### Compound for use

In another aspect, the disclosure provides a compound of formula (I) or (II) or another oxytocin antagonist described herein, such as epelsiban, retosiban, barusiban, and atosiban, or a salt, derivative, variant, crystal form, or formulation thereof, for use in in the treatment of a subject undergoing embryo transfer therapy, wherein said compound is administered to said subject prior to transfer of one or more embryos to the uterus of said subject, and wherein said administering reduces the likelihood of embryo implantation failure.

### Medicament

In an further aspect, the disclosure provides the use of a compound of formula (I) or (II) or another oxytocin antagonist described herein, such as epelsiban, retosiban, barusiban, and atosiban, or a salt, derivative, variant, crystal form, or formulation thereof, in the preparation of a medicament for the treatment of a subject undergoing embryo transfer therapy, wherein said medicament is administered to said subject prior to transfer of one or more embryos to the uterus of said subject, and wherein said administering reduces the likelihood of embryo implantation failure.

**Table 1. Characteristic X-ray powder diffraction (XRPD) peaks of crystal form of compound (II)**

| **XRPD Peak (°2θ)** | ***d* space (Å)** | **Intensity (%)** |
|---|---|---|
| 7.05 ± 0.20 | 12.520 ± 0.354 | 45 |
| 12.25 ± 0.20 | 7.218 ± 0.117 | 36 |
| 13.13 ± 0.20 | 6.739 ± 0.102 | 55 |
| 14.16 ± 0.20 | 6.250 ± 0.088 | 8 |
| 16.54 ± 0.20 | 5.356 ± 0.064 | 38 |
| 18.00 ± 0.20 | 4.923 ± 0.054 | 36 |
| 18.77 ± 0.20 | 4.723 ± 0.050 | 34 |
| 21.32 ± 0.20 | 4.165 ± 0.039 | 5 |
| 21.84 ± 0.2 | 4.066 ± 0.037 | 36 |
| 23.34 ± 0.20 | 3.808 ± 0.032 | 100 |
| 24.08 ± 0.20 | 3.693 ± 0.030 | 14 |
| 24.67 ± 0.20 | 3.605 ± 0.029 | 1 |
| 25.45 ± 0.20 | 3.497 ± 0.027 | 27 |
| 25.69 ± 0.20 | 3.465 ± 0.027 | 8 |
| 26.45 ± 0.20 | 3.367 ± 0.025 | 10 |
| 27.09 ± 0.20 | 3.289 ± 0.024 | 2 |
| 28.05 ± 0.20 | 3.179 ± 0.022 | 14 |
| 28.56 ± 0.20 | 3.123 ± 0.021 | 3 |
| 29.26 ± 0.20 | 3.050 ± 0.020 | 16 |
| 30.72 ± 0.20 | 2.908 ± 0.018 | 2 |
| 31.00 ± 0.20 | 2.882 ± 0.018 | |
| 31.19 ± 0.20 | 2.865 ± 0.018 | 5 |
| 33.19 ± 0.20 | 2.697 ± 0.016 | 2 |
| 33.60 ± 0.20 | 2.665 ± 0.015 | 6 |
| 34.36 ± 0.20 | 2.608 ± 0.015 | 4 |
| 34.75 ± 0.20 | 2.580 ± 0.014 | 2 |
| 35.91 ± 0.20 | 2.499 ± 0.013 | 2 |
| 36.52 ± 0.20 | 2.458 ± 0.013 | 3 |
| 37.38 ± 0.20 | 2.404 ± 0.012 | 2 |
| 37.70 ± 0.20 | 2.384 ± 0.012 | 1 |
| 38.73 ± 0.20 | 2.323 ± 0.012 | 3 |
| 39.11 ± 0.20 | 2.301 ± 0.011 | 2 |
| 39.80 0.20 | 2.264 0.011 | 4 |

The foregoing crystal form has been shown to exhibit enhanced stability to aqueous media and physical stress, and is described in detail, for instance, in US 2016/0002160.

Compounds of formula (I) or (II) can be administered by a variety of routes, such as orally or intravenously. When formulated for oral administration, for instance, the compound may be administered in the form of a tablet, capsule, gel cap, powder, liquid solution, or liquid suspension. In some embodiments, the compound is administered to the subject in the form of a tablet, such as a dispersible tablet. The dispersible tablet may have, for example, one or more, or all, of the following components:
a. about 1-20% by weight of calcium silicate;
b. about 0.1-20% by weight of PVP30K;
c. about 0.01-5% by weight of poloxamer 188;
d. about 0.5-20% by weight of sodium croscarmellose;
e. about 1-90% by weight of microcrystalline cellulose 112;
f. about 1-90% by weight of lactose monohydrate;
g. about 0.01-0.5% by weight of sodium saccharine; and
h. about 0.1-10% by weight of glycerol dibehenate.

For instance, the dispersible tablet may have the following composition:
a. about 5% by weight of calcium silicate;
b. about 1% by weight of PVP30K;
c. about 2% by weight of poloxamer 188;
d. about 5% by weight of sodium croscarmellose;
e. about 1.5% by weight of microcrystalline cellulose 112;
f. about 47.8% by weight of lactose monohydrate;
g. about 0.2% by weight of sodium saccharine; and
h. about 4% by weight of glycerol dibehenate.

The foregoing formulations of compound (II) have been shown to exhibit rapid absorption kinetics upon administration to a subject, and are described in detail, for instance, in US 2015/0164859.

Pharmaceutical compositions of compound (I) or (II) may include sterile aqueous solutions, dispersions, or powders, e.g., for the extemporaneous preparation of sterile solutions or dispersions. In all cases the form may be sterilized using techniques known in the art and may be fluidized to the extent that may be easily administered to a subject in need of treatment.

### Examples

The following examples are put forth so as to provide those of ordinary skill in the art with a description of how the compositions and methods described herein may be used, made, and evaluated, and are intended to be purely exemplary of the invention and are not intended to limit the scope of what the inventors regard as their invention.

### Example 1. Oral administration of compound (II) promotes successful embryo implantation and prolongs pregnancy in subjects undergoing embryo transfer therapy

### Materials and Methods

In a randomized, double-blind, parallel groups, Phase 2 clinical study of the efficacy of compound (II) in enhancing endometrial receptivity and promoting successful embryo implantation in humans, this compound was orally administered to subjects undergoing embryo transfer therapy in doses of varying strength. A total of 247 female subjects were selected for treatment based on a variety of inclusion criteria. Of these, 244 subjects completed the study. The study was open to healthy female volunteers from 18 to 36 years of age that had previously undergone up to one IVF or ICSI cycle that resulting in a negative pregnancy test as assessed by hCG detection, despite the transfer of at least one embryo of good quality, which was defined as an embryo having from six to eight blastomeres of uniform size and shape on the day of embryo transfer, ooplasm having no granularity, absence of multinucleation, and a maximum fragmentation of 10%. Subjects included in the study had at least one functional ovary and were capable of communicating with the investigator and research staff and complying with the requirements of the study protocol. A demographic summary of the subjects included in the study is shown in Table 2, below. Data are presented in the form of mean (standard deviation).

**Table 2. Demographic summary of subjects included in study**

| **Parameter** | **Unit** | **Placebo** | **Compound (II)** | | |
|---|---|---|---|---|---|
| | | | 100 mg dose | 300 mq dose | 900 mg dose |
| | | n = 65 | n = 62 | n = 60 | n = 60 |
| Age | years | 31.5 (3.3) | 31.5 (3.1) | 31.8 (3.1) | 31.1 (3.3) |
| Body mass index | kg/m² | 23.37 (4.15) | 23.86 (3.72) | 23.72 (6.29) | 23.65 (3.99) |
| Endometrium thickness | mm | 7.0 (2.9) | 7.0 (2.8) | 6.8 (2.4) | 6.9 (2.9) |
| Oocytes retrieved | n | 11.0 (5.2) | 10.3 (4.4) | 11.7 (5.6) | 10.2 (4.2) |
| Embryos generated | n | 6.6 (3.1) | 6.2 (3.7) | 7.3 (4.1) | 5.9 (3.2) |
| Good quality embryos generated | n | 3.7 (2.3) | 3.6 (3.5) | 4.4 (3.6) | 3.5 (2.9) |
| Embryos transferred | % n = 1 | 60.0% | 62.9% | 60.0% | 59.3% |
| | % n = 2 | 40.0% | 37.1% | 40.0% | 40.7% |
| Embryo transfer difficult | % | 1.50% | 3.20% | 1.70% | 0.0% |
| Uterine contraction rate at time of embryo transfer | n/min | 2.01 (0.68) | 2.05 (0.49) | 1.97 (0.56) | 2.12 (0.48) |
| Serum P4 level at time of embryo transfer | nM | 287 (156) | 256 (155) | 321 (155) | 238 (130) |
| Serum E2 level at time of embryo transfer | pM | 4255 (2790) | 3833 (2127) | 4988 (2913) | 4265 (2781) |
| Serum compound (II) level at time of embryo transfer | ng/mL | N/A | 484.1 (159.8) | 1453.1 (453.8) | 4159.0 (1367.7) |

Subjects included in the study underwent an initial screening period beginning up to 12 weeks prior to the day of oocyte retrieval from the subject. During this 12-week period, subjects underwent physical and gynecological examination in preparation for oocyte retrieval. This analysis included a recordation of the subjects' vital signs, hematology and biochemistry analysis of blood samples withdrawn from the subjects, urinary analysis, and a comprehensive review of the subjects' medical histories.

At the conclusion of the screening period, subjects underwent controlled ovarian hyperstimulation by administration of a GnRH antagonist so as to prevent a premature rise in serum LH concentration. Concurrent pre-treatment with an oral contraceptive prior to controlled ovarian hyperstimulation was allowed, but not required. Final follicular maturation was performed with a single administration of hCG to the subject. Luteal support was performed by intravaginal administration of micronized natural progesterone at a dose of 600 mg (3 x 200 mg dosage forms) daily, commencing within 6-24 hours of oocyte retrieval. Progesterone administration continued for at least 6 weeks following embryo transfer for subjects testing positive for pregnancy at 14 days following embryo transfer. Retrieved oocytes contained at least 1-4 mature oocytes (i.e., ova), which were subsequently used for IVF or ICSI for embryo generation.

The embryo transfer procedure was conducted three days after the oocyte retrieval day (OPU + 3 days). Subjects undergoing embryo transfer were monitored prior to initiating the procedure. This analysis included a recordation of vital signs, as well as a transvaginal ultrasound to assess uterine contraction rate and endometrial thickness. Subjects were considered eligible for embryo transfer if the uterine contraction rate was found to be greater than or equal to 1.5 contractions per minute. Eligible subjects subsequently underwent blood sample analysis to determine pre-treatment levels of serum E2 and P4.

Upon confirming eligibility, subjects were randomized to one of four treatment arms: those receiving a single 100 mg dose of compound (II), a single 300 mg dose of compound (II), a single 900 mg dose of compound (II), or placebo. Subjects receiving a 100 mg dose of compound (II) received 2 x 50 mg dispersible tablets. Subjects receiving a 300 mg dose of compound (II) received 2 x 50 mg dispersible tablets and 1 x 200 mg dispersible tablet. Subjects receiving a 900 mg dose of compound (II) received 2 x 50 mg dispersible tablets and 4 x 200 mg dispersible tablets. Subjects not treated with compound (II) were administered a placebo, for instance, in 2 x 50 mg dispersible tablets and 4 x 200 mg dispersible tablets. Subjects did not consume food or fluids, with the exception of water, for 2 hours prior to administration and for 1 hour following administration.

Subjects were administered the indicated dose of compound (II) or placebo approximately 4 hours prior to embryo transfer. About 30 minutes prior to embryo transfer (approximately 3.5 hours following administration of compound (II) or placebo), a transvaginal ultrasound was conducted so as to record uterine contraction rate, and blood sample analysis was performed to obtain a post-treatment measurement of serum concentrations of compound (II), E2, and P4. At 4 hours following treatment with compound (II) or placebo, subjects underwent an ultrasound-guided embryo transfer according to conventional procedures. From one to two embryos of good quality were transferred to each subject. To reduce uterine contractions at the time of embryo transfer, soft or ultra-soft catheters were used and contact with the uterine fundus was avoided. Any difficulties that occurred during the embryo transfer procedure were recorded, including instances in which uterine sounding or cervical dilation were required, instances in which a harder catheter was required, or instances in which blood was found in any part of the catheter.

Approximately 1 hour following embryo transfer, subjects underwent a final physical examination and were subsequently discharged from the clinical unit until the first follow-up visit, which occurred at about 14 days following oocyte retrieval (OPU + 14 days). At this time, subjects underwent a physical examination as well as a blood sample analysis to assess pregnancy by detection of hCG. Subjects testing positive for pregnancy continued the study and were scheduled for follow-up examinations at about 6 weeks following embryo transfer and at about 10 weeks following oocyte retrieval (OPU + 10 weeks). Subjects that returned for examination at about 6 weeks following embryo transfer underwent ultrasound analysis. Pregnancy status was monitored by detecting embryo heartbeat. Subjects that exhibited a live birth during the study were scheduled for follow-up consultations to assess the subjects' physical state.

### Statistical Analysis

A two-sided type I error rate of 0.1 (corresponding to a one-sided type I error rate of 0.05) was used for analysis of data collected from this study. Subjects with a negative blood pregnancy test at 14 days following oocyte retrieval were considered as negative for the subsequent efficacy endpoints (e.g., pregnancy tests at 6 weeks following embryo transfer and 10 weeks following oocyte retrieval, as well as live birth rate).

Analysis of pregnancy rate at 6 weeks following embryo transfer was conducted via the Cochran-Armitage test of a linear trend in proportions, using all of the treatment arms as an ordinal scaled variable. A secondary analysis was conducted by fitting a logistic regression model with dose as a covariate and testing whether the slope was equal to zero. As higher pregnancy rates may occur with increasing number of transferred embryos, any potential effect of the number of embryos transferred on efficacy was explored, for example, by using the embryo transfer rate as a covariate. In addition, a potential dose time embryo transfer rate interaction was explored. Any potential effect of the embryo transfer difficulty on efficacy was also explored. Any possible site to site effect was explored as well.

Individual dose versus placebo comparisons were tested via Fisher's exact test and as contrasts within the logistic regression models. Corresponding confidence intervals were produced. No multiplicity adjustment was planned for these individual comparisons.

Positive blood pregnancy test at 14 days following oocyte retrieval and positive embryo heartbeat at 10 weeks following oocyte retrieval were assessed in the same manner as described above. Change from baseline to the time of embryo transfer in uterine contraction rate was analyzed by the Wilcoxon Rank Sum Test by comparing the uterine contraction rate associated with each dose to that observed with placebo-treated subjects.

For descriptive statistics of plasma concentrations of compound (II), E2, and P4, concentrations below the limit of quantification (LOQ) were assigned a value of zero, and results were provided if at least 2/3 of the plasma values per time point were above the LOQ.

### Results

A summary of the results of the clinical study over the entirety of the subjects that participated in the trial is shown in Table 3, below. The primary parameters of interest included the relative change in uterine contractility, positive pregnancy rates at about 14 days and 6 weeks following embryo transfer, positive pregnancy rates at 10 weeks following oocyte retrieval, as well as the live birth rate at a gestational age of at least 24 weeks.

**Table 3. Results of compound (II) treatment among all subjects that participated in clinical trial**

| **Parameter** | **Placebo** | **Compound (II)** | | | |
|---|---|---|---|---|---|
| | | 100 mg dose | 300 mg dose | 900 mg dose | All doses |
| | n = 65 | n = 62 | n = 60 | n = 60 | n = 182 |
| Relative changes in uterine contractions | 0.0% | -8.7% | -4.0% | -13.3% | |
| | Wilcoxon Rank Test | p=0.30 | p=0.72% | p=0.05 | p=0.14 |
| Positive pregnancy test at 14 days post embryo transfer | 50.8% | 56.5% | 50.0% | 53.3% | |
| | Fisher Exact Test | p=0.59 | p=1.00 | p=0.86 | |
| | Logistic Model* | p=0.52 | p=0.93 | p=0.77 | Trend test p=0.96 |
| Ongoing pregnancy rate at 6 weeks post embryo transfer | 33.8% | 46.8% | 35.0% | 46.7% | 42.9% |
| | Fisher Exact Test | p=0.15 | p=1.00 | p=0.15 | p=0.24 |
| | Logistic Model II** | p=0.09 | p=0.99 | p=0.12 | Trend test p=0.33 |
| Ongoing pregnancy rate at 10 weeks post oocyte retrieval | 29.2% | 43.5% | 35.0% | 45.0% | 41.2% |
| | Fisher Exact Test | p=0.10 | p=0.57 | p=0.09 | p=0.10 |
| | Logistic Model | p=0.10 | p=0.49 | p=0.07 | Trend test p=0.15 |
| Live birth rate at gestational age of at least 24 weeks | 29.2% | 40.3% | 35.0% | 43.3% | 39.6% |
| | Fisher Exact Test | p=0.20 | p=0.57 | p=0.14 | p=0.18 |
| | Logistic Model | p=0.19 | p=0.49 | p=0.10 | Trend test p=0.20 |

| | | | | | |
|---|---|---|---|---|---|
| *Logistic Model: Endpoint as dependent variable and treatment, site, and embryo transfer rate as independent variable **Logistic Model II: Endpoint as dependent variable and treatment as independent variable | | | | | |

During the course of the analysis, it was noted that subjects in the 300 mg compound (II) treatment arm exhibited elevated pre-treatment serum P4 concentrations relative to the remainder of the subjects studied (Table 2). These heightened P4 levels are indicative of an elevated P4 concentration on the day of oocyte retrieval from the subject, and can reflect a P4 concentration of from 1.0 ng/ml to 2.0 ng/ml, such as a P4 concentration of 1.5 ng/ml on the day of oocyte retrieval. It was discovered that the effect of compound (II) was particularly robust among subjects that did not exhibit an elevated serum P4 concentration at the time of embryo transfer, and thus, likely did not exhibit a P4 concentration at or above a level of 1.5 ng/ml on the day of oocyte retrieval. Table 4, below, provides a summary of the pregnancy rate at 6 weeks following embryo transfer exhibited by subjects from each pre-treatment serum P4 concentration quartile.

**Table 4. Pregnancy rate at about 6 weeks following embryo transfer by pre-treatment serum P4 concentration quartile**

| **Ongoing pregnancy rate at 6 weeks post embryo transfer** | Pre-dose Serum [P4] Quartile | | | | |
|---|---|---|---|---|---|
| Frequency | 1 | 2 | 3 | 4 | Total |
| Negative | 32 (51.61 %) | 37 (59.68%) | 33 (54.10%) | 45 (72.58%) | 147 |
| Positive | 30 (48.39%) | 25 (40.32%) | 28 (45.90%) | 17 (27.42%) | 100 |
| Total | 62 | 62 | 61 | 62 | 247 |

Table 5, below, provides a summary of the live birth rate at a gestational age of at least 24 weeks (i) exhibited by all subjects and (ii) excluding subjects that exhibited a pre-treatment serum P4 concentration from the upper quartile of this metric.

**Table 5. Live birth rate at a gestational age of at least 24 weeks among subjects from all pre-treatment serum P4 quartiles and excluding subjects from the upper quartile of this metric**

| **Live birth rate at a gestational age of at least 24 weeks** | Frequency | | | | | |
|---|---|---|---|---|---|---|
| Subject Population | No. of embryos transferred | Placebo | 100 mg dose | 300 mg dose | 900 mg dose | All doses |
| All P4 quartiles | 1 | 11/39 (28.21%) | 13/39 (33.33%) | 12/36 (33.33%) | 14/35 (40.00%) | 39/110 (35.45%) |
| | | Fisher Exact Test | p=0.81 | p=0.80 | p=0.33 | p=0.44 |
| | | | | | | Trend test: p=0.24 |
| | 2 | 8/26 (30.77%) | 12/23 (52.17%) | 9/24 (37.50%) | 12/24 (50.00%) | 33/71 (46.48%) |
| | | Fisher Exact Test | p=0.15 | p=0.77 | p=0.25 | p=0.25 |
| | | | | | | Trend test: p=0.31 |
| Excluding upper P4 quartile | 1 | 9/30 (30.00%) | 11/34 (32.35%) | 10/22 (45.45%) | 13/29 (44.83%) | 34/85 (40.00%) |
| | | Fisher Exact Test | p=1.00 | p=0.38 | p=0.29 | p=0.39 |
| | | | | | | Trend test: p=0.16 |
| | 2 | 6/19 (31.58%) | 8/16 (50.00%) | 7/13 (53.85%) | 12/20 (60.00%) | 27/49 (55.10%) |
| | | Fisher Exact Test | p=0.32 | p=0.28 | p=0.11 | p=0.11 |
| | | | | | | Trend test: p=0.08 |

Collectively, these data demonstrate that lower overall pregnancy rates were observed among subjects with elevated pre-dose serum P4 concentrations. Upon analyzing, post hoc, the collected data with respect to subjects from pre-dose serum P4 concentration quartiles 1-3, an enhanced therapeutic effect of compound (II) was observed (Figures 3-5). This analysis is summarized in Table 6, below. Collectively, these data demonstrate that treatment with compound (II) lead to an overall increase in pregnancy and live-birth rates in the treatment arms versus the placebo group with in a significant dose-dependent fashion (p<0.02).

**Table 6. Results of compound (II) treatment excluding subjects from pre-treatment serum P4 concentration Q4**

| **Parameter** | **Placebo** | **Compound (II)** | | | |
|---|---|---|---|---|---|
| | | 100 mg dose | 300 mg dose | 900 mg dose | All doses |
| | n = 49 | n = 50 | n = 35 | n = 49 | n = 134 |
| Positive pregnancy test at 14 days post embryo transfer | 53.1% | 54.0% | 62.9% | 59.2% | |
| | Fisher Exact Test | p=1.00 | p=0.50 | p=0.68 | p=0.61 |
| | | | | | Trend test p=0.42 |
| Ongoing pregnancy rate at 6 weeks post embryo transfer | 36.7% | 44.0% | 48.6% | 53.1% | 48.5% |
| | Fisher Exact Test | p=0.54 | p=0.37 | p=0.15 | p=0.18 |
| | | | | | Trend test p=0.095 |
| Ongoing pregnancy rate at 10 weeks post oocyte retrieval | 30.6% | 42.0% | 48.6% | 51.0% | 47.0% |
| | Fisher Exact Test | p=0.30 | p=0.11 | p=0.064 | p=0.063 |
| | | | | | Trend test p=0.035 |
| Live birth rate at gestational age of at least 24 weeks | 15/49; 30.6% | 19/50; 38.0% | 17/35; 48.6% | 25/49; 51.0% | 61/134; 45.5% |
| | Fisher Exact Test | p=0.53 | p=0.11 | p=0.064 | p=0.090 |
| | | | | | Trend test p=0.025 |
| Absolute increase in live birth rate vs placebo | | 7.4% | 18.0% | 20.4% | 14.9% |
| Relative increase in live birth rate vs placebo | | 24.2% | 58.8% | 66.7% | 48.7% |

This post hoc analysis revealed that subjects exhibiting an elevated serum P4 concentration on the day of embryo transfer also exhibited an elevated serum P4 concentration on the day of oocyte retrieval, such as a serum P4 concentration above the threshold level of 1.5 ng/ml. Table 7, below, summarizes the quantity of subjects for which data were available that exhibited a serum P4 concentration above 1.5 ng/ml on the day of oocyte retrieval prior to administration of hCG to induce final follicular maturation.

**Table 7. Subjects that exhibited a serum P4 concentration above 1.5 ng/ml on the day of oocyte retrieval prior to hCG administration**

| **Subject Population** | **Placebo** | **Compound (II)** | | | Total |
|---|---|---|---|---|---|
| | | 100 mg dose | 300 mg dose | 900 mg dose | |
| Serum P4 greater than 1.5 ng/ml on the day of oocyte retrieval | 1/24 (4.17%) | 1/23 (4.35%) | 5/24 (20.83%) | 2/23 (8.70%) | 9/94 (9.57%) |

As shown in Table 7, the 300 mg treatment arm contained the highest proportion of subjects having a serum P4 concentration of greater than 1.5 ng/ml on the day of oocyte retrieval prior to hCG administration. Table 2, above, demonstrates that subjects in the 300 mg treatment arm exhibited an elevated serum P4 concentration on the day of embryo transfer as well (e.g., an average serum P4 concentration of about 320 nM). Taken together, these data demonstrate that subjects exhibiting an elevated serum P4 concentration on the day of embryo transfer, such as 320 nM or greater, also exhibited a heightened serum P4 concentration on the day of oocyte retrieval, such as 1.5 ng/ml or greater.

As described above, removal of subjects from the upper serum P4 quartile from the analysis revealed a particularly robust therapeutic effect of compound (II). A regression analysis was conducted to quantify the ability of pre-treatment serum progesterone concentration on the day of embryo transfer to serve as a negative predictor of clinical pregnancy. This regression analysis is summarized in Table 8, below.

**Table 8. Regression model of utility of pre-treatment serum P4 as a negative predictor of clinical pregnancy**

| **Regression Parameter** | **Variable** | | |
|---|---|---|---|
| | Pre-treatment serum E2 | Pre-treatment serum P4 | No. of embryos transferred |
| N | 245 | 245 | 245 |
| Adjusted odds ratio | 1.05 | 0.78 | 1.72 |
| 90% Confidence interval of adjusted odds ratio | 0.66-1.44 | 0.65-0.93 | 1.10-3.69 |
| p-value | 0.40 | 0.020 | 0.045 |

As shown in Table 8, a significant negative relationship was identified between pre-treatment serum progesterone concentration and clinical pregnancy rate.

It has been presently discovered that compound (II) may promote the transient overexpression of PGF2α and the subsequent downregulation of PGF2α signaling, for instance, by desensitization of the PGF2α receptor. This heightened expression of PGF2α and subsequent attenuation of PGF2α signaling can in turn enhance the receptivity of the endometrium to exogenously administered embryos. Notably, P4 is a negative regulator of PGF2α expression, which may explain why compound (II) has a particularly strong therapeutic effect on subjects that do not exhibit elevated pre-treatment serum P4 concentrations.

Taken together, the data obtained from this study demonstrate the ability of compound (II) to promote endometrial receptivity, reduce the likelihood of embryo implantation failure in subjects undergoing embryo transfer therapy, and prolong pregnancy in such subjects through various gestational ages, as well as the ability of pre-treatment serum P4 concentration to serve as a predictive indicator of a subject's propensity to benefit from oxytocin antagonist treatment during the course of an assisted reproductive technology procedure.

### Example 2. Administration of an oxytocin antagonist to a subject undergoing embryo transfer therapy on the basis of the subject's pre-treatment serum progesterone level

Using the compositions and methods described herein, a skilled practitioner can assess the likelihood that a human subject undergoing embryo transfer therapy will benefit from oxytocin antagonist treatment by comparing the serum progesterone concentration of a subject to a progesterone reference level. For example, on the basis of a subject's pre-treatment serum progesterone concentration, a practitioner of skill in the art can determine whether the subject is likely to exhibit increased endometrial receptivity in response to oxytocin antagonist treatment. This determination can subsequently inform the practitioner's decision of whether to administer to the subject an oxytocin antagonist, such as a pyrrolidine-3-one oxime compound of formula (I) or (II) or another oxytocin antagonist described herein or known in the art, such as epelsiban, retosiban, barusiban, and atosiban, or a salt, derivative, variant, crystal form, or formulation thereof.

For instance, a physician of skill in the art can withdraw a sample from a subject undergoing embryo transfer therapy on the day of oocyte or ovum retrieval in the case of a subject using an autologous gamete for the ex vivo production of an embryo. In such instances, the progesterone reference level may be from 1.0 ng/ml to 2.0 ng/ml, such as 1.0 ng/ml, 1.1 ng/ml, 1.2 ng/ml, 1.3 ng/ml, 1.4 ng/ml, 1.5 ng/ml, 1.6 ng/ml, 1.7 ng/ml, 1.8 ng/ml, 1.9 ng/ml, or 2.0 ng/ml. The progesterone reference level may be, for instance, 1.5 ng/ml in such instances. The physician may then compare the progesterone level in the sample (e.g., serum sample) isolated from the subject to that of the progesterone reference level. A determination that the subject exhibits a reduced serum progesterone concentration relative to the progesterone reference level indicates that the subject is particularly well suited for, and likely to benefit from (e.g., likely to exhibit enhanced endometrial receptivity in response to) treatment with an oxytocin antagonist. Upon making such a determination, the physician may subsequently administer an oxytocin antagonist to the subject. The oxytocin antagonist may be administered to the subject prior to, concurrently with, and/or after the transfer of one or more embryos to the subject.

Additionally or alternatively, the physician may withdraw a sample (e.g., a serum sample) from the subject on the day of the embryo transfer procedure (e.g., following oocyte or ovum retrieval in the case of a subject using an autologous gamete for the ex vivo production of an embryo). In such instances, the progesterone reference level may be from 200 nM to 300 nM or more, such as 320 nM. The physician may then compare the progesterone level in the sample (e.g., serum sample) isolated from the subject to that of the progesterone reference level. A determination that the subject exhibits a reduced serum progesterone concentration relative to the progesterone reference level indicates that the subject is particularly well suited for, and likely to benefit from (e.g., likely to exhibit enhanced endometrial receptivity in response to), treatment with an oxytocin antagonist. Upon making such a determination, the physician may subsequently administer an oxytocin antagonist to the subject. The oxytocin antagonist may be administered to the subject prior to, concurrently with, and/or after the transfer of one or more embryos to the subject.

### Example 3. Beneficial oxytocin antagonistic effects and metabolic profile of compound (II)

Using the compositions and methods described herein, one of skill in the art can administer an oxytocin antagonist to a subject undergoing an embryo transfer procedure, such as an oxytocin antagonist represented by formula (I), e.g., compound (II), so as to promote enhanced endometrial receptivity, reduce the likelihood of embryo implantation failure, and/or prevent miscarriage in a subject following the transfer of one or more embryos to the uterus of the subject. When compound (II) is administered as the oxytocin antagonist, it can be particularly advantageous to administer compound (II) in a substantially pure form with respect to its (3E) diastereomer, (3*E*,5*S*)-5-(hydroxymethyl)-1-[(2'-methyl-1,1'-biphenyl-4-yl)carbonyl]pyrrolidin-3-one O-methyloxime, such as in a form containing less than 15%, less than 10%, less than 5%, less than 1%, or less than 0.1% of the (3E) diastereomer. This advantage derives from the discovery that substantially pure compound (II) exhibits a superior ability to inhibit spontaneous uterine contractions relative to the substantially pure (3E) diastereomer. Uterine contractility is one component of endometrial receptivity, and elevated uterine contractility can lead to the expulsion of an embryo from the uterus and failed embryo implantation. This surprising disparity in uterine contractility inhibition between compound (II) and its (3E) diastereomer is described, for instance, in US Patent No. 9,670,155. As described therein, there is a dose-dependent reduction in spontaneous uterine contractions when substantially pure compound (II) is administered at 10, 30, and 60 mg/kg to anesthetized late-term pregnant rats. Inhibition of spontaneous uterine contractions of from about 10% to about 20% was observed from 5 to 15 minutes after oral administration of the substantially pure compound (II), and inhibition of about 42% was observed from 170 to 180 minutes after oral administration of the substantially pure compound (II) at a dose of 60 mg/kg. The inhibitory activity of the substantially pure compound (II) with respect to uterine contraction was found to be markedly higher than that of the substantially pure (3E) diastereomer using the same vehicle and in the same model organism.

This difference in inhibitory activity leads to an important clinical benefit, as the substantially pure compound (II) may be administered to a subject at a lower therapeutically effective dosage relative to the (3E) diastereomer or an isomeric mixture of both compounds.

In addition to exhibiting different inhibitory potencies, the substantially pure compound (II) exhibits superior metabolic properties relative to its (3E) diastereomer. It has been discovered that the substantially pure compound (II) is preferentially metabolized by cytochrome P450 isoform 3A4 (CYP3A4), while the substantially pure (3E) diastereomer is preferentially metabolized by cytochrome P450 isoforms 2D6 (CYP2D6) and 2C19 (CYP2C19).

To measure the metabolic properties of the substantially pure compound (II) and its (3E) diastereomer, microsomal stability assays were conducted. These experiments were designed to investigate the metabolism of the substantially pure compound (II) and its (3E) diastereomer by cytochrome P450 alone (CYP) or in combination with uridine 5'-diphosphoglucuronosyl transferase (UGT). The substantially pure compound (II) and its (3E) diastereomer were each incubated at a concentration of 3 µM with pooled liver microsomes and with appropriate co-factors for either cytochrome P450 alone or in combination with UGT. At five time points over the course of a 45 minute experiment, the compounds were analyzed by liquid chromatography and tandem mass spectrometry (LC-MS/MS). Intrinsic clearance values (CLint) with standard error (SE CLᵢₙₜ) and metabolic half-life (t_{½}) were calculated and are indicated in Table 9, below.

**Table 9. Metabolism of substantially pure (3Z) and (3E) isomers by cytochrome P450 alone or in combination with UGT**

| Compound | Metabolic Stability - CYP | | | | Metabolic Stability - CYP/UGT | | | |
|---|---|---|---|---|---|---|---|---|
| | CLᵢₙₜ (µL/min/mg protein) | SE CLᵢₙₜ | t_{½} (min) | n | CLᵢₙₜ (µL/min/mg protein) | SE CLᵢₙₜ | t_{½} (min) | n |
| Compound (II) | 11.6 | 3.74 | 120 | 5 | 9.33 | 3.87 | 149 | 5 |
| (3*E*) isomer | 6.40 | 3.49 | 216 | 5 | 5.38 | 3.32 | 258 | 5 |
| Z/E ratio | | | 0.56 | | | | 0.58 | |

As shown in Table 9, the metabolic stability of each of the substantially pure compound (II) and its (3E) diastereomer in the presence of co-factors required for cytochrome P450 activity is similar to that of each isomer in the presence of co-factors required for combined cytochrome P450 and UGT activity, indicating that cytochrome P450 is primarily responsible for the metabolic degradation of each isomer.

To determine the selectivity of each of the CYP3A4, CYP2D6, and CYP2C19 isoforms of cytochrome P450, the substantially pure compound (II) and its (3E) diastereomer were each incubated at a concentration of 5 µM with each of the CYP3A4, CYP2C19, and CYP2D6 isoforms. At five time points over the course of a 45 minute experiment, the compounds were analyzed by LC-MS/MS. The percentage of each compound remaining at each time point, along with the metabolic half-lives of each compound in the presence of each cytochrome P450 isoform, are indicated in Tables 10-12, below.

**Table 10. Metabolism of substantially pure (3Z) and (3E) isomers by CYP3A4 isoform**

| Compound | t_{½} (min) | n | Compound Remaining (% of compound present at t=0 min) | | | | |
|---|---|---|---|---|---|---|---|
| | | | 0 min | 5 min | 15 min | 30 min | 45 min |
| Compound (II) | 73.7 | 5 | 100 | 86.5 | 75.9 | 67.3 | 63.9 |
| (3*E*) isomer | 281 | 5 | 100 | 107 | 88.8 | 92.5 | 92.2 |
| Z/E ratio | 0.26^{a} | | | | | | |

| | | | | | | | |
|---|---|---|---|---|---|---|---|
| ^{a}Student's t-test: p=0.37 | | | | | | | |

**Table 11. Metabolism of substantially pure (3Z) and (3E) isomers by CYP2D6 isoform**

| Compound | t_{½} (min) | n | Compound Remaining (% of compound present at t=0 min) | | | | |
|---|---|---|---|---|---|---|---|
| | | | 0 min | 5 min | 15 min | 30 min | 45 min |
| Compound (II) | 14.5 | 5 | 100 | 80.3 | 50.5 | 22.5 | 12.2 |
| (3*E*) isomer | 5.62 | 4 | 100 | 49.3 | 14.1 | 2.43 | 0.845 |
| Z/E ratio | 2.6^{b} | | | | | | |

| | | | | | | | |
|---|---|---|---|---|---|---|---|
| ^{b}Student's t-test: p<0.0001 | | | | | | | |

**Table 12. Metabolism of substantially pure (3Z) and (3E) isomers by CYP2C19 isoform**

| Compound | t_{½} (min) | n | Compound Remaining (% of compound present at t=0 min) | | | | |
|---|---|---|---|---|---|---|---|
| | | | 0 min | 5 min | 15 min | 30 min | 45 min |
| Compound (II) | 60.4 | 5 | 100 | 93.7 | 79.1 | 67.8 | 59.9 |
| (3*E*) isomer | 41.4 | 5 | 100 | 87.6 | 82.7 | 57.1 | 47.4 |
| Z/E ratio | 1.5^{c} | | | | | | |

| | | | | | | | |
|---|---|---|---|---|---|---|---|
| ^{c}Student's t-test: p=0.016 | | | | | | | |

The data shown in Tables 10-12 demonstrate that the substantially pure compound (II) is preferentially metabolized by the CYP3A4 isoform of cytochrome P450, while the substantially pure (3E) diastereomer is preferentially metabolized by the CYP2D6 and CYP2C19 isoforms of cytochrome P450. The selectivity exhibited by these cytochrome P450 isoforms provides a significant clinical benefit. Allelic variation in the CYP2D6 and CYP2D19 isoforms has been correlated with reduced drug metabolism in vivo in certain segments of the population (see, for example, Lynch et al., Am. Fam. Physician 76:391-396, 2007). For example, according to Lynch, 7 percent of white persons and 2 to 7 percent of black persons are poor metabolizers of drugs dependent on CYP2D6, and one in five Asian persons is a poor metabolizer of drugs dependent on CYP2C19. In view of the discovery that the substantially pure compound (II) is preferentially metabolized by CYP3A4, this compound is expected to exhibit more uniform therapeutic and toxicity profiles than the substantially pure (3E) diastereomer.

## Claims

1. A compound, (3Z,5S)-5-(hydroxymethyl)-1-[(2'-methyl-1,1'-biphenyl-4-yl)carbonyl]pyrrolidin-3-one O-methyloxime, represented by formula (II) 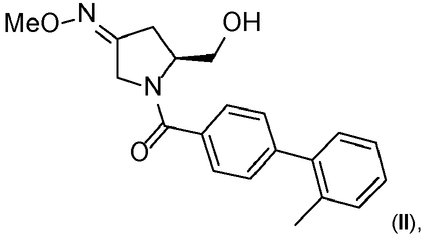 for use in a method of treating a human female subject undergoing embryo transfer therapy, wherein the concentration of progesterone in a sample isolated from the subject has been determined, the method comprising:
a. comparing the concentration of progesterone to a progesterone reference level; and
b. administering a therapeutically effective amount of the compound to the subject if the concentration of progesterone in the sample isolated from the subject is below the progesterone reference level;
wherein one or more human embryos are transferred to the uterus of the subject, and wherein the progesterone reference level is from 1.0 ng/ml to 2.0 ng/ml.

2. A compound, (3Z,5S)-5-(hydroxymethyl)-1-[(2'-methyl-1,1'-biphenyl-4-yl)carbonyl]pyrrolidin-3-one O-methyloxime, represented by formula (II) 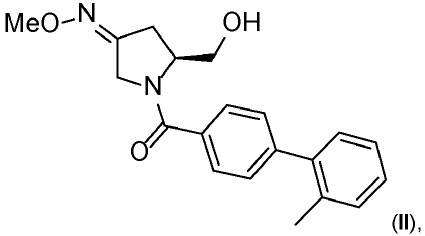 for use in a method of treating a human female subject undergoing embryo transfer therapy, wherein the concentration of progesterone in a sample isolated from the subject has been determined, the method comprising:
a. comparing the concentration of progesterone to a progesterone reference level;
b. administering a therapeutically effective amount of the compound to the subject if the concentration of progesterone in the sample isolated from the subject is below the progesterone reference level; and
c. transferring one or more human embryos to the uterus of the subject;
wherein the progesterone reference level is from 1.0 ng/ml to 2.0 ng/ml.

3. The compound for use according to claim 1 or 2, wherein the compound is administered to the subject prior to the transfer of the one or more embryos to the subject.

4. The compound for use according to claim 3, wherein the compound is administered to the subject from 3 hours to 5 hours prior to the transfer of the one or more embryos to the subject, optionally wherein the compound is administered to the subject about 4 hours prior to the transfer of the one or more embryos to the subject.

5. The compound for use according to any one of claims 1-4, wherein the compound is administered to the subject in a single oral dose, optionally wherein the compound is administered to the subject in a single, oral dose of from 850 mg to 950 mg, optionally wherein the compound is administered to the subject in a single oral dose of about 900 mg.

6. The compound for use according to any one of claims 1-5, wherein the sample isolated from the subject is a blood sample.

7. The compound for use according to any one of claims 1-6, wherein a single embryo is transferred to the subject.

8. The compound for use according to any one of claims 1-7, wherein the one or more embryos have been produced ex vivo by in vitro fertilization (IVF), optionally wherein the one or more embryos have been produced ex vivo by IVF of one or more ova isolated from the subject.

9. The compound for use according to any one of claims 1-7, wherein the one or more embryos have been produced ex vivo by intracytoplasmic sperm injection (ICSI), optionally wherein the one or more embryos have been produced ex vivo by ICSI into one or more ova isolated from the subject.

10. The compound for use according to claim 8 or 9, wherein the one or more ova have been isolated from the subject from 1 day to 7 days prior to the transfer of the one or more embryos to the subject.

11. The compound for use according to claim 10, wherein the one or more ova have been isolated from the subject about 5 days prior to the transfer of the one or more embryos to the subject, or wherein the one or more ova have been isolated from the subject about 3 days prior to the transfer of the one or more embryos to the subject.

12. The compound for use according to any one of claims 8-11, wherein human chorionic gonadotropin (hCG) is administered to the subject prior to isolation of the one or more ova from the subject, optionally wherein the hCG is administered to the subject by a single intravenous injection.

13. The compound for use according to any one of claims 8-12, wherein progesterone is administered to the subject following isolation of the one or more ova from the subject, optionally wherein the progesterone is administered intravaginally.

14. The compound for use according to claim 13, wherein from 300 mg to 600 mg of progesterone per dose is administered to the subject, optionally wherein the progesterone is administered to the subject once daily, optionally beginning within 24 hours of isolation of the one or more ova from the subject and continuing for 6 or more weeks following the transfer of the one or more embryos to the subject.

15. The compound for use according to any one of claims 8-14, wherein the one or more embryos are transferred to the subject during the same menstrual cycle as isolation of the one or more ova from the subject.

16. The compound for use according to any one of claims 1-9, wherein the one or more embryos are frozen and thawed prior to the transfer of the one or more embryos to the subject.

17. The compound for use according to any one of claims 1-16, wherein the progesterone reference level is 1.5 ng/ml, 1.6 ng/ml, 1.7 ng/ml, 1.8 ng/ml, 1.9 ng/ml, or 2.0 ng/ml.

18. The compound for use according to any one of claims 1-16, wherein the progesterone reference level is about 1.5 ng/ml.

19. The compound for use according to any one of claims 1-18, wherein the sample is isolated from the subject from 1 day to 7 days prior to the transfer of the one or more embryos to the subject.

20. The compound for use according to any one of claims 1-18, wherein the sample is isolated from the subject up to 24 hours prior to isolation of one or more ova from the subject.

21. The compound for use according to any one of claims 1-18, wherein the sample is isolated from the subject within about 1 hour of administering hCG to the subject.

22. The compound for use according to any one of claims 1-21, wherein:
(i) the subject sustains pregnancy for at least 14 days following the transfer of the one or more embryos to the subject;
(ii) the subject sustains pregnancy for at least 6 weeks following the transfer of the one or more embryos to the subject;
(iii) the subject sustains pregnancy for at least 10 weeks following isolation of one or more ova from the subject; and/or
(iv) the subject sustains pregnancy and exhibits a live birth following administration of the compound to the subject, optionally wherein the subject exhibits the live birth at a gestational age of at least 24 weeks.

23. The compound for use according to any one of claims 1-22, wherein the compound has a purity of at least 85%, optionally wherein the compound has a purity of from 85% to 99.9%.

## Patentansprüche

1. Verbindung, (3Z,5S)-5-(Hydroxymethyl)-1-[(2'-methyl-1,1'-biphenyl-4-yl)carbonyl]pyrrolidin-3-on-O-methyloxim, dargestellt durch die Formel (II) 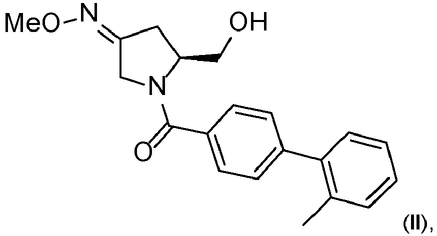 zur Verwendung in einem Verfahren zur Behandlung eines menschlichen weiblichen Subjekts, das sich einer Embryotransfertherapie unterzieht, wobei die Konzentration von Progesteron in einer Probe, die aus dem Subjekt isoliert wurde, bestimmt worden ist, wobei das Verfahren umfasst:
a. Vergleichen der Konzentration von Progesteron mit einem Progesteron-Referenzniveau; und
b. Verabreichen einer therapeutisch wirksamen Menge der Verbindung an das Subjekt, wenn die Konzentration von Progesteron, die aus dem Subjekt isoliert wurde, unter dem Progesteron-Referenzvniveau liegt;
wobei ein oder mehrere menschliche Embryonen in den Uterus des Subjekts transferiert werden, und wobei das Progesteron-Referenzvniveau von 1,0 ng/ml bis 2,0 ng/ml beträgt.

2. Verbindung, (3Z,5S)-5-(Hydroxymethyl)-1-[(2'-methyl-1,1'-biphenyl-4-yl)carbonyl]pyrrolidin-3-on-O-methyloxim, dargestellt durch die Formel (II) 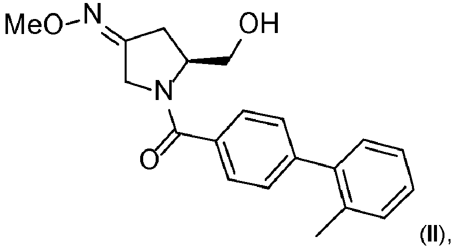 zur Verwendung in einem Verfahren zur Behandlung eines menschlichen weiblichen Subjekts, das sich einer Embryotransfertherapie unterzieht, wobei die Konzentration von Progesteron in einer Probe, die aus dem Subjekt isoliert wurde, bestimmt worden ist, wobei das Verfahren umfasst:
a. Vergleichen der Konzentration von Progesteron mit einem Progesteron-Referenzniveau;
b. Verabreichen einer therapeutisch wirksamen Menge der Verbindung an das Subjekt, wenn die Konzentration von Progesteron, die aus dem Subjekt isoliert wurde, unter dem Progesteron-Referenzvniveau liegt; und
c. Transferieren eines oder mehrerer menschlicher Embryonen in den Uterus des Subjekts; wobei das Progesteron-Referenzniveau 1,0 ng/ml bis 2,0 ng/ml beträgt.

3. Verbindung zur Verwendung nach Anspruch 1 oder 2, wobei vor dem Transfer des einen oder der mehreren Embryonen in das Subjekt die Verbindung an das Subjekt verabreicht wird.

4. Verbindung zur Verwendung nach Anspruch 3, wobei 3 Stunden bis 5 Stunden vor dem Transfer des einen oder der mehreren Embryonen in das Subjekt die Verbindung an das Subjekt verabreicht wird, wobei optional etwa 4 Stunden vor dem Transfer des einen oder der mehreren Embryonen in das Subjekt die Verbindung an das Subjekt verabreicht wird.

5. Verbindung zur Verwendung nach einem der Ansprüche 1-4, wobei die Verbindung in einer einzelnen oralen Dosis an das Subjekt verabreicht wird, wobei optional die Verbindung in einer einzelnen oralen Dosis von 850 mg bis 950 mg an das Subjekt verabreicht wird, wobei optional die Verbindung in einer einzelnen oralen Dosis von etwa 900 mg an das Subjekt verabreicht wird.

6. Verbindung zur Verwendung nach einem der Ansprüche 1-5, wobei die Probe, die aus dem Subjekt isoliert wurde, eine Blutprobe ist.

7. Verbindung zur Verwendung nach einem der Ansprüche 1-6, wobei ein einzelner Embryo in das Subjekt transferiert wird.

8. Verbindung zur Verwendung nach einem der Ansprüche 1-7, wobei der eine oder die mehreren Embryonen ex vivo durch In-Vitro-Fertilisierung (IVF) erzeugt worden sind, wobei optional der eine oder die mehreren Embryonen ex vivo durch IVF mit einer oder mehreren Eizellen, die aus dem Subjekt isoliert wurden, erzeugt worden sind.

9. Verbindung zur Verwendung nach einem der Ansprüche 1-7, wobei der eine oder die mehreren Embryonen ex vivo durch intrazytoplasmatische Spermieninjektion (ICSI) erzeugt worden sind, wobei optional der eine oder die mehreren Embryonen ex vivo durch ICSI in eine oder mehrere Eizellen, die aus dem Subjekt isoliert wurden, erzeugt worden sind.

10. Verbindung zur Verwendung nach Anspruch 8 oder 9, wobei die eine oder die mehreren Eizellen aus dem Subjekt 1 Tag bis 7 Tage vor dem Transfer des einen oder der mehreren Embryonen in das Subjekt isoliert worden sind.

11. Verbindung zur Verwendung nach Anspruch 10, wobei die eine oder die mehreren Eizellen aus dem Subjekt etwa 5 Tage vor dem Transfer des einen oder der mehreren Embryonen in das Subjekt isoliert worden sind, oder wobei etwa 3 Tage vor dem Transfer des einen oder der mehreren Embryonen in das Subjekt die eine oder die mehreren Eizellen aus dem Subjekt isoliert worden sind.

12. Verbindung zur Verwendung nach einem der Ansprüche 8-11, wobei vor der Isolierung der einen oder der mehreren Eizellen aus dem Subjekt menschliches Choriongonadotropin (hCG) an das Subjekt verabreicht wird, wobei optional das hCG durch eine einzelne intravenöse Injektion an das Subjekt verabreicht wird.

13. Verbindung zur Verwendung nach einem der Ansprüche 8-12, wobei auf die Isolierung der einen oder der mehreren Eizellen aus dem Subjekt folgend Progesteron an das Subjekt verabreicht wird, wobei das Progesteron optional intravaginal verabreicht wird.

14. Verbindung zur Verwendung nach Anspruch 13, wobei 300 mg bis 600 mg Progesteron pro Dosis an das Subjekt verabreicht wird, wobei das Progesteron optional einmal täglich an das Subjekt verabreicht wird, beginnend innerhalb von 24 Stunden nach Isolierung der einen oder der mehreren Eizellen aus dem Subjekt und weiter für 6 oder mehr Wochen auf den Transfer des einen oder der mehreren Embryonen in das Subjekt folgend.

15. Verbindung zur Verwendung nach einem der Ansprüche 8-14, wobei der eine oder die mehreren Embryonen während desselben Menstruationszyklus wie die Isolierung der einen oder der mehreren Eizellen aus dem Subjekt in das Subjekt transferiert werden.

16. Verbindung zur Verwendung nach einem der Ansprüche 1-9, wobei der eine oder die mehreren Embryonen vor dem Transfer des einen oder der mehreren Embryonen in das Subjekt eingefroren und aufgetaut werden.

17. Verbindung zur Verwendung nach einem der Ansprüche 1-16, wobei das Progesteron-Referenzvniveau 1,5 ng/ml, 1,6 ng/ml, 1,7 ng/ml, 1,8 ng/ml, 1,9 ng/ml oder 2,0 ng/ml beträgt.

18. Verbindung zur Verwendung nach einem der Ansprüche 1-16, wobei das Progesteron-Referenzvniveau etwa 1,5 ng/ml beträgt.

19. Verbindung zur Verwendung nach einem der Ansprüche 1-18, wobei 1 Tag bis 7 Tage vor dem Transfer des einen oder der mehreren Embryonen in das Subjekt die Probe aus dem Subjekt isoliert wird.

20. Verbindung zur Verwendung nach einem der Ansprüche 1-18, wobei bis zu 24 Stunden vor der Isolierung einer oder mehrerer Eizellen aus dem Subjekt die Probe aus dem Subjekt isoliert wird.

21. Verbindung zur Verwendung nach einem der Ansprüche 1-18, wobei innerhalb von etwa 1 Stunde nach Verabreichung des hCG an das Subjekt die Probe aus dem Subjekt isoliert wird.

22. Verbindung zur Verwendung nach einem der Ansprüche 1-21, wobei:
(i) das Subjekt mindestens für 14 Tage auf den Transfer des einen oder der mehreren Embryonen in das Subjekt folgend schwanger bleibt;
(ii) das Subjekt mindestens für 6 Wochen auf den Transfer des einen oder der mehreren Embryonen in das Subjekt folgend schwanger bleibt;
(iii) das Subjekt mindestens für 10 Wochen auf Isolierung einer oder mehrerer Eizellen aus dem Subjekt folgend schwanger bleibt; und/oder
(iv) das Subjekt schwanger bleibt und auf die Verabreichung der Verbindung folgend eine Lebendgeburt zeigt, wobei das Subjekt optional die Lebendgeburt bei einem Gestationsalter von mindestens 24 Wochen zeigt.

23. Verbindung zur Verwendung nach einem der Ansprüche 1-22, wobei die Verbindung eine Reinheit von mindestens 85 % aufweist, wobei optional die Verbindung eine Reinheit von 85 % bis 99,9 % aufweist.

## Revendications

1. Composé, O-méthyloxime de (3Z,5S)-5-(hydroxyméthyl)-1-[(2'-méthyl-1,1'-biphényl-4-yl)carbonyl]pyrrolidin-3-one, représenté par la formule (II) 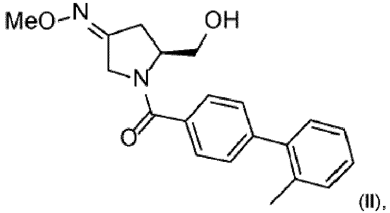 pour une utilisation dans un procédé de traitement d'un sujet humain féminin subissant une thérapie de transfert d'embryons, où la concentration de progestérone dans un échantillon isolé du sujet a été déterminée, le procédé comprenant le fait :
a. de comparer la concentration de progestérone à un niveau de référence de progestérone ; et
b. d'administrer une quantité thérapeutiquement efficace du composé au sujet si la concentration de progestérone dans l'échantillon isolé du sujet est inférieure au niveau de référence de progestérone ;
dans lequel un ou plusieurs embryon(s) humain(s) est/sont transféré(s) dans l'utérus du sujet, et dans lequel le niveau de référence de progestérone est compris entre 1,0 ng/ml et 2,0 ng/ml.

2. Composé, O-méthyloxime de (3Z,5S)-5-(hydroxyméthyl)-1-[(2'-méthyl-1,1'-biphényl-4-yl)carbonyl]pyrrolidin-3-one, représenté par la formule (II) 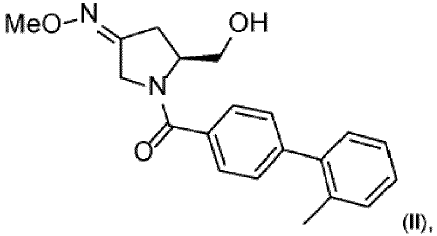 pour une utilisation dans un procédé de traitement d'un sujet humain féminin subissant une thérapie de transfert d'embryons, où la concentration de progestérone dans un échantillon isolé du sujet a été déterminée, le procédé comprenant le fait :
a. de comparer la concentration de progestérone à un niveau de référence de progestérone ;
b. d'administrer une quantité thérapeutiquement efficace du composé au sujet si la concentration de progestérone dans l'échantillon isolé du sujet est inférieure au niveau de référence de progestérone ; et
c. de transférer un ou plusieurs embryon(s) humain(s) dans l'utérus du sujet ;
dans lequel le niveau de référence de progestérone est compris entre 1,0 ng/ml et 2,0 ng/ml.

3. Composé pour une utilisation selon la revendication 1 ou 2, dans lequel le composé est administré au sujet avant le transfert du ou des plusieurs embryon(s) au sujet.

4. Composé pour une utilisation selon la revendication 3, où le composé est administré au sujet 3 heures à 5 heures avant le transfert du ou des plusieurs embryon(s) au sujet, éventuellement où le composé est administré au sujet environ 4 heures avant le transfert du ou des plusieurs embryon(s) au sujet.

5. Composé pour une utilisation selon l'une quelconque des revendications 1 à 4, où le composé est administré au sujet en une seule dose orale, éventuellement où le composé est administré au sujet en une seule dose orale allant de 850 mg à 950 mg, éventuellement où le composé est administré au sujet en une seule dose orale d'environ 900 mg.

6. Composé pour une utilisation selon l'une quelconque des revendications 1 à 5, où l'échantillon isolé du sujet est un échantillon de sang.

7. Composé pour une utilisation selon l'une quelconque des revendications 1 à 6, où un seul embryon est transféré au sujet.

8. Composé pour une utilisation selon l'une quelconque des revendications 1 à 7, où le ou les plusieurs embryon(s) a/ont été produit (s) ex vivo par fécondation in vitro (FIV), éventuellement où le ou les plusieurs embryon(s) a/ont été produit (s) ex vivo par FIV d'un ou de plusieurs ovule(s) isolé(s) du sujet.

9. Composé pour une utilisation selon l'une quelconque des revendications 1 à 7, où le ou les plusieurs embryon(s) a/ont été produit(s) ex vivo par injection intracytoplasmique de spermatozoïdes (ICSI), éventuellement où le ou les plusieurs embryon(s) a/ont été produit(s) ex vivo par ICSI dans un ou plusieurs ovule (s) isolé (s) du sujet.

10. Composé pour une utilisation selon la revendication 8 ou 9, où le ou les plusieurs ovule(s) a/ont été isolé (s) du sujet 1 jour à 7 jours avant le transfert du ou des plusieurs embryon(s) au sujet.

11. Composé pour une utilisation selon la revendication 10, où le ou les plusieurs ovule(s) a/ont été isolé(s) du sujet environ 5 jours avant le transfert du ou des plusieurs embryon(s) au sujet, ou où le ou les plusieurs ovule(s) a/ont été isolé(s) du sujet environ 3 jours avant le transfert du ou des plusieurs embryon(s) au sujet.

12. Composé pour une utilisation selon l'une quelconque des revendications 8 à 11, où la gonadotrophine chorionique humaine (hCG) est administrée au sujet avant d'isoler le ou les plusieurs ovule(s) du sujet, éventuellement où l'hCG est administrée au sujet par une seule injection intraveineuse.

13. Composé pour une utilisation selon l'une quelconque des revendications 8 à 12, où la progestérone est administrée au sujet après isolement du ou des plusieurs ovule(s) du sujet, éventuellement où la progestérone est administrée par voie intravaginale.

14. Composé pour une utilisation selon la revendication 13, où 300 mg à 600 mg de progestérone par dose sont administrés au sujet, éventuellement où la progestérone est administrée au sujet une fois par jour, éventuellement en commençant dans les 24 heures qui suivent l'isolement du ou des plusieurs ovule (s) du sujet et en continuant pendant 6 semaines ou plus après le transfert du ou des plusieurs embryon(s) au sujet.

15. Composé pour une utilisation selon l'une quelconque des revendications 8 à 14, où le ou les plusieurs embryon(s) est/sont transféré(s) au sujet pendant le même cycle menstruel que l'isolement du ou des plusieurs ovule(s) du sujet.

16. Composé pour une utilisation selon l'une quelconque des revendications 1 à 9, où le ou les plusieurs embryon(s) est/sont congelé(s) et décongelé(s) avant le transfert du ou des plusieurs embryon(s) au sujet.

17. Composé pour une utilisation selon l'une quelconque des revendications 1 à 16, où le niveau de référence de progestérone est de 1,5 ng/ml, 1,6 ng/ml, 1,7 ng/ml, 1,8 ng/ml, 1,9 ng/ml ou de 2,0 ng/ml.

18. Composé pour une utilisation selon l'une quelconque des revendications 1 à 16, où le niveau de référence de progestérone est d'environ 1,5 ng/ml.

19. Composé pour une utilisation selon l'une quelconque des revendications 1 à 18, où l'échantillon est isolé du sujet 1 jour à 7 jours avant le transfert du ou des plusieurs embryon(s) au sujet.

20. Composé pour une utilisation selon l'une quelconque des revendications 1 à 18, où l'échantillon est isolé du sujet jusqu'à 24 heures avant l'isolement d'un ou de plusieurs ovule(s) du sujet.

21. Composé pour une utilisation selon l'une quelconque des revendications 1 à 18, où l'échantillon est isolé du sujet environ dans l'heure qui suit l'administration de l'hCG au sujet.

22. Composé pour une utilisation selon l'une quelconque des revendications 1 à 21, où :
(i) le sujet maintient une grossesse pendant au moins 14 jours après le transfert du ou des plusieurs embryon(s) au sujet ;
(ii) le sujet maintient une grossesse pendant au moins 6 semaines après le transfert du ou des plusieurs embryon(s) au sujet ;
(iii) le sujet maintient une grossesse pendant au moins 10 semaines après l'isolement d'un ou de plusieurs ovule(s) du sujet ; et/ou
(iv) le sujet maintient une grossesse et présente une naissance vivante après l'administration du composé au sujet, éventuellement où le sujet présente la naissance vivante à un âge gestationnel d'au moins 24 semaines.

23. Composé pour une utilisation selon l'une quelconque des revendications 1 à 22, où le composé a une pureté d'au moins 85%, éventuellement où le composé a une pureté allant de 85% à 99,9%.
